# EUROPÄISCHE PATENTSCHRIFT

(11) **EP 3 262 163 B1**
(45) Veröffentlichungstag und Bekanntmachung des Hinweises auf die Patenterteilung: **19.06.2019**
(21) Anmeldenummer: 15715955.9
(22) Anmeldetag: 24.02.2015
(51) Int. Cl.: C12N 9/18

(54) **FUSARIUMTOXINE SPALTENDE POLYPEPTIDVARIANTEN, ZUSATZSTOFF ENTHALTEND DIESELBEN UND VERWENDUNG DERSELBEN SOWIE VERFAHREN ZUR SPALTUNG VON FUSARIUMTOXINEN**
FUSARIUM TOXIN-CLEAVING POLYPEPTIDE VARIANTS, ADDITIVES CONTAINING SAME, USE OF SAME, AND METHOD FOR SPLITTING FUSARIUM TOXINS
VARIANTS POLYPEPTIDIQUES DISSOCIANT DES TOXINES DE FUSARIUM, ADDITIF CONTENANT CES VARIANTS ET UTILISATION DE CET ADDITIF ET DE CES VARIANTS ET PROCÉDÉ POUR LA DISSOCIATION DE TOXINES DE FUSARIUM

(43) Veröffentlichungstag der Anmeldung: 03.01.2018
(73) Patentinhaber: Erber Aktiengesellschaft, 3131 Getzersdorf bei Traismauer (AT)
(72) Erfinder: ALESCHKO, Markus, 1210 Wien (AT); KERN, Corinna, 1150 Wien (AT); MOLL, Dieter, 2000 Stockerau (AT); BINDER, Eva Maria, 3430 Tulln (AT); SCHATZMAYR, Gerd, 3430 Tulln (AT)
(74) Vertreter: Cunow, Gerda
(86) Internationale Anmeldenummer: PCT/AT2015/000032
(87) Internationale Veröffentlichungsnummer: WO 2016/134387

(56) Entgegenhaltungen:
- WO-A1-2010/031100
- WO-A2-00/04158
- WO-A2-2004/085624
- HEINL S ET AL: "Degradation of fumonisin B1 by the consecutive action of two bacterial enzymes", JOURNAL OF BIOTECHNOLOGY, ELSEVIER SCIENCE PUBLISHERS, AMSTERDAM, NL, Bd. 145, Nr. 2, 15. Januar 2010 (2010-01-15), Seiten 120-129, XP002566276, ISSN: 0168-1656, DOI: 10.1016/J.JBIOTEC.2009.11.004 [gefunden am 2009-11-14]

## Beschreibung

Die vorliegende Erfindung bezieht sich auf Fusariumtoxine spaltende Polypeptidvarianten, einen Zusatzstoff enthaltend dieselben und eine Verwendung der Polypeptidvarianten und/oder des Zusatzstoffes, sowie auf Verfahren zur Spaltung von Fusariumtoxinen mittels der Polypeptidvarianten und/oder dem die Polypeptidvarianten enthaltenden Zusatzstoff.

Mykotoxine treten in landwirtschaftlichen, pflanzlichen Produkten sehr häufig auf und verursachen je nach Art und Konzentration des Mykotoxins schwere wirtschaftliche Schäden, insbesondere in aus landwirtschaftlichen Produkten hergestellten Nahrungs- oder Futtermitteln und auch bei mit derartigen Nahrungs- oder Futtermitteln ernährten Tieren und Menschen, wobei derartige Schäden äußerst vielfältig sind. Es wurden bereits zahlreiche Methoden entwickelt, mit welchen versucht wird, Mykotoxine unschädlich zu machen, nämlich zu entgiften oder abzubauen, um die durch die Mykotoxine verursachten Schäden in den Bereichen der tierischen und menschlichen Ernährung, der Tierzucht, der Verarbeitung von Futter- und Nahrungsmitteln und dgl. möglichst hintanzuhalten.

Eine prominente Gruppe der Mykotoxine sind Fusariumtoxine, wobei die Begriffe "Fusariumtoxin" oder "Fusariumtoxine" gleichgestellt sind und sich jeweils auf wenigstens ein oder mehrere oder die Gesamtheit der vom Schimmelpilz *Fusarium sp.* produzierten Fumonisine sowie deren Derivate und Abbauprodukte beziehen, jedoch insbesondere auf Fumonisine A1-2 (FA1-2), Fumonisine B1-4 (FB1-4), Fumonisine C1, 2, 4 (FC1, FC2, FC4) und HFC1 als auch auf partiell hydrolysierte Fumonisine FA1-2, FB1-4, FC1-2, FC4 und HFC1. Partiell hydrolysierte Fumonisine besitzen lediglich einen Tricarballylsäurerest, wohingegen FA1-2, FB1-4, FC1-2, FC4 und HFC1 zwei Tricarballylsäurereste aufweisen. Des Weiteren sind auch die strukturell ähnlichen *Alternaria alternata lycopersici* (AAL) Toxine von der Gruppe der Fusariumtoxine mitumfasst, wobei AAL-Toxine in die Gruppen AAL-TA1 (CAS No 79367-52-5), AAL-TA2 (CAS No 79367-51-4), AAL-TB1 (CAS No 176590-32-2) und AAL-TB2 (CAS No 176705-51-4) untergliedert sind. FA1-2, FB1-4, FC1-2, FC4 und HFC1 besitzen folgende Strukturformel:

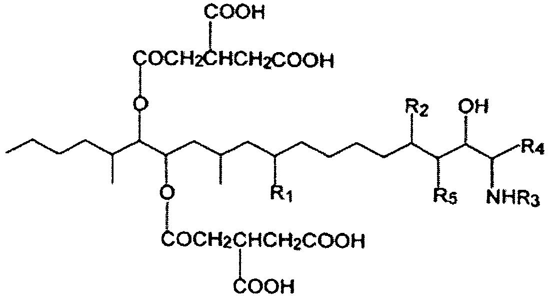

| Fusariumtoxin | R1 | R2 | R3 | R4 | R5 |
|---|---|---|---|---|---|
| FA1 | -OH | -OH | -CH2CO | -CH3 | -H |
| FA2 | -H | -OH | -CH2CO | -CH3 | -H |
| FB1 | -OH | -OH | -H | -CH3 | -H |
| FB2 | -H | -OH | -H | -CH3 | -H |
| FB3 | -OH | -H | -H | -CH3 | -H |
| FB4 | -H | -H | -H | -CH3 | -H |
| FC1 | -OH | -OH | -H | -H | -H |
| FC2 | -OH | -H | -H | -H | -H |
| FC4 | -H | -H | -H | -H | -H |
| HFC1 | -OH | -OH | -H | -H | -OH |

FB1 ist das am häufigsten vorkommende Toxin aus der Gruppe der Fusariumtoxine, jedoch sind zahlreiche Derivate und verwandte Moleküle bekannt, welche ebenfalls toxische Wirkungen bei Menschen und Tieren zeigen. Die Krankheiten, die durch Aufnahme von Mykotoxinen bei Menschen oder Tieren verursacht werden, werden als Mykotoxikosen, im Fall von Fusariumtoxinen auch als Fusariumtoxin-Mykotoxikosen, bezeichnet. So ist es bekannt, dass Fusariumtoxine den Sphingolipidstoffwechsel durch eine Wechselwirkung mit dem Enzym Ceramidsynthase behindern. Sphingolipide sind nicht nur Bestandteil von Zellmembranen, sondern spielen auch in vielen elementaren, zellulären Prozessen, wie dem Zellwachstum, der Zellwanderung und Zellanbindung, bei Entzündungsprozessen oder intrazellulären Transportvorgängen, eine wichtige Rolle als Signal- und Botenmoleküle. Aufgrund der Behinderung des Sphingolipidstoffwechsels werden Fusariumtoxine für die toxische Wirkung auf unterschiedlichste Tierarten und den Menschen verantwortlich gemacht. So konnte nachgewiesen werden, dass Fusariumtoxine immunsuppressiv, bei Nagetieren krebserregend wirken, und sie wurden durch epidemiologische Daten mit Speiseröhrenkrebs und dem Neuralrohrdefekt bei Menschen in Zusammenhang gebracht. Bei verschiedenen Tierarten, wie beispielsweise Schweinen, werden sie für die typische Toxikose durch Lungenödeme verantwortlich gemacht. Beispiele für Fusariumtoxin-Mykotoxikosen sind neurotoxische Erkrankungen wie die equine Leukoenzephalomalazie oder Lungenödeme durch Fumonisinvergiftung im Schwein. Da die Kontamination mit Fusariumtoxinen auf verschiedensten Getreidearten und insbesondere auf Mais, Nüssen und Gemüse nahezu allgegenwärtig ist, sind ihre stark negativen Effekte in Bezug auf die Gesundheit von Menschen und Tieren nicht vernachlässigbar.

Der mikrobielle Abbau von Fumonisinen wurde bereits in der EP-A 1 860 954 beschrieben, gemäß welcher Mikroorganismen zur Entgiftung von Fumonisinen und Fumonisinderivaten eingesetzt werden, bei welchen detoxifizierende Bakterien oder Hefen, gewählt aus genau definierten Stämmen zur Entgiftung von Fumonisinen, Futtermitteln zugesetzt werden.

Auch wurden bereits katabolische Stoffwechselwege für den biologischen Abbau von Fumonisinen und die hierfür verantwortlichen Gene und Enzyme beschrieben. So beschreibt beispielsweise die EP-A 0 988 383 Fumonisin entgiftende Zusammensetzungen und Verfahren, wobei die eingesetzten Fumonisin abbauenden Enzyme in erster Linie in transgenen Pflanzen produziert werden, bei welchen die Entgiftung von Fumonisinen mit Hilfe einer Aminooxidase, welche für ihre enzymatische Aktivität molekularen Sauerstoff benötigt, erfolgt.

Des Weiteren beschreibt die WO 2004/085624 Transaminasen, Deaminasen und Aminomutasen und Verfahren zur enzymatischen Detoxifizierung von aminierten Toxinen, beispielsweise von Fumonisinen. Hierbei werden Polypeptide, welche eine Deaminaseaktivität besitzen, zur Entgiftung eingesetzt.

Die oben genannten Produkte oder Verfahren haben den Nachteil, dass für eine Detoxifizierung der Mykotoxine molekularer Sauerstoff sowie gegebenenfalls Cofaktoren benötigt werden, wobei insbesondere die genannten Aminooxidasen unter sauerstofffreien Reaktionsbedingungen keine Wirkung zeigen.

EP-A 2 326 713 bezieht sich auf einen Zusatzstoff, sowie auf ein Verfahren zur Herstellung desselben, mit dem es gelingt Fumonisine in einer sauerstoffunabhängigen und cofaktorfreien enzymatischen Reaktion abzubauen. Allerdings ist die Temperaturstabilität des darin beschriebenen und für die Detoxifikation maßgeblichen Enzyms, einer Carboxylesterase gering, sodass sich der Zusatzstoff bzw. die Carboxylesterase der SEQ ID-Nr. 46 nicht für Anwendungen bei erhöhten Temperaturen eignet.

In der Nahrungs- und Futtermittelindustrie ist die Hitzebehandlung zur Herstellung von hygienisierten Produkten, in denen die mikrobielle Belastung reduziert ist, von großer Bedeutung. Dabei ist speziell das Pelletieren von Futtermitteln aus einer Mehrzahl von Gründen, wie z.B. zur Verbesserung der Rieseleigenschaft, zur Reduktion der Staubentwicklung, zur Verringerung der mikrobiellen Belastung, insbesondere von Salmonellen, weit verbreitet und stellt einen standardisierten Prozess dar. Während des Pelletierprozesses wird das Gut in der Regel mittels Heißdampf befeuchtet, erhitzt und im Anschluss unter Druck durch eine Matrix gepresst. Der Einsatz von Polypeptiden bzw. Enzymen als Zusatzstoffe zu pelletierenden Nahrungs- oder Futtermitteln stellt eine technische Herausforderung dar, da die Enzyme bzw. Polypeptide in der Regel empfindlich gegenüber erhöhten Temperaturen sind. Die Hitzebehandlung von Enzymen bzw. Polypeptiden kann sich in einer Reduktion ihrer spezifischen Aktivität und/oder in einer irreversiblen Denaturierung auswirken. Eine Möglichkeit dem entgegenzuwirken ist das Verkapseln oder Coaten der Proteine, wie z.B. in der WO 92/12645 beschrieben. Dadurch gelingt es, Proteine vor thermischen Einflüssen zu schützen, jedoch bringt diese Vorgehensweise die Gefahr mit sich, dass die Proteine im Mund-Magen-Darm Trakt nicht schnell genug freigesetzt werden und somit ihre Wirkung entweder nicht schnell genug oder gar nicht entfalten können. Die bisher bekannten Polypeptide zur Detoxifizierung von Fusariumtoxinen können aufgrund ihrer geringen Temperaturstabilität nicht ohne vorherige Verkapselung oder ein vorheriges Coaten den zu pelletierenden Futter- oder Nahrungsmitteln zugesetzt werden.

Technologische Prozesse bei denen eine Entgiftung von Fusariumtoxinen bei erhöhter Temperatur wichtig ist, sind beispielsweise die Produktion von Teigwaren und anderen Produkten aus Mais, wie Polenta, Popcorn, Corn Flakes, Maisbrot oder Tortillas, sowie Verflüssigungsprozesse von Stärke, Saccharifizierungsprozesse oder Fermentationsprozesse, wie z.B. der Maisch- oder Gärprozess insbesondere in der Bioethanolherstellung. Wichtig ist hierbei sicherzustellen, dass die mittels dieser Prozesse hergestellten Nahrungs- oder Futtermittel keine Fusariumtoxine in schädlichen Mengen enthalten. Bisher bekannte Polypeptide können in diesen Prozessen aufgrund ihrer zu geringen bzw. nicht vorhandenen Aktivität bei den jeweiligen Prozesstemperaturen nicht eingesetzt werden.

Es besteht daher der Bedarf an Enzymen bzw. Polypeptiden zur spezifischen, sicheren und zuverlässigen Spaltung, insbesondere zur Detoxifizierung von Fusariumtoxinen, wobei die enzymatische Reaktion weder Sauerstoff noch einen Cofaktor benötigt und das Enzym bzw. Polypeptid darüber hinaus eine ausreichende Temperaturstabilität sowie eine ausreichende Temperaturaktivität aufweist, damit das Enzym bzw. Polypeptid in technologischen Prozessen bei erhöhter Temperatur eingesetzt werden kann.

Die vorliegende Erfindung zielt nun darauf ab, Fusariumtoxine spaltende Polypeptidvarianten einer Fusariumtoxin Carboxylesterase der SEQ ID-Nr. 46 zur Verfügung zu stellen, mit der es gelingt wenigstens ein Fusariumtoxin sauerstoffunabhängig und cofaktorfrei in nicht bzw. weniger toxische Produkte zu spalten, wobei die Polypeptidvarianten eine erhöhte Temperaturstabilität bzw. eine erhöhte Temperaturaktivität im Vergleich zur Fusariumtoxin Carboxylesterase der SEQ ID-Nr. 46 aufweisen.

Zur Lösung dieser Aufgabe ist die vorliegende Erfindung im Wesentlichen dadurch gekennzeichnet, Fusariumtoxine spaltende Polypeptidvarianten einer Fusariumtoxin Carboxylesterase mit der SEQ ID-Nr. 46, dadurch gekennzeichnet, dass die Polypeptidvarianten jeweils eine am N-Terminus um 47 Aminosäuren gekürzte Aminosäuresequenz besitzen, wobei die Aminosäuresequenzen wenigstens 70 %, vorzugsweise 80 % insbesondere bevorzugt 100 % Sequenzidentität, nämlich SEQ ID-Nr. 1, zum Aminosäuresequenzabschnitt 48 - 540 der SEQ ID-Nr. 46 aufweisen, dass die Temperaturstabilitäten (T(50%)) des Polypeptids der SEQ ID-Nr. 46 mit 42 °C und jene der Polypeptidvariante der SEQ ID-Nr. 1 mit 45 °C bestimmt ist oder Modifikationen der SEQ ID-Nr. 1 eine relative Steigerung von T (50 %) im Verhleich zum parenteralen Enzym der SEQ ID-Nr.1 aufweisen, wobei an wenigstens einer Position gewählt aus der Gruppe bestehend aus 10, 33, 66, 107, 140, 144, 149, 151, 157, 199, 266, 267, 270, 272, 275, 280, 284, 286, 293, 302, 312, 329, 332, 360, 363, 364, 365, 367, 371, 372, 377, 389, 391, 394, 418, 419, 424, 427, 429, 430, 436, 440, 443, 447, 453, 455, 456, 457, 462, 463, 464, 465, 469, 473, 478, 487 und 490 als Modifikation eine Aminosäuresubstitution enthalten ist und dass die Aminosäuresubstituenten an den Positionen 10 und 456 gewählt aus Q, E, N, H, K und R sind, an den Positionen 33, 107, 293 und 332 gewählt aus E, Q, D, K, R und N sind, an den Positionen 66, 463 und 478 gewählt aus D, E, K, N, Q und R sind, an den Positionen 140 und 490 gewählt aus P, A, S und N sind, an den Positionen 144 und 367 gewählt aus I, L, M und V sind, an den Positionen 149, 270, 312, 329 und 372 gewählt aus F, Y, W und H sind, an den Positionen 151 und 453 gewählt aus D, E, K und R sind, an den Positionen 157 und 462 gewählt aus F, H, W und Y sind, an den Positionen 199, 302, 365 und 464 gewählt aus I, L, M und V sind, an den Positionen 266 und 455 und gewählt aus A, S und T sind, an den Positionen 267, 394 und 429 gewählt aus A, N, P und S sind, an der Position 272 gewählt aus H, N, Q und S sind, an der Position 275 gewählt aus A, D, E, G, K, N, Q, R und S sind, an der Position 280 gewählt aus A, D, E, K, N, P, Q, R und S, an der Position 284 gewählt aus A, N, P, S, T und V sind, an der Position 286 gewählt aus A, D, E, K, N, P, R und S sind, an den Positionen 360, 377, 391, 419 und 427 gewählt aus A, I, L S, T und V sind, an den Positionen 363, 443 und 457 gewählt aus A, S, T und V sind an der Position 364 gewählt aus H, I, L, M, N, Q, S und V sind, an der Position 371 gewählt aus A, I, L, M, S, T und V sind, an der Position 389 gewählt aus I, L, M und V sind, an den Positionen 418, 430, 447 und 473 gewählt aus A, G und S sind, an der Position 424 gewählt aus A, D, E, G, K, R und S sind, an der Position 436 gewählt aus A, G, S und T sind, an der Position 440 gewählt aus A, G, S und T sind, an der Position 465 gewählt aus A, G, H, N, Q, S und T sind, an der Position 469 gewählt aus D, E, K und R sind und/oder an der Position 487 gewählt aus N, D, Q, H und S sind. Überraschenderweise hat sich gezeigt, dass eine Aminosäuresequenz, welche um 47 Aminosäuren gegenüber der SEQ ID-Nr. 46 gekürzt ist, gegenüber dieser Sequenz sowohl bedeutend aktiver ist als auch eine gegenüber dieser Sequenz erhöhte Temperaturstabilität aufweist. Indem Polypeptidvarianten der SEQ ID Nr. 1 gebildet werden, insbesondere Polypeptidvarianten, die eine Aminosäuresequenz mit wenigstens 70 % Sequenzidentität zur SEQ ID-Nr. 1 aufweisen und an wenigstens einer Position gewählt aus der Gruppe bestehend aus 10, 33, 66, 107, 140, 144, 149, 151, 157, 199, 266, 267, 270, 272, 275, 280, 284, 286, 293, 302, 312, 329, 332, 360, 363, 364, 365, 367, 371, 372, 377, 389, 391, 394, 418, 419, 424, 427, 429, 430, 436, 440, 443, 447, 453, 455, 456, 457, 462, 463, 464, 465, 469, 473, 478, 487 und 490 eine Aminosäuresubstitution aufweisen, wird eine gegenüber der Fusariumtoxin Carboxylesterase mit der SEQ ID-Nr. 1 um wenigstens 4 % gesteigerte Temperaturstabilität erzielt. Durch den Einsatz derartiger Polypeptidvarianten, die eine Fusariumtoxine spaltende Eigenschaft aufweisen, gelingt es Fusariumtoxine beispielsweise während technologischen Prozessen bei erhöhter Temperatur zu detoxifizieren. Dies wird insbesondere durch die zusätzlich zur Temperaturstabilität ebenfalls gesteigerte Temperaturaktivität ermöglicht. Insbesondere kann dadurch sichergestellt werden, dass die enzymatische Aktivität der Polypeptidvarianten auch bei erhöhter Temperaturbeanspruchung, wie dies z.B. auch während des Transportes in Containern der Fall sein kann, beibehalten bleibt.

Gemäß der Erfindung wird eine besonders starke Erhöhung der Temperaturstabilität dadurch erzielt, indem die Aminosäuresubstituenten an den Positionen 10 und 456 gewählt aus Q, E, N, H, K und R sind, an den Positionen 33, 107, 293 und 332 gewählt aus E, Q, D, K, R und N sind, an den Positionen 66, 463 und 478 gewählt aus D, E, K, N, Q und R sind, an den Positionen 140 und 490 gewählt aus P, A, S und N sind, an den Positionen 144 und 367 gewählt aus I, L, M und V sind, an den Positionen 149, 270, 312, 329 und 372 gewählt aus F, Y, W und H sind, an den Positionen 151 und 453 gewählt aus D, E, K und R sind, an den Positionen 157 und 462 gewählt aus F, H, W und Y sind, an den Positionen 199, 302, 365 und 464 gewählt aus I, L, M und V sind, an den Positionen 266 und 455 und gewählt aus A, S und T sind, an den Positionen 267, 394 und 429 gewählt aus A, N, P und S sind, an der Position 272 gewählt aus H, N, Q und S sind, an der Position 275 gewählt aus A, D, E, G, K, N, Q, R und S sind, an der Position 280 gewählt aus A, D, E, K, N, P, Q, R und S, an der Position 284 gewählt aus A, N, P, S, T und V sind, an der Position 286 gewählt aus A, D, E, K, N, P, R und S sind, an den Positionen 360, 377, 391, 419 und 427 gewählt aus A, I, L S, T und V sind, an den Positionen 363, 443 und 457 gewählt aus A, S, T und V sind an der Position 364 gewählt aus H, I, L, M, N, Q, S und V sind, an der Position 371 gewählt aus A, I, L, M, S, T und V sind, an der Position 389 gewählt aus I, L, M und V sind, an den Positionen 418, 430, 447 und 473 gewählt aus A, G und S sind, an der Position 424 gewählt aus A, D, E, G, K, R und S sind, an der Position 436 gewählt aus A, G, S und T sind, an der Position 440 gewählt aus A, G, S und T sind, an der Position 465 gewählt aus A, G, H, N, Q, S und T sind, an der Position 469 gewählt aus D, E, K und R sind und/oder an der Position 487 gewählt aus N, D, Q, H und S vorgenommen sind, wobei die ursprünglich an den genannten Stellen vorhandenen Aminosäuren jedenfalls ausgetauscht sind.

Die Bezeichnung "Carboxylesterase" bezieht sich auf alle Enzyme, Polypeptide bzw. Polypeptidvarianten, die in der Lage sind Carboxylesterverbindungen mit Hilfe von Wasser in die entsprechenden Alkoholverbindungen und Carbonsäureverbindungen zu spalten. Die Bezeichnung "Fusariumtoxin Carboxylesterase" bezieht sich auf alle Enzyme, Polypeptide bzw. Polypeptidvarianten, die in der Lage sind wenigstens ein Fusariumtoxin zu hydrolysieren, indem von diesem wenigstens eine Tricarballylsäure (Propan-1,2,3-tricarbonsäure) hydrolytisch abgespalten wird. Eine hierin beschriebene "Fusariumtoxin spaltende Eigenschaft", bezieht sich auf die Fähigkeit wenigstens einen Tricarballylsäurerest von wenigstens einem Fusariumtoxin, insbesondere von FA1-2, FB1-4, FC1-2, FC4, HFC1, AAL-TA1-2 und AAL-TB1-2 oder Derivaten bzw. Abbauprodukten davon, hydrolytisch abzuspalten.

Die im Nachfolgenden verwendeten Ausdrücke sind der Fachsprache entnommen und werden jeweils, außer es ist etwas anderes angeführt, in den herkömmlichen Bedeutungen verwendet. So bezieht sich der Ausdruck "Polynukleotid" auf jegliche Arten von genetischem Material aller Längen und Sequenzen, wie z.B. Einzelstrang und Doppelstrang DNS- und RNS- Moleküle, inklusive regulatorischer Elemente, Strukturelemente, Gruppen von Genen, Plasmiden, gesamte Genome und Fragmente davon. Die Bezeichnung "Polypeptid" umfasst Proteine, wie beispielsweise Enzyme, Antikörper sowie Polypeptide mit bis zu 500 Aminosäuren, wie beispielsweise Peptidinhibitoren, Domänen von Proteinen aber auch kurze Polypeptide mit geringen Sequenzlängen, z.B. weniger als 10 Aminosäuren, wie Rezeptoren, Liganden, Peptidhormone, Tags und dgl. Die Bezeichnung "Position" in einem Polynukleotid oder Polypeptid bezieht sich auf eine einzelne, spezifische Base oder Aminosäure in der Sequenz des Polynukleotids oder des Polypeptids. Die Namen der Aminosäuren werden mit den dem Fachmann geläufigen Einbuchstabencodes oder dem Dreibuchstabencodes abgekürzt.

Die Bezeichnung "Unit" oder "U" bezieht sich auf das Maß der katalytischen Aktivität eines Enzyms, Polypeptids bzw. einer Polypeptidvariante und wird definiert als die Anzahl an Mikromol (µmol) Substrat, hier Fumonisin B1, die in einer Minute unter definierten Bedingungen umgesetzt bzw. gespalten werden. Wird z.B. in 15 min. von 60 µmol FB1 wenigstens ein Tricarballylsäurerest abgespalten, so entspricht dies einer katalytischen Aktivität von 4 Units. Für die FB1 Spaltung sind die Reaktionsbedingungen wie folgt definiert: Die Umsetzungsreaktion wird in 20 mM Tris-HCl Puffer (pH 8,0) mit 0,1 mg/ml Rinderserumalbumin bei einer Temperatur von 30 °C für 30 min. durchgeführt. Die Substratkonzentration in der Reaktion beträgt 100 µM FB1.

Als "katalytische Aktivität", "katalytische Enzymaktivität" oder "Aktivität" einer Enzym-, oder Polypeptidlösung oder Lösung einer Polypeptidvariante wird die enzymatische Konzentration der Enzym-, oder Polypeptidlösung oder Lösung der Polypeptidvariante definiert, die in Units pro Milliliter Lösung angegeben wird.

Die Bezeichnung "spezifische Aktivität" wird als katalytische Aktivität pro Milligramm Enzym-, Polypeptid oder Polypeptidvariante definiert und wird aus dem Verhältnis der katalytischen Aktivität einer Enzymlösung und der Massenkonzentration (Masse pro Volumeneinheit) des Enzyms in dieser Lösung berechnet. Hat eine Enzymlösung z.B. eine katalytische Aktivität von 50 U/ml und eine Massenkonzentration von 1 mg/ml so beträgt die spezifische Aktivität 50 U/mg.

Die Bezeichnung "Temperaturstabilität" bezieht sich auf die Eigenschaft von Enzymen, Polypeptiden bzw. Polypeptidvarianten ihre katalytische Aktivität nach einer zeitlich begrenzten Exposition an erhöhte Temperatur (Vorinkubation) beizubehalten. Die Temperaturstabilität wird bestimmt, indem die Aktivität einer Enzym-, oder Polypeptidlösung oder Lösung einer Polypeptidvariante nach 5 minütiger Hitzebehandlung und ohne Hitzebehandlung bei gleichen und definierten Bedingungen gemessen und verglichen wird. Die Temperaturstabilität ist somit ein Maß für die Widerstandsfähigkeit von Enzymen gegenüber zeitlich befristeter Hitzeeinwirkung. Die Temperatur, bei der die Restaktivität des hitzebehandelten Enzyms im Vergleich zur nicht-hitzebehandelten 100 %-Kontrolle 50 % beträgt, ist das Maß für die Temperaturstabilität und wird mit T (50 %) abgekürzt. Beträgt beispielsweise die Aktivität einer Enzymlösung ohne Vorinkubation 50 U/ml und nach 5 minütiger Vorinkubation bei 50 °C 25 U/ml, so ist Temperaturstabilität des Enzyms 50 °C bzw. ist das Enzym bis 50 °C temperaturstabil. Die Steigerungen von T (50 %) von Polypeptidvarianten gegenüber dem parentalen Polypeptid der SEQ ID-Nr. 1 ist als ein Maß für die gesteigerte Temperaturstabilität definiert und kann relativ als Prozentwert oder absolut in Grad Celsius angegeben werden.

Die Begriff "Temperaturaktivität" definiert diejenige Temperatur bei der das Enzym, Polypeptid bzw. die Polypeptidvariante die höchste Aktivität aufweist, wobei die katalytische Aktivität über einen Zeitraum von 30 min. gemessen wird.

Die Ausdrücke "Polypeptidvariante" oder "Variante" beziehen sich insbesondere auf Polypeptidsequenzen, die im Vergleich zur SEQ ID-Nr. 46 wenigstens eine Aminosäuresubstitution besitzen, wobei die enzymatische Funktion, d.h. die Fusariumtoxin spaltende Eigenschaft, beibehalten bleibt. Des Weiteren kann eine "Polypeptidvariante" zusätzlich Aminosäureinsertionen oder -deletionen, insbesondere eine C- oder N-terminal verlängerte oder verkürzte Sequenz, relativ zur Polypeptidsequenz der SEQ ID-Nr. 46, besitzen. Eine enzymatische Funktion ist dann im Wesentlichen beibehalten, wenn der enzymatische Reaktionsmechanismus unverändert bleibt, d.h. das Fusariumtoxin an derselben Stelle hydrolysiert wird und die spezifische Aktivität der Variante wenigstens 10 %, bevorzugt wenigstens 50 %, bevorzugter wenigstens 90 %, insbesondere aber >100 % bezogen auf das ursprüngliche, parentale Polypeptid der SEQ ID-Nr. 46, beträgt.

Substitutionen von Aminosäuren an definierten Positionen werden mit folgender Nomenklatur beschrieben: ursprüngliche Aminosäure; Position; neue Aminosäure. Wird z.B. ein Prolin an der Position 134 durch Glycin ersetzt wird dies durch Pro134Gly oder P134G angezeigt. Multiple Mutationen werden durch ein Plus oder einen Schrägstrich getrennt. Wird z.B. Prolin an der Position 134 durch Glycin und Arginin an der Position 136 durch Lysin ersetzt wird dies durch Pro134Gly+Arg136Lys oder Pro134Gly/Arg136Lys oder P134G+R136K oder P134G/R136K angezeigt. Wird eine Aminosäure an einer Position durch zwei oder mehrere alternative Aminosäuren ersetzt, werden die alternativen Aminosäuren durch einen Beistrich oder Schrägstrich getrennt. Wird z.B. Prolin an der Position 134 nicht nur durch Glycin, sondern auch durch Serin, Valin und Methionin ersetzt, wird dies durch Pro134Gly,Ser,Val,Met oder Pro134Gly/SerNal/Met oder P134G,S,V,M oder P134G/S/V/M angezeigt. Wird eine Substitution bzw. ein Austausch einer Aminosäure an einer bestimmten Position nicht näher definiert, ist dies so zu interpretieren, dass diese Aminosäure durch alle anderen Aminosäuren ersetzt werden kann. Wird z.B. eine Mutation von Prolin an Position 134 nicht näher definiert, so kann das Prolin entweder durch eine der folgenden Aminosäuren A, R, N, D, C, Q, E, G, H, I, L, K, M, F, S, T, W, Y oder V ersetzt werden.

Die Bezeichnung "Sequenzidentität" bezieht sich auf eine prozentuale Sequenzidentität. Für Aminosäuresequenzen und Nukleotidsequenzen kann die Sequenzidentität visuell bestimmt, bevorzugt aber mit einem Computerprogramm errechnet werden. Als Referenzsequenz wird die Aminosäuresequenz mit der Sequenz ID-Nr. 1 definiert. Der Sequenzvergleich wird auch innerhalb von Sequenzabschnitten durchgeführt, wobei als Abschnitt eine durchgängige Sequenz der Referenzsequenz zu verstehen ist. Die Länge der Sequenzabschnitte beträgt für Nukleotidsequenzen normalerweise 18 bis 600, bevorzugt 45 bis 200, bevorzugter 100 bis 150 Nukleotide. Die Länge der Sequenzabschnitte beträgt für Peptidsequenzen normalerweise 3 bis 200, bevorzugter 15 bis 65, am bevorzugtesten 30 bis 50 Aminosäuren. Es gibt eine Vielzahl von käuflichen oder kostenfrei verfügbaren bioinformatischen Programmen, die zur Homologiebestimmung herangezogen werden können und kontinuierlich weiterentwickelt werden. Beispiele hierfür sind GCG Wisconsin Bestfit package (Devereux et al. 1984), BLAST (Altschul et al. 1990) oder BLAST 2 (Tatusova und Madden 1999). Aufgrund der unterschiedlichen Einstellungsmöglichkeiten dieser Algorithmen ist es möglich, dass diese bei gleichen Inputsequenzen zu unterschiedlichen Ergebnissen kommen. Daher ist es notwendig den Suchalgorithmus und die dazugehörigen Einstellung zu definieren. Im vorliegenden Fall wurde die Sequenzidentität mit Hilfe des Programms NCBI BLAST (Basic Local Alignment Search Tool), insbesondere mit BLASTP für Polypeptide und BLASTN für Polynukleotide, welche auf der Homepage des "National Center for Biotechnology Information" (NCBI; http://www.ncbi.nlm.nih.gov/) zur Verfügung gestellt werden, durchgeführt. Damit ist es möglich zwei oder mehrere Sequenzen miteinander nach dem Algorithmus von Altschul et al., 1997 (Nucleic Acids Res., 25:3389-3402) zu vergleichen. Hierbei wurden die Programme in der Version vom 12. Aug. 2014 verwendet. Als Programmeinstellungen wurden die Basiseinstellungen herangezogen, insbesondere für den Aminosäuresequenzvergleich: "max target sequence" = 100; "expected treshold" = 10; "word size" = 3; "matrix" = BLOSOM62; "gap costs" = "Existence: 11; Extention: 1"; "computational adjustment" = "Conditional compositional score matrix adjustment "; sowie für den Nukleotidsequenzvergleich Word Size: 11; Expect value: 10; Gap costs: Existence = 5, Extension = 2; Filter = low complexity activated; Match/Mismatch Scores: 2,-3; Filter String: L; m.

Gemäß einer bevorzugten Weiterbildung der Erfindung ist die Aminosäuresubstitution aus der Gruppe 10Q, 33E, 66D, 107E, 140P, 144M, 149F, 151R, 157Y, 1991, 266S, 267P, 270F, 272H, 275E, 275A, 280D, 280P, 284T, 284P, 286P, 286R, 293E, 302I, 312F, 329F, 332E, 360V, 363T, 364H, 364L, 365I, 367H, 371V, 371M, 372F, 377V, 389L, 391V, 394P, 418A, 419V, 424A, 424K, 427V, 429P, 430A, 436A, 436S, 440G, 440S, 443T, 447A, 453R, 455S, 456Q, 457T, 462Y, 463D, 464I, 465H, 465S, 465G, 469K, 473A, 478D, 487N und 490P gewählt. Indem die Polypeptidvarianten wenigstens eine derartige Substitution aufweisen, kann die Temperaturstabilität bzw. Temperaturaktivität noch gezielter gesteigert werden, wobei zusätzlich andere Enzymparameter, wie spezifische Aktivität, pH-Stabilität oder pH-Aktivität ebenfalls verbessert werden können, welche jedoch mindestens die Werte der Fusariumtoxin Carboxylesterase mit der SEQ ID-Nr. 1 aufweisen.

Gemäß einer Weiterbildung der Erfindung weisen die Polypeptidvarianten an wenigstens einer Position gewählt aus der Gruppe bestehend aus 66, 199, 302, 377, 394, 424, 430 und 463 eine Aminosäuresubstitution und eine gegenüber der Fusariumtoxin Carboxylesterase mit der SEQ ID-Nr. 1 um wenigstens 6 % gesteigerte Temperaturstabilität auf. Mittels derartiger Polypeptidvarianten ist es möglich während technologischer Prozesse mit erhöhter Temperatur wie z.B. der Produktion von Teigwaren und anderen Produkten aus Mais, wie Polenta, Popcorn, Corn Flakes, Maisbrot oder Tortillas, sowie Verflüssigungsprozessen von Stärke, Saccharifizierungsprozessen oder Fermentationsprozessen, wie z.B. der Maisch- oder Gärprozess insbesondere in der Bioethanolherstellung Fusariumtoxine zu spalten.

Gemäß einer bevorzugten Weiterbildung der Erfindung ist die Aminosäuresubstitution aus der Gruppe 66D, 1991, 302I, 377V, 394P, 424A, 430A und 463D gewählt. Mit Hilfe einer derartigen Substitution kann die Temperaturstabilität sowie die Temperaturaktivität der Polypeptidvarianten relativ zur Fusariumtoxin Carboxylesterase der SEQ ID-Nr. 1 um wenigstens 3°C gesteigert werden.

Gemäß einer Weiterbildung der Erfindung weisen die Polypeptidvarianten wenigstens an zwei, insbesondere drei Positionen der Aminosäuresequenz gewählt aus der Gruppe bestehend aus 10, 33, 66, 107, 140, 144, 149, 151, 157, 199, 266, 267, 270, 272, 275, 280, 284, 286, 293, 302, 312, 329, 332, 360, 363, 364, 365, 367, 371, 372, 377, 389, 391, 394, 418, 419, 424, 427, 429, 430, 436, 440, 443, 447, 453, 455, 456, 457, 462, 463, 464, 465, 469, 473, 478, 487 und 490 eine Aminosäuresubstitution auf wobei die Aminosäuresubstitutionen aus der Gruppe 10Q, 66D, 144M, 151R, 1991, 266S, 267P, 272H, 275E, 275A, 280D, 284T, 286P, 286R, 293E, 302I, 360V, 363T, 364H, 364L, 365I, 367H, 371V, 371M, 372F, 377V, 389L, 391V, 394P, 418A, 419V, 424A, 424K, 427V, 429P, 430A, 436A, 436S, 440G, 440S, 443T, 447A, 453R, 455S, 456Q, 457T, 462Y, 463D, 464I, 465H, 465S, 465G, 469K, 473A, 478D, 487N und 490P gewählt sind und besitzen gegenüber der Fusariumtoxin Carboxylesterase der SEQ ID-Nr. 1 eine um wenigstens 15 % gesteigerte Temperaturstabilität. Durch Substitution mehrerer Aminosäuren hat sich überraschenderweise gezeigt, dass deren positiver Effekt auf die Temperaturstabilität ungefähr addiert wird, wobei durch das Einfügen von wenigstens drei von den ursprünglich in der Sequenz vorhandenen Aminosäuren verschiedenen Aminosäuren die Temperaturstabilität um mehr als 7 °C gesteigert wird. Eine derartige Steigerung ist ausreichend um die Enzyme z.B. beim Maischerasten bei etwa 55 °C in der Bioethanolherstellung bzw. beim Pelletieren von Futtermitteln bei moderaten Temperaturen von ca. 65 bis 70 °C einzusetzen.

Gemäß einer Weiterbildung der Erfindung enthält die Aminosäuresequenz der Polypeptid-varianten Kombinationen von mehreren Aminosäuresubstitutionen, wobei die Kombinationen der Positionen aus der Gruppe
66/199/302/394/424/430, 66/199/302/377/394/424/430, 66/199/302/377/394/424/430/463, 66/144/199/302/360/372/377/394/424/430/443/463, 199/302/377/394/424/430/463, 66/199/302/377/394, 66/199/302/364/377/394/424/430/463, 66/199/302/377/394/424/430/463/465, 66/199/302/377/394/424/430/440/463, 66/199/302/377/394/424/430/447/463, 66/199/302/377/394/418/424/430/463, 66/199/302/377/394/424/436/430/463, 66/199/302/364/377/394/424/430/463, 66/199/302/377/394/424/430/463/490, 66/199/302/377/394/424/430/463/469, 66/199/302/377/389/394/424/430/463, 66/199/302/377/394/424/430/463/465, 66/199/302/377/394/424/430/463/464, 66/199/302/377/394/424/430/463/465, 66/199/302/377/394/424/430/440/463, 66/199/302/377/394/424/430/457/463, 66/199/302/377/394/424/430/436/463, 66/199/302/363/371/377/394/424/430/463, 66/199/302/377/394/424/430/447/453/463, 66/199/302/377/394/424/430/456/462/463, 66/199/302/377/394/419/424/427/430/463, 66/199/302/365/377/394/424/430/463/487 und 66/199/302/371/377/394/424/430/463/487 gewählt sind. Derart substituierte Polypeptid-varianten weisen gegenüber der Fusariumtoxin Carboxylesterase der SEQ ID-Nr. 1 eine um wenigstens 25 % gesteigerte Temperaturstabilität auf. Durch derartige Kombinationen an Substitutionen ist es nicht nur möglich die Temperaturstabilität sondern auch die Temperaturaktivität der Polypeptidvarianten weiter zu steigern.

Gemäß einer bevorzugten Weiterbildung der Erfindung sind die Aminosäuresequenzen der Polypeptidvarianten gewählt aus der Gruppe der SEQ ID-Nrn. 2 bis 29. Derartige Polypeptidvarianten besitzen gegenüber dem Enzym der SEQ ID-Nr. 1 eine gesteigerte Temperaturstabilität von mindestens 11 °C, bevorzugt von mindestens 13 °C und noch bevorzugter von mindestens 15 °C, wodurch eine Aktivität des jeweiligen Polypeptids und dadurch eine Detoxifizierung von Fusariumtoxinen während oder nach technologischen Prozessen mit erhöhter Temperaturbelastung, wie z.B. einem Maischerasten bei 65 °C in der Bioethanolherstellung oder beim Pelletieren bei Temperaturen um 75 - 80 °C, gewährleistet ist.

Gemäß einer Weiterbildung der Erfindung enthält jede Aminosäuresequenz der Polypeptidvarianten Kombinationen von mehreren Aminosäuresubstitutionen, wobei die Kombinationen der Positionen aus der Gruppe 66/99/302/364/377/389/394/419/424/427/430/447/463/465/469, 66/199/302/377/389/394/419/424/427/430/447/463/465/469, 66/199/302/363/364/371/377/389/394/419/424/427/430/447/463/464/465/469, 66/199/302/363/371/377/389/394/419/424/427/430/447/463/464/465/469, 66/199/302/364/367/371/377/389/394/418/419/424/427/430/436/440/447/463/464/465/ 469/490, 66/199/302/367/371/377/389/394/418/419/424/427/430/436/440/447/463/464/465/469/ 490,
66/199/302/363/367/371/377/394/424/430/463/490, 66/199/302/377/394/418/419/424/427/430/436/440/447/463, 66/199/302/377/389/394/424/430/457/463/464/465/469, 66/199/302/363/371/377/389/394/419/424/427/430/440/447/457/463/464/469/490, 66/199/302/377/394/424/430/463/447/490/469/465, 66/199/302/377/394/424/430/463/490/469/465/419/427/371/487, 66/199/302/371/377/394/419/424/427/430/447/453/463/465/469/487/490, 66/199/302/367/371/377/389/394/418/419/424/427/429/430/436/440/447/457/463/464/46 5/469/490, 66/199/302/371/377/389/394/419/424/427/430/436/447/453/456/462/463/465/469/490/ 487
und 66/199/302/367/371/377/389/394/418/419/424/427/429/430/436/440/447/453/456/457/46 2/463/464/465/469/487/490 gewählt sind. Diese Polypeptidvarianten weisen gegenüber der Fusariumtoxin Carboxylesterase der SEQ ID-Nr. 1 eine um wenigstens 40 % gesteigerte Temperaturstabilität sowie eine erhöhte Temperaturaktivität auf, wodurch diese in einer Vielzahl von höhere Temperaturen erfordernden Verfahren einsetzbar sind.

Gemäß einer bevorzugten Weiterbildung der Erfindung sind die Aminosäuresequenzen der Polypeptidvarianten aus der Gruppe der SEQ ID-Nrn. 30 bis 45 gewählt. Derartige Polypeptidvarianten besitzen gegenüber dem Enzym der SEQ ID-Nr. 1 eine gesteigerte Temperaturstabilität von mindestens 18 °C, bevorzugt von mindestens 22 °C und bevorzugter von mindestens 27 °C, wodurch eine Aktivität der Polypeptide und dadurch eine Detoxifizierung von Fusariumtoxinen während oder nach technologischen Prozessen mit hoher Temperaturbelastung, wie z.B. beim Pelletieren bei Temperaturen von über 80 °C, insbesondere von über 85 - 90 °C, gewährleistet ist. Das Pelletieren bei hohen Temperaturen von ca. 90 °C hat insbesondere in der Geflügelindustrie große Bedeutung, um eine ausreichende Reduktion der Salmonellenbelastung des Futtermittels zu gewährleisten. Die Bezeichnung "konservierte Mutation" bezieht sich auf die Substitution von Aminosäuren durch andere Aminosäuren, die vom Fachmann als konserviert angesehen werden, d.h. die ähnliche spezifische Eigenschaften besitzen beziehungsweise, dass Eigenschaften der Aminosäure erhalten, d.h. konserviert bleiben. Spezifische Eigenschaften von Aminosäuren sind beispielsweise deren Größe, Polarität, Hydrophobizität, Ladung oder pKs-Wert. Aminosäuren können aufgrund ihrer Eigenschaften in Gruppen eingeteilt und die Gruppen in dem Venn Diagramm dargestellt werden. Aminosäuren aus derselben Gruppe und bevorzugt aus derselben Untergruppe können gegenseitig substituiert werden. Die Einteilung der Aminosäuren nach den Eigenschaften Hydrophobizität, Polarität und Größe in Gruppen und Untergruppen ist Taylor W. R. (1986) zu entnehmen. Unter einer konservierten Mutation wird z.B. eine Substitution einer sauren Aminosäure gegen eine andere saure Aminosäure, eine basische Aminosäure gegen eine andere basische Aminosäure, eine polare gegen eine andere polare Aminosäure und dergleichen verstanden. Insbesondere können die Polypeptidvarianten zusätzlich wenigstens eine konservative Aminosäuresubstitution an wenigstens einer Position enthalten, wobei die konservative Aminosäuresubstitution aus der Gruppe von Substitutionen G zu A, A zu G/S, V zu I/L/A/T/S, I zu V/L/M, L zu I/M/V, M zu L/I/V, P zu A/S/N, F zu Y/W/H, Y zu F/W/H, W zu Y/F/H, R zu K/E/D, K zu R/E/D, H zu Q/N/S, D zu N/E/K/R/ Q, E zu Q/D/K/R/N, S zu T/A, T zu S/V/A, C zu S/T/A, N zu D/Q/H/S und Q zu E/N/H/K/R gewählt ist.

Führt eine Substitution in einer erfindungsgemäßen Polypeptidvariante an einer definierten Position dazu, dass z.B. eine polare Aminosäure wie Asp durch eine hydrophobe Aminosäure wie Ala ersetzt wird, so werden unter konservierten Mutationen auch alle Mutationen verstanden, die zu einer anderen hydrophoben Aminosäure (z.B. Glycin, Leucin, Phenylalanin, Valin) an der Position führen. Diese weiteren Polypeptidvarianten, enthaltend eine alternative konservierte Mutation sind hier ebenfalls umfasst.

Die vorliegende Erfindung zielt des Weiteren darauf ab, Polynukleotide zur Verfügung zu stellen, die für eine Fusariumtoxin spaltende Polypeptidvariante einer Fusariumtoxin Carboxylesterase der SEQ ID-Nr. 1 codieren, mit welchen es gelingt wenigstens ein Fusariumtoxin sauerstoffunabhängig und cofaktorfrei in nicht bzw. weniger toxische Produkte zu spalten und die eine erhöhte Temperaturstabilität im Vergleich zur Fusariumtoxin Carboxylesterase der SEQ ID-Nr. 1 aufweisen.

Zur Lösung dieser Aufgabe ist die Erfindung dadurch gekennzeichnet, dass das Polynukleotid eine Nukleotidsequenz besitzt, die für eine Fusariumtoxin spaltende Polypeptidvariante einer Fusariumtoxin Carboxylesterase mit der Aminosäuresequenz der SEQ ID-Nr. 1 codiert, wobei die Polypeptidvarianten eine Aminosäuresequenz mit wenigstens 70 % Sequenzidentität zur Aminosäuresequenz mit der SEQ ID-Nr. 1 aufweisen und dass die Polypeptidvarianten an wenigstens einer Position gewählt aus der Gruppe bestehend aus 10, 33, 66, 107, 140, 144, 149, 151, 157, 199, 266, 267, 270, 272, 275, 280, 284, 286, 293, 302, 312, 329, 332, 360, 363, 364, 365, 367, 371, 372, 377, 389, 391, 394, 418, 419, 424, 427, 429, 430, 436, 440, 443, 447, 453, 455, 456, 457, 462, 463, 464, 465, 469, 473, 478, 487 und eine Aminosäuresubstitutionen aufweisen, insbesondere jedoch die Substitutionen 10Q, 33E, 66D, 107E, 140P, 144M, 149F, 151R, 157Y, 1991, 266S, 267P, 270F, 272H, 275E, 275A, 280D, 280P, 284T, 284P, 286P, 286R, 293E, 302I, 312F, 329F, 332E, 360V, 363T, 364H, 364L, 365I, 367H, 371V, 371M, 372F, 377V, 389L, 391V, 394P, 418A, 419V, 424A, 424K, 427V, 429P, 430A, 436A, 436S, 440G, 440S, 443T, 447A, 453R, 455S, 456Q, 457T, 462Y, 463D, 464I, 465H, 465S, 465G, 469K, 473A, 478D, 487N und 490P oder Kombinationen davon aufweisen. Mit einem derartigen isolierten Polynukleotid kann mit Hilfe eines Expressionsvektors eine transgene Wirtszelle zur Produktion der erfindungsgemäßen Polypeptidvarianten hergestellt werden.

Die Bezeichnung "Expressionsvektor" bezieht sich auf ein DNS Konstrukt, das in der Lage ist, *in vivo* oder *in vitro* ein Gen zu exprimieren. Im Besonderen werden darunter DNS Konstrukte verstanden, die geeignet sind, die das Polypeptid codierende Nukleotidsequenz in die Wirtszelle zu transferieren, sodass diese in das Genom integriert werden oder frei im extrachromosomalen Raum vorliegen und die Polypeptid codierende Nukleotidsequenz intrazellulär zu exprimieren und gegebenenfalls das Polypeptid aus der Zelle auszuschleusen.

Die Bezeichnung "Wirtszelle" bezieht sich auf alle Zellen, die entweder eine zu exprimierende Nukleotidsequenz oder einen Expressionsvektor enthalten und in der Lage sind, ein erfindungsgemäßes Enzym bzw. Polypeptid herzustellen. Im Besonderen werden darunter prokaryotische und/oder eukaryotische Zellen verstanden, bevorzugt *P. pastoris, E. coli, Bacillus subtilis, Streptomyces, Hansenula, Trichoderma, Lactobacillus, Aspergillus,* Pflanzenzellen und/oder Sporen von *Bacillus, Trichoderma* oder *Aspergillus.* Die hier verwendete Bezeichnung *Pichia pastoris* ist synonym zur Bezeichnung *Komagataella pastoris,* wobei *Pichia pastoris* die ältere und *Komagataella pastoris* die systematisch neuere Bezeichnung darstellt (Yamada et al., 1995).

Die vorliegende Erfindung zielt des Weiteren darauf ab, einen Fusariumtoxin spaltenden Zusatzstoff zur Verfügung zu stellen, der wenigstens eine Fusariumtoxin spaltende Polypeptidvariante einer Fusariumtoxin Carboxylesterase mit der Aminosäuresequenz der SEQ ID-Nr. 46 enthält, wobei die jeweilige Polypeptidvariante wenigstens ein Fusariumtoxin sauerstoffunabhängig und cofaktorfrei in nicht bzw. weniger toxische Produkte spaltet und eine erhöhte Temperaturstabilität im Vergleich zur Fusariumtoxin Carboxylesterase der SEQ ID-Nr. 46 aufweist.

Zur Lösung dieser Aufgabe ist die Erfindung dadurch gekennzeichnet, dass der Fusariumtoxin spaltender Zusatzstoff wenigstens eine erfindungsgemäße Polypeptidvariante einer Fusariumtoxin Carboxylesterase und gegebenenfalls wenigstens einen Hilfsstoff enthält. Indem ein derartiger Zusatzstoff zu Fusariumtoxin kontaminierten Futtermitteln zugesetzt wird, gelingt es die Fusariumtoxine zu detoxifiziern, was durch eine Reduktion der Sphinganin zu Sphingosin Verhältnisse in Plasma und/oder Niere und/oder Lunge und/oder Leber signifikant des mit den Zusatzstoff gefütterten Subjekts gemessen werden kann.

Das Verhältnis von Sphinganin zu Sphingosin in unterschiedlichen Organen sowie im Plasma von Tieren ist ein allgemein akzeptierter und sensitiver Biomarker für den toxischen Effekt von Fusariumtoxinen, insbesondere von FB1. Durch Fusariumtoxine verursache Störungen des Sphingolipid Stoffwechsels werden unter anderem mit Gehirnerkrankung von Pferden oder Lungenödeme bei Schweinen in Verbindung gebracht. Die Relevanz des Verhältnisses von Sphinganin zu Sphingosin als Biomarker sowie dessen analytische Messung ist in Grenier et al. (Biochem. Pharmaceuticals Vol. 83 (2012) S. 1465-1473) sowie im EFSA Journal (2014; 12(5):3667) beschrieben.

Ge4mäß einer Weiterbildung der Erfindung ist der Zusatzstoff so ausgebildet, dass der Hilfsstoff gewählt ist aus Gruppe von inerten Trägern, Vitaminen, Mineralstoffen, phytogenen Substanzen, Enzymen und weiteren Komponenten zur Detoxifizierung von Mykotoxinen, wie Mykotoxin-abbauende Enzyme, insbesondere Aflatoxin Oxidasen, Ergotamin Hydrolasen, Ergotamin Amidasen, Zearalenon Esterasen, Zearalenon Lactonasen, Zearalenon Hydrolasen, Ochratoxin Amidasen, Fumonisin Aminotransferasen, Aminopolyol Aminoxidasen, Deoxynivalenol Epoxidhydrolasen, Deoxynivalenol Dehydrogenasen, Deoxynivalenol Oxidasen, Trichothecene Dehydrogenasen, Trichothecene Oxidasen; Mykotoxin-abbauenden Mikroorganismen; und Mykotoxin-bindenden Stoffen, beispielsweise mikrobielle Zellwände oder anorganische Materialien, wie Bentonite. Durch den Einsatz eines derartigen Zusatzstoffes kann beispielsweise in Futter- oder Nahrungsmitteln sichergestellt werden, dass die gegebenenfalls enthaltenen Mengen an Fusariumtoxinen mit Sicherheit soweit gespalten, insbesondere detoxifiziert werden, dass eine schädliche Wirkung auf den Organismus des dieses Futter- oder Nahrungsmittel aufnehmenden Subjekts ausbleibt.

Weitere Einsatzbereiche der Erfindung sind Zusatzstoffe, die neben wenigstens einer erfindungsgemäßen Polypeptidvariante zusätzlich wenigstens ein Enzym enthalten, das beispielsweise am Abbau von Proteinen beteiligt ist, wie z.B. Proteasen, oder das beim Metabolismus von Stärke oder Faser oder Fett oder Glycogen beteiligt ist, wie z.B. Amylase, Cellulase oder Glucanase, sowie beispielsweise Hydrolasen, lipolytische Enzyme, Mannosidasen, Oxidasen, Oxidoreductasen, Phytasen oder Xylanasen.

Die vorliegende Erfindung zielt des Weiteren auf eine Verwendung eines erfindungsgemäßen Zusatzstoffes zur Spaltung von wenigstens einem Fusariumtoxin in, insbesondere pelletierten Nahrungs- oder Futtermitteln, insbesondere für Schweine, Geflügel, Rinder, Pferde, Fische oder Aquakultur ab. Als Nahrungs- oder Futtermittel können alle für die menschliche oder tierische Ernährung, insbesondere auch für Haustiere, Schafe oder Ziegen, geeigneten Nahrungs- oder Futtermittel, insbesondere auch Trockenschlempe, eingesetzt werden.

Die vorliegende Erfindung zielt des Weiteren auf eine Verwendung eines erfindungsgemäßen Zusatzstoffes zur Spaltung von wenigstens einem Fusariumtoxin in einem Prozess, insbesondere bei Temperaturen von mindestens 50 °C, zur Herstellung oder Verarbeitung von Nahrungs- oder Futtermitteln ab. Durch eine derartige Verwendung des Zusatzstoffes wird eine Entgiftung von Fusariumtoxinen z.B. während lebensmitteltechnologischen Prozessen bei denen eine Behandlung bei erhöhter Temperatur wichtig ist, wie etwa bei der Verarbeitung von Mais oder Getreide, bei Verflüssigungsprozessen von Stärke, bei Saccharifizierungsprozessen oder bei Fermentationsprozessen, wie z.B. dem Maische- oder Gärprozess insbesondere in der Bioethanolherstellung gewährleistet. Dadurch kann sichergestellt werden, dass in keinem aus derartigen Verfahren stammenden Produkt, wie z.B. Futtermittelpellets, Teigwaren, Polenta, Popcorn, Corn Flakes, Maisbrot, Tortillas, Trockenschlempe oder Stärke, relevante, insbesondere gesundheitsschädliche Mengen an Fusariumtoxinen intakt bleiben.

Die vorliegende Erfindung zielt des Weiteren auf eine Verwendung von wenigstens einer erfindungsgemäßen Polypeptidvariante oder von wenigstens einem erfindungsgemäßen Zusatzstoff zur Herstellung eines Präparats zur Prophylaxe und/oder zur Behandlung von Fusariumtoxin-Mykotoxikosen ab. Durch eine derartige Verwendung einer Polypeptidvariante oder eines Zusatzstoffes ist es im Falle der Prophylaxe möglich den Gesundheitszustand von Menschen oder Tieren trotz einer oraler Aufnahme von Fusariumtoxinen im Wesentlichen auf dem Niveau zu halten, das demjenigen ohne bzw. mit einer verringerten oralen Aufnahme von Fusariumtoxinen entspricht. Im Falle der Behandlung von Fusariumtoxin-Mykotoxikosen ist es möglich die Symptome einer solchen Erkrankung zu lindern und insbesondere das Verhältnis Sphinganin zu Sphingosin in Organen und/oder Plasma signifikant zu verbessern. Des Weiteren ist es möglich durch eine derartige Verwendung die Leistungsfähigkeit von Nutztieren, insbesondere die Futterverwertung und Gewichtszunahme zu verbessern und die Mortalitätsrate zu senken.

Die vorliegende Erfindung zielt des Weiteren darauf ab, ein Verfahren zur enzymatischen Spaltung von wenigstens einem Fusariumtoxin zu Verfügung zu stellen, mit dem wenigstens ein Fusariumtoxin mit einem Polypeptid sauerstoffunabhängig, spezifisch, sicher und zuverlässig in nicht bzw. weniger toxische Produkte hydrolytisch gespalten werden kann, wobei die hydrolytische Spaltung entweder während oder nach einer Temperaturbehandlung erfolgt.

Zur Lösung dieser Aufgabe ist das Verfahren so geführt, dass vom Fusariumtoxin durch eine erfindungsgemäße Polypeptidvariante oder einem erfindungsgemäßen Zusatzstoff wenigstens eine Tricarballylsäure hydrolytisch abgespalten wird. Hierbei wird das wenigstens eine Fusariumtoxin mit wenigstens einer erfindungsgemäßen Polypeptidvariante oder wenigstens einem erfindungsgemäßen Zusatzstoff vermischt, wenigstens eine Polypeptidvariante wird vom wenigstens einem Fusariumtoxin wenigstens eine Tricarballylsäure hydrolytisch abspalten wodurch das Fusariumtoxin detoxifiziert wird, wobei die Mischung aus der jeweiligen Polypeptidvariante und dem Fusariumtoxin einer Temperaturbehandlung von mindestens 50 °C, bevorzugt von mindestens 70 °C, unterworfen wird und die hydrolytische Spaltung entweder während oder nach der Temperaturbehandlung erfolgt.

In einer bevorzugten Weiterbildung der Erfindung ist das Verfahren so geführt, dass die Polypeptidvariante oder der Zusatzstoff mit einem mit wenigstens einem Fusariumtoxin kontaminierten Futter- oder Nahrungsmittel vermischt wird und die Temperaturbehandlung gegebenenfalls durch einen Pelletierprozess erfolgt. Dadurch wird sichergestellt, dass die im kontaminierten und gegebenenfalls pelletierten Futter- oder Nahrungsmittel enthaltene Fusariumtoxine gespalten werden, sobald die Mischung aus der Polypeptidvariante und dem Fusariumtoxin mit Feuchtigkeit in Kontakt gebracht wird. Bei feuchten Futter- oder Nahrungsmitteln, wie Maische oder Breien, wird die Hydrolyse der Fusariumtoxine vor der oralen Aufnahme im feuchten Futter- oder Nahrungsmittel stattfinden. Dadurch wird erreicht, dass die schädlichen Wirkungen von Fusariumtoxinen auf Mensch und Tier weitestgehend eliminiert oder zumindest reduziert werden. Als Feuchtigkeit wird hierbei die Anwesenheit von Wasser oder wasserhaltigen Flüssigkeiten verstanden, wobei darunter auch z.B. Speichel oder andere im Verdauungstrakt vorhandene Flüssigkeiten fallen. Als Verdauungstrakt wird die Mundhöhle, die Pharynx (Rachen), die Speiseröhre und der Magen-Darm-Trakt oder Äquivalente davon definiert, wobei unterschiedliche Bezeichnungen bei Tieren vorkommen können bzw. einzelne Bestandteile nicht im Verdauungstrakt von Tieren vorhanden sein können.

In einer bevorzugten Weiterbildung der Erfindung ist das Verfahren so geführt, dass die Polypeptidvariante pro Kilogramm Futter- oder Nahrungsmittel in einem Konzentrationsbereich von 5 U bis 500 U, bevorzugt von 10 U bis 300 U und bevorzugter von 15 U bis 100 eingesetzt wird. Durch Zugabe dieser Mengen der Polypeptidvariante gelingt es, in Abhängigkeit von der Konzentration der Fusariumtoxine, diese im Nahrungs- oder Futtermittel, insbesondere in Trockenschlempe, zu spalten und dadurch soweit zu detoxifizieren, dass wenigstens 70 %, bevorzugt wenigstens 80 %, insbesondere wenigstens 90 % von wenigstens einem Fusariumtoxin gespalten werden.

Sofern nicht explizit etwas anderes erwähnt wird, so sind singuläre Bezeichnungen wie beispielsweise "ein", "eine", "das", "die" oder "der" exemplarisch zu verstehen und beziehen eine Vielzahl von Möglichkeiten mit ein. Wird etwa auf "ein Gen", "ein Enzym" oder "eine Zelle" Bezug genommen, so ist immer die Mehrzahl gemeint.

Die Erfindung wird nachfolgend anhand von Beispielen näher erläutert.

### Polypeptidvarianten

### Beispiel 1: Modifikation, Klonierung und Expression von Polynukleotiden, die für Fusariumtoxin spaltende Polypeptide codieren

Aminosäuresubstitutionen, -insertionen oder -deletionen wurden durch Mutation der Nukleotidsequenzen mittels PCR unter Verwendung des "Quick-change Site-directed Mutagenesis Kits" (Stratagene) laut Anleitung durchgeführt. Alternativ dazu wurden auch komplette Nukleotidsequenzen synthetisch hergestellt (GeneArt). Die mittels PCR-Mutagenese generierten bzw. von GeneArt bezogenen Nukleotidsequenzen wurden mittels Standardmethoden in Expressionsvektoren zur Expression in *E. coli* oder *P. pastoris* integriert, in *E. coli* oder *P. pastoris* transformiert, sowie in *E. coli* oder *P. pastoris* exprimiert (J.M. Cregg, Pichia Protocols, second Edition, ISBN-10: 1588294293, 2007; J. Sambrook et al. 2012, Molecular Cloning, A Laboratory Manual 4th Edition, Cold Spring Harbor), wobei für diese Aufgabe auch jede andere geeignete Wirtszelle herangezogen werden kann.

Die Bezeichnung "Expressionsvektor" bezieht sich auf ein DNS Konstrukt das in der Lage ist *in vivo* oder *in vitro* ein Gen zu exprimieren. Im Besonderen werden darunter DNS Konstrukte verstanden, die geeignet sind, die das Polypeptid codierende Nukleotidsequenz in die Wirtszelle zu transferieren, um dort ins Genom zu integrieren oder frei im extrachromosomalen Raum vorzuliegen und die das Polypeptid codierende Nukleotidsequenz intrazellulär zu exprimieren und gegebenenfalls das Polypeptid aus der Zelle auszuschleusen. Die Bezeichnung "Wirtszelle" bezieht sich auf alle Zellen, die entweder eine zu exprimierende Nukleotidsequenz oder einen Expressionsvektor enthalten und in der Lage sind, ein erfindungsgemäßes Enzym bzw. Polypeptid herzustellen. Im Besonderen werden darunter prokaryotische und/oder eukaryotische Zellen verstanden, bevorzugt *P. pastoris, E. coli, Bacillus subtilis, Streptomyces, Hansenula, Trichoderma, Lactobacillus, Aspergillus,* Pflanzenzellen und/oder Sporen von *Bacillus, Trichoderma* oder *Aspergillus.* Das lösliche Zelllysat im Falle von *E. coli* beziehungsweise der Kulturüberstand im Falle von *P. pastoris* wurden zur Bestimmung der katalytischen Eigenschaften der Polypeptidvarianten herangezogen.

### Beispiel 2: Bestimmung der katalytischen Aktivität und spezifischen Aktivität von Fusariumtoxin abbauenden Polypeptiden

Die entsprechenden Gene, die für die Fusariumtoxin abbauenden Polypeptid-Varianten codieren, wurden mit Hilfe von Standardverfahren in *Escherichia coli* kloniert, intrazellulär exprimiert und anschließend mittels Ultraschall aufgeschlossen und zentrifugiert. Der klare Überstand wurde mit 20 mM Tris-HCl Puffer (pH 8,0) mit 0,1 mg/ml Rinderserumalbumin verdünnt (ca. 10⁻³ bis 10⁻⁵) und im FB1 Abbauansatz so eingesetzt, dass durch das Polypeptid 10 % bis 90 % der im Abbauansatz enthaltenen FB1 Menge abgebaut wurde. Zur Bestimmung der katalytischen Enzymaktivität wurden Versuche zur hydrolytischen Spaltung von Fumonisin B1 (FB1) durchgeführt, wobei der Versuchsansatz in einem 20 mM Tris-HCl Puffer (pH 8,0) mit 0,1 mg/ml Rinderserumalbumin und bei einer Temperatur von 30 °C für 30 min. durchgeführt wurde. Zusätzlich enthielt der Ansatz eine Substratkonzentration von 100 µM FB1 (Biopure Referenzsubstanzen GmbH Tulln, Austria, BRM 001007) und eines der zu testenden Polypeptide. Nach 30 min. Inkubation wurde zum Stoppen der Reaktion der Ansatz bei 99 °C für 5 min. hitzeinaktiviert.

Zur Bestimmung der enzymatischen Aktivität aus Futterproben müssen die Fusariumtoxin transformierenden Polypeptid-Varianten vor dem Versuch aus den Futterproben extrahiert werden. Dazu wurden 10 g Futter in 100 ml 20 mM Tris-HCl Puffer (pH 8,0) mit 0,1 mg/ml Rinderserumalbumin gelöst und 1 h bei 20 °C und 150 rpm geschüttelt. Danach wurden die Proben bei 4000 g für 15 min. zentrifugiert, und der klare Überstand wurde nach Bedarf verdünnt (10⁻² bis 10⁻³) und im FB1 Ansatz eingesetzt.

Die Quantifizierung von FB1 erfolgte mittels LC-MS (Flüssigkeitschromatographie - Massenspektrometrie) nach der Methode von Heinl et al. (J. of Biotechnology, 2010, 145, S. 120-129, 2.6.3. "Liquid chromatography-mass spectrometry"). Dazu wurde eine Kalibrierung mit FB1 Standards, die zusätzlich einen vollständigen ¹³C markierten, internen FB1 Standard enthielten (Biopure Referenzsubstanzen GmbH Tulln, Austria) herangezogen. Im Unterschied zu Heinl et al. (2010) wurde lediglich der Abbau von FB1 gemessen, um die katalytische Enzymaktivität der eingesetzten Polypeptidlösungen zu bestimmen. Die katalytische Enzymaktivität der eingesetzten Polypeptidlösungen wird in Units pro ml angegeben, wobei ein "Unit" definiert ist als die Reduktion von einem µmol FB1 pro Minute unter obigen Reaktionsbedingungen im Versuch.

Zur Bestimmung der spezifischen Aktivitäten wurden die Enzymkonzentrationen mittels quantitativem Westernblot oder ELISA bestimmt. Die spezifischen Enzymaktivitäten wurden berechnet, indem die Aktivitäten (Units) auf die eingesetzten Enzymmengen bezogen wurden und werden in Units pro mg angegeben.

### Beispiel 3: Temperaturstabilität von Fusariumtoxin abbauenden Polypeptiden

Die Expression und Quantifizierung der Fusariumtoxin abbauenden Polypeptide erfolgte wie in den Beispielen 1 und 2 beschrieben. Vor der Aktivitätsbestimmung wurde die Menge des Zellaufschlusses in mehrere Teile aufgeteilt (zu je ca. 60 µL). 2 bis 10 Teile wurden für 5 min. einer Hitzebehandlung in einem handelsüblichen PCR-Cycler (z.B. Eppendorf Mastercycler Gradient) unterzogen, wobei jeder Teil bei unterschiedlichen Temperaturen inkubiert wurde. Ein weiterer Teil des Zellaufschlusses, die 100 %-Kontrolle, wurde währenddessen auf Eis inkubiert. Im Anschluss an die Hitzebehandlung wurden alle Proben/Versuchsansätze für eine Minute bei 10 °C inkubiert, um die Temperaturen anzugleichen. Von den hitzebehandelten Proben sowie von der 100 %-Kontrolle wurde die enzymatische Aktivität, wie in Beispiel 2 beschrieben, bestimmt. Die verbleibende Aktivität nach Hitzebehandlung wird als Restaktivität bezeichnet. Die Temperatur, bei der die Restaktivität im Vergleich zur nicht-hitzebehandelten 100 %-Kontrolle 50 % beträgt, wird mit T (50 %) abgekürzt und ist das Maß für die Temperaturstabilität des Polypeptids.

Die Steigerungen von T (50 %), angegeben in Grad Celsius, von Polypeptidvarianten gegenüber dem Polypeptid mit der SEQ ID-Nr. 46 bzw. SEQ ID-Nr. 1 ist ein Maß für die gesteigerte Temperaturstabilität. Die Steigerung des T (50 %)-Werts kann in °C, aber auch als Prozentwert relativ zum T (50 %)-Wert des parentalen Polypeptids angegeben werden. Zur Veranschaulichung soll folgendes Beispiel dienen: Besäße das parentale Enzym eine katalytische Aktivität von 50 U/ml nach 5 minütiger Inkubation auf Eis und eine katalytische Aktivität von 25 U/ml nach 5 minütiger Inkubation bei 48 °C, so wäre die T (50 %) 48 °C. Hätte eine Polypeptidvariante eine T (50 %) von 51 °C, so wäre die relative Steigerung Temperaturstabilität (T (50 %)) 6,25. Dies ergibt sich aus der Differenz der beiden T (50 %) Werte von 3 °C dividiert durch den T (50 %)-Wert des parentalen Ausgangsenzyms von 48 °C multipliziert mit 100.

Zur Bestimmung der Temperaturstabilität kann statt der katalytischen Aktivität auch die spezifische Aktivität herangezogen werden.

Die Bestimmung der Temperaturstabilität kann auch mittels alternativer enzymatischer Assays durchgeführt werden und auch ohne die katalytische Aktivität der FB1 Umsetzung zu bestimmen. Wichtig hierbei ist, dass in den Ansätzen jedenfalls die gleichen Mengen von hitzebehandeltem Polypeptid und von der 100 %-Kontrolle eingesetzt werden, dies ist z.B. durch den Einsatz von gleichen Volumina an Zellaufschluss gegeben.

Zur Bestimmung der Temperaturstabilität können statt der katalytischen Aktivität auch die Messsignale von enzymatischen Abbauversuchen (z.B. MS-Signal, Extinktion, etc.) herangezogen werden. Ist das Messsignal direkt proportional zur enzymatischen Aktivität (z.B. Peakfläche von umgesetztem FB1), so ist der T (50 %)-Wert eines Polypeptids die Temperatur, bei der Wert des Messsignals des hitzebehandelten Polypeptids 50 % des Wertes des Messsignals der 100 %-Kontrolle des Polypeptids aufweist.

Die Temperaturstabilität (T (50 %)) des Polypeptids mit der SEQ ID-Nr. 46 wurde mit 42 °C, die des Polypeptids mit der SEQ ID-Nr. 1 mit 45 °C bestimmt. Somit beträgt die relative Steigerung der Temperaturstabilität, die durch die Verkürzung der N-terminalen Sequenz erreicht werden konnte, etwa 7 %. Des Weiteren konnte auch eine gesteigerte enzymatische Aktivität des Polypeptids mit der SEQ ID-Nr. 1 gegenüber dem parentalen Polypeptid mit der SEQ ID-Nr. 46 ermittelt werden.

Die Temperaturstabilität des Polypeptids mit der SEQ ID-Nr. 1 konnte durch gezielte Substitution einzelner Aminosäuren weiter gesteigert werden. Die relative Steigerung der Temperaturstabilität dieser Polypeptidvarianten gegenüber dem parentalen Polypeptid mit der SEQ ID-Nr. 1 sind in Tabelle 1 dargestellt.

**Tabelle 1: Modifikationen der Polypeptidvarianten und deren relative Steigerung der Temperaturstabilität in Prozent in Vergleich zum parentalen Enzym mit der SEQ ID-Nr. 1.**

| Modifikation(en) der SEQ ID-Nr. 1 | Relative Steigerung von T (50 %) | SEQ ID-Nr. des Polypeptids enthaltend die Modifikationen |
|---|---|---|
| H10Q | 4,4 % | - |
| A33E | 4,4 % | - |
| N66D | 6,7 % | - |
| G107E | 4,4 % | - |
| I140P | 4,4 % | - |
| L144M | 4,4 % | - |
| L149F | 4,4 % | - |
| K151R | 4,4 % | - |
| V157Y | 4,4 % | - |
| L199I | 6,7 % | - |
| R266S | 4,4 % | - |
| Q267P | 4,4 % | - |
| K270F | 4,4 % | - |
| R272H | 4,4 % | - |
| G275E | 4,4 % | - |
| G275A | 4,4 % | - |
| G280D | 4,4 % | - |
| G280P | 4,4 % | - |
| R284T | 4,4 % | - |
| R284P | 4,4 % | - |
| L286P | 4,4 % | - |
| L286R | 4,4 % | - |
| K293E | 4,4 % | - |
| L302I | 6,7 % | - |
| A312F | 4,4 % | - |
| L329F | 4,4 % | - |
| Q332E | 4,4 % | - |
| F360V | 4,4 % | - |
| S363T | 4,4 % | - |
| Q364H | 4,4 % | - |
| Q364L | 4,4 % | - |
| F365I | 4,4 % | - |
| N367H | 4,4 % | - |
| L371V | 4,4 % | - |
| L371M | 4,4 % | - |
| L372F | 4,4 % | - |
| A377V | 6,7 % | - |
| T389L | 4,4 % | - |
| I391V | 4,4 % | - |
| A394P | 6,7 % | - |
| S418A | 4,4 % | - |
| M419V | 4,4 % | - |
| E424A | 6,7 % | - |
| E424K | 4,4 % | - |
| A427V | 4,4 % | - |
| A429P | 4,4 % | - |
| S430A | 6,7 % | - |
| T436A | 4,4 % | - |
| T436S | 4,4 % | - |
| A440G | 4,4 % | - |
| A440S | 4,4 % | - |
| V443T | 4,4 % | - |
| V447A | 4,4 % | - |
| Q453R | 4,4 % | - |
| T455S | 4,4 % | - |
| K456Q | 4,4 % | - |
| S457T | 4,4 % | - |
| F462Y | 4,4 % | - |
| E463D | 6,7 % | - |
| R464I | 4,4 % | - |
| R465H | 4,4 % | - |
| R465S | 4,4 % | - |
| R465G | 4,4 % | - |
| M469K | 4,4 % | - |
| S473A | 4,4 % | - |
| G478D | 4,4 % | - |
| K487N | 4,4 % | - |
| Q490P | 4,4 % | - |
| L1991/A394P | 13,3 % | - |
| N66D/L199I | 11,1 % | - |
| L199I/L302I | 13,3 % | - |
| L199I/A377V | 15,6 % | - |
| L199I/E424A | 11,1 % | - |
| L199I/S430A | 11,1 % | - |
| L199I/E463D | 13,3 % | - |
| L199I/L302I/A394P | 20,0 % | - |
| L199I/L302I/A377V | 17,8 % | - |
| L199I/L302I/E424A | 17,8 % | - |
| L199I/A377V/A394P | 22,2 % | - |
| L199I/A394P/A429P | 17,8 % | - |
| L372F/A394P/V443T | 17,8 % | - |
| L199I/L302I/L372F | 15,6 % | - |
| L144M/L199I/L302I | 20,0 % | - |
| F360V/A394P/V443T | 17,8 % | - |
| H10Q/K151R/A302I | 15,6 % | - |
| R266S/A377V/E424K | 20,0 % | - |
| Q267/A394P/T436S | 17,8 % | - |
| R272H/G280D/E463D | 20,0 % | - |
| G275A/L302I/F360V | 17,8 % | - |
| N66D/L286P/N367H | 15,6 % | - |
| R284T/L286R/S430A | 15,6 % | - |
| K293E/E424A/M469K | 20,0 % | - |
| S363T/A377V/K456Q | 20,0 % | - |
| Q364H/L371V/S430A | 17,8 % | - |
| L199I/Q364L/Q490P | 15,6 % | - |
| F365I/A394P/R464I | 15,6% | - |
| L371M/A377V/A429P | 17,8 % | - |
| L302I/L372F/Q453R | 15,6 % | - |
| T389L/M419V/E463D | 17,8 % | - |
| 1391V/A394P/A440G | 20,0 % | - |
| S418A/S430A/F462Y | 20,0 % | - |
| N66D/A427V/V443T | 17,8 % | - |
| A440S/S457T/E463D | 20,0 % | - |
| L199I/V447A/T455S | 20,0 % | - |
| A377V/R465H/K487N | 17,8 % | - |
| L302I/R465S/G478D | 15,6 % | - |
| A377V/R465G/S473A | 20,0 % | - |
| N66D/L199I/L302I/A394P/E424A/S430A | 26,7 % | SEQ ID-Nr. 2 |
| N66D/L199I/L302I/A377V/A394P/E424A/S430A | 31,1 % | SEQ ID-Nr. 3 |
| N66D/L199I/L302I/A377V/A394P/E424A/S430A/E463D | 37,8 % | SEQ ID-Nr. 4 |
| N66D/L144M/L199I/L302I/F360V/L372F/A377V/A394P/E424A/S430A/V443T/E463D | 33,3 % | SEQ ID-Nr. 5 |
| L199I/L302I/A377V/A394P/E424A/S430A/E463D | 31,1 % | SEQ ID-Nr. 6 |
| N66D/L199I/L302I/A377V/A394P | 26,7 % | SEQ ID-Nr. 7 |
| N66D/L199I/L302I/Q364H/A377V/A394P/E424A/S430A/E463D | 35,5 % | SEQ ID-Nr. 8 |
| N66D/L199I/L302I/A377V/A394P/E424A/S430A/E463D/R465H | 40,0 % | SEQ ID-Nr. 9 |
| N66D/L199I/L302I/A377V/A394P/E424A/S430A/A440G/E463D | 37,8 % | SEQ ID-Nr. 10 |
| N66D/L199I/L302I/A377V/A394P/E424A/S430A/V447A/E463D | 35,5 % | SEQ ID-Nr. 11 |
| N66D/L199I/L302I/A377V/A394P/S418A/E424A/S430A/E463D | 35,5 % | SEQ ID-Nr. 12 |
| N66D/L199I/L302I/A377V/A394P/E424A/T436A/S430A/E463D | 35,5 % | SEQ ID-Nr. 13 |
| N66D/L199I/L302I/Q364L/A377V/A394P/E424A/S430A/E463D | 35,5 % | SEQ ID-Nr. 14 |
| N66D/L199I/L302I/A377V/A394P/E424A/S430A/E463D/Q490P | 37,8 % | SEQ ID-Nr. 15 |
| N66D/L199I/L302I/A377V/A394P/E424A/S430A/E463D/M469K | 37,8 % | SEQ ID-Nr. 16 |
| N66D/L199I/L302I/A377V/T389L/A394P/E424A/S430A/E463D | 40,0 % | SEQ ID-Nr. 17 |
| N66D/L 199I/L302I/A377V/A394P/E424A/S430A/E463D/R465S | 35,5 % | SEQ ID-Nr. 18 |
| N66D/L199I/L302I/A377V/A394P/E424A/S430A/E463D/R464I | 40,0 % | SEQ ID-Nr. 19 |
| N66D/L199I/L302I/A377V/A394P/E424A/S430A/E463D/R465G | 35,5 % | SEQ ID-Nr. 20 |
| N66D/L199I/L302I/A377V/A394P/E424A/S430A/A440S/E463D | 33,3 % | SEQ ID-Nr. 21 |
| N66D/L199I/L302I/A377V/A394P/E424A/S430A/S457T/E463D | 37,8 % | SEQ ID-Nr. 22 |
| N66D/L199I/L302I/A377V/A394P/E424A/S430A/T436S/E463D | 35,5 % | SEQ ID-Nr. 23 |
| N66D/L199I/L302I/S363T/L371V/A377V/A394P/E424A/S430A/E463D | 40,0 % | SEQ ID-Nr. 24 |
| N66D/L199I/L302I/A377V/A394P/E424A/S430A/V447A/Q453R/E463D | 35,5 % | SEQ ID-Nr. 25 |
| N66D/L199I/L302I/A377V/A394P/E424A/S430A/K456Q/F462Y/E463D | 40,0 % | SEQ ID-Nr. 26 |
| N66D/L199I/L302I/A377V/A394P/M419V/E424A/A427V/S430A/E463D | 35,5 % | SEQ ID-Nr. 27 |
| N66D/L199I/L302I/F365I/A377V/A394P/E424A/S430A/E463D/K487N | 33,3 % | SEQ ID-Nr. 28 |
| N66D/L199I/L302I/L371M/A377V/A394P/E424A/S430A/E463D/K487N | 33,3 % | SEQ ID-Nr. 29 |
| N66D/L199I/L302I/Q364L/A377V/T389L/A394P/M419V/E424A/A427V/S430A/V447A/E463D/R465S/M469K | 51,1 % | SEQ ID-Nr. 30 |
| N66D/L199I/L302I/A377V/T389L/A394P/M419V/E424A/A427V/S430AA/447A/E463D/R465S/M469K | 46,7 % | SEQ ID-Nr. 31 |
| N66D/L199I/L302I/S363T/Q364L/L371V/A377V/T389L/A394P/M419V/E424A/A427V/S430A/V447A/E463D/R464I/R465S/M469K | 60,0 % | SEQ ID-Nr. 32 |
| N66D/L199I/L302I/S363T/L371V/A377V/T389L/A394P/M419V/E424A/A427V/S430A/V447A/E463D/R464I/R465S/M469K | 57,8 % | SEQ ID-Nr. 33 |
| N66D/L199I/L302I/Q364L/N367H/L371V/A377V/T389L/A394P/S418A/M419V/E424A/A427V/S430A/T436A/A440S/V447A/E463D/R464I/R4 65S/M469K/Q490P | 62,2 % | SEQ ID-Nr. 34 |
| N66D/L199I/L302I/N367H/L371V/A377V/T389 L/A394P/S418A/M419V/E424A/A427V/S430A/ T436A/A440SA/447A/E463D/R464I/R465S/M4 69K/Q490P | 62,2 % | SEQ ID-Nr. 35 |
| N66D/L199I/L302I/S363T/N367H/L371V/A377 V/A394P/E424A/S430A/E463D/Q490P | 42,2 % | SEQ ID-Nr. 36 |
| N66D/L199I/L302I/A377V/A394P/S418A/M419 V/E424A/A427V/S430A/T436A/A440S/V447A/ E463D | 46,7 % | SEQ ID-Nr. 37 |
| N66D/L199I/L302I/A377V/T389L/A394P/E424A/S430A/S457T/E463D/R4641/R465S/M469K | 48,9 % | SEQ ID-Nr. 38 |
| N66D/L199I/L302I/S363T/L371V/A377V/T389L/A394P/M419V/E424A/A427V/S430A/A440S/V447A/S457T/E463D/R464I/M469K/Q490P | 55,5 % | SEQ ID-Nr. 39 |
| N66D/L199I/L302I/A377V/A394P/E424A/S430A/V447A/E463D/R465S/M469K/Q490P | 46,7 % | SEQ ID-Nr. 40 |
| N66D/L199I/L302I/L371M/A377V/A394P/M419V/E424A/A427V/S430A/E463D/R465S/M469K/K487N/Q490P | 51,1 % | SEQ ID-Nr. 41 |
| N66D/L199I/L302I/L371M/A377V/A394P/M419V/E424A/A427V/S430A/V447A/Q453R/E463D/R465S/M469K/K487N/Q490P | 55,5 % | SEQ ID-Nr. 42 |
| N66D/L 199I/L302I/N367H/L371V/A377V/T389L/A394P/S418A/M419V/E424A/A427V/A429P/S430A/T436A/A440SN447A/S457T/E463D/R 464I/R465S/M469K/Q490P | 64,4 % | SEQ ID-Nr. 43 |
| N66D/L199I/L302I/L371M/A377V/T389L/A394P/M419V/E424A/A427V/S430A/T436A/V447A/Q453R/K456Q/F462Y/E463D/R465S/M469K/ K487N/Q490P | 62,2 % | SEQ ID-Nr. 44 |
| N66D/L1991/L3021/N367H/L371V/A377V/T389L/A394P/S418A/M419V/E424A/A427V/A429P/S430A/T436A/A440S/V447A/Q453R/K456Q/S457T/F462Y/E463D/R464I/R465S/M469K/K487N/Q490P | 65,2 % | SEQ ID-Nr. 45 |

### Beispiel 4: Temperaturabhängige Aktivität (Temperaturaktivität) von Fusariumtoxin abbauenden Polypeptiden

Die auf ihre temperaturabhängige Aktivität zu testenden, Fusariumtoxin abbauenden Polypeptidvarianten wurden vor der Versuchsdurchführung aufgereinigt. Dazu wurden die Polypeptidvarianten aus Fermentationsüberständen mittels Anionenaustausch-chromatographie und anschließender Größenausschlusschromatographie in einem zweistufigen Prozess gereinigt. Die Polypeptidvarianten wurden auf eine Konzentration von 1 mg/ml eingestellt und im Reaktionsansatz in einer Verdünnung von 10⁻⁵ - 10⁻⁶ in einem Reaktionsvolumen von 1 ml eingesetzt. Die Aktivitätsbestimmung erfolgte wie in Beispiel 3 beschrieben Versuchen durch FB1 Hydrolyse- und anschließender Quantifizierung von FB1 mittels LC-MS, wobei der Versuch bei verschiedenen Temperaturen durchgeführt wurde. Die Inkubation erfolgte mit zwei Heizblöcken (Eppendorf, ThermoMixer) bei den Temperaturen 10 °C, 20 °C, 30 °C, 35 °C, 40 °C, 45 °C, 50 °C, 55 °C, 60 °C, 65 °C und 70 °C. 30 min. nach dem Start der Hitzeeinwirkung wurden je 100 µl des Reaktionsansatzes entnommen und für 5 min. bei 99 °C hitzeinaktiviert. Der Versuch, welcher bei 30 °C im Heizblock durchgeführt wurde, diente dabei als 100 % Kontrolle. Exemplarische Ergebnisse sind in Tabelle 2 angeführt.

**Tabelle 2: Temperaturabhängige Aktivität von Fusariumtoxin abbauenden Polypeptiden. < LOQ. Werte sind unterhalb der Detektionsgrenze (Level of Quantification: < 0,15 U / l im Versuchsansatzes)**

| **Sequenz ID inkl. Aminosäuresubstitutionen** | **Temperatur** | **Relative Aktivität bezogen auf Aktivität bei 30 °C [%]** |
|---|---|---|
| | 10 °C | 35 |
| | 20 °C | 59 |
| | 30 °C | 100 |
| | 35 °C | 123 |
| | 40 °C | 100 |
| SEQ ID-Nr. 1 | 45 °C | 105 |
| | 50 °C | 90 |
| | 55 °C | 26 |
| | 60 °C | < LOQ |
| | 65 °C | < LOQ |
| | 70 °C | < LOQ |
| | 30 °C | 100 |
| SEQ ID-Nr. 1 mit | 40 °C | 100 |
| | 50 °C | 95 |
| L199I/L302I/A394P | 60 °C | 62 |
| | 70 °C | 16 |
| | 30 °C | 100 |
| SEQ ID-Nr. 1 mit | 40 °C | 104 |
| | 50 °C | 91 |
| L144M/L199I/L302I | 60 °C | 60 |
| | 70 °C | 18 |
| | 30 °C | 100 |
| SEQ ID-Nr. 1 mit | 40 °C | 110 |
| | 50 °C | 97 |
| R266S/A377V/E424K | 60 °C | 63 |
| | 70 °C | 29 |
| | 30 °C | 100 |
| SEQ ID-Nr. 1 mit | 40 °C | 98 |
| | 50 °C | 81 |
| K293E/E424A/M469K | 60 °C | 50 |
| | 70 °C | < LOQ |
| | 30 °C | 100 |
| SEQ ID-Nr. 1 mit | 40 °C | 105 |
| | 50 °C | 84 |
| F365I/A394P/R464I | 60 °C | 57 |
| | 70 °C | 14 |
| | 30 °C | 100 |
| SEQ ID-Nr. 1 mit | 40 °C | 103 |
| | 50 °C | 89 |
| S418A/S430A/F462Y | 60 °C | 53 |
| | 70 °C | < LOD |
| | 10 °C | 33 |
| | 20 °C | 58 |
| | 30 °C | 100 |
| | 35 °C | 109 |
| | 40 °C | 110 |
| SEQ ID-Nr. 4 | 45 °C | 114 |
| | 50 °C | 148 |
| | 55 °C | 113 |
| | 60 °C | 94 |
| | 65 °C | 83 |
| | 70 °C | 46 |
| | 10 °C | 31 |
| | 20 °C | 56 |
| | 30 °C | 100 |
| | 35 °C | 113 |
| | 40 °C | 120 |
| SEQ ID-Nr. 43 | 45 °C | 126 |
| | 50 °C | 157 |
| | 55 °C | 168 |
| | 60 °C | 134 |
| | 65 °C | 102 |
| | 70 °C | 98 |
| | 10 °C | 33 |
| | 20 °C | 59 |
| | 30 °C | 100 |
| | 35 °C | 110 |
| | 40 °C | 124 |
| SEQ ID-Nr. 44 | 45 °C | 131 |
| | 50 °C | 162 |
| | 55 °C | 174 |
| | 60 °C | 126 |
| | 65 °C | 99 |
| | 70 °C | 95 |
| | 10 °C | 41 |
| SEQ ID-Nr. 45 | 20 °C | 68 |
| | 30 °C | 100 |
| | 35 °C | 115 |
| | 40 °C | 136 |
| | 45 °C | 141 |
| | 50 °C | 164 |
| | 55 °C | 177 |
| | 60 °C | 137 |
| | 65 °C | 121 |
| | 70 °C | 100 |
| | | |

| **Enzym** | **Pelletiertemperatur** | **Restaktivität [%]** |
|---|---|---|
| SEQ ID-Nr. 1 | 75 °C | 15 |
| | 80 °C | < LOQ |
| | 85 °C | < LOQ |
| | 90 °C | < LOQ |
| SEQ ID-Nr. 4 | 75 °C | 57 |
| | 80 °C | 46 |
| | 85 °C | 37 |
| | 90 °C | 14 |
| SEQ ID-Nr. 43 | 75 °C | 78 |
| | 80 °C | 73 |
| | 85 °C | 55 |
| | 90 °C | 31 |
| SEQ ID-Nr. 44 | 75 °C | 70 |
| | 80 °C | 59 |
| | 85 °C | 48 |
| | 90 °C | 25 |
| SEQ ID-Nr. 45 | 75 °C | 72 |
| | 80 °C | 68 |
| | 85 °C | 48 |
| | 90 °C | 30 |

### Beispiel 5: Bestimmung der Pelletierstabilität von Fusariumtoxin abbauenden Polypeptiden

Ausgewählte Polypeptid-Varianten wurden mit Hilfe von Standardverfahren in *Pichia pastoris* kloniert, unter kontrollierten, aeroben Bedingungen im Bioreaktor exprimiert, und extrazellulär sekretiert. Der klare Überstand wurde von der Biomasse getrennt, mit einer Trägersubstanz (Maltodextrin) versetzt und mittels einer Sprühtrocknungsanlage zu einem pelletierfähigen Pulver verarbeitet. Die als Pulver vorliegenden Fusariumtoxin abbauenden Polypeptid-Varianten wurden in jeweils derselben Konzentration von 100 U/kg zu Ferkelaufzuchtfutter beigemengt und in einem kontrollierten Prozess zu Futterpellets verarbeitet. Während des Pelletierprozesses wurde das Futter in einzelnen Chargen bei genau definierten Temperaturen (75 - 95 °C in 5 °C-Schritten) mittels Heißdampf befeuchtet und erhitzt. Nach dieser Aufbereitungsphase erfolgte der eigentliche Pelletierprozess. Die Restaktivität der in den Pellets enthaltenen Fusariumtoxin abbauenden Polypeptidvarianten wurde wie in Beispiel 2 beschrieben bestimmt, wobei als 100 %-Kontrolle nicht pelletiertes Futter mit den jeweiligen Fusariumtoxin abbauenden Polypeptidvarianten diente. Als Restaktivität wird daher die nach dem Pelletierprozess verbliebene Enzymaktivität definiert. Die Werte sind in Tabelle 3 angegeben.

**Tabelle 3: Pelletiertemperaturen und Restaktivitäten von Fusariumtoxin abbauenden Polypeptiden. < LOQ .. Werte sind unterhalb der Detektionsgrenze (Level of Quantification: < 0,15 U / l im Versuchsansatzes)**

| **Enzym** | **Pelletiertemperatur** | **Restaktivität %** |
|---|---|---|
| SEQ ID-Nr. 1 | 75 °C | 15 |
| | 80 °C | < LOQ |
| | 85 °C | < LOQ |
| | 90 °C | < LOQ |
| SEQ ID--Nr. 4 | 75 °C | 57 |
| | 80 °C | 46 |
| | 85°C | 37 |
| | 90 °C | 14 |
| SEQ ID-Nr. 43 | 75 °C | 78 |
| | 80 °C | 73 |
| | 85 °C | 58 |
| | 90 °C | 31 |
| SEQ ID-Nr. 44 | 75 °C | 70 |
| | 80 °C | 59 |
| | 85 °C | 48 |
| | 90 °C | 25 |
| SEQ ID-Nr. 45 | 75 °C | 72 |
| | 80 °C | 68 |
| | 85 °C | 48 |
| | 90 °C | 30 |

## Patentansprüche

1. Fusariumtoxine spaltende Polypeptidvarianten einer Fusariumtoxin Carboxylesterase mit der SEQ ID-Nr. 46, **dadurch gekennzeichnet, dass** die Polypeptidvarianten jeweils eine am N-Terminus um 47 Aminosäuren gekürzte Aminosäuresequenz besitzen, wobei die Aminosäuresequenzen wenigstens 70 %, vorzugsweise 80 % insbesondere bevorzugt 100 % Sequenzidentität, zum Aminosäuresequenzabschnitt 48 - 540 der SEQ ID-Nr. 46 aufweisen, dass die Temperaturstabilitäten (T(50%)) des Polypeptids der SEQ ID-Nr. 46 mit 42 °C und jene der Polypeptidvariante der SEQ ID-Nr. 1 mit 45 °C bestimmt ist oder Modifikationen der SEQ ID-Nr. 1 eine relative Steigerung von T (50 %) im Vergleich zum parentalen Enzym der SEQ ID-Nr.1 aufweisen, wobei an wenigstens einer Position gewählt aus der Gruppe bestehend aus 10, 33, 66, 107, 140, 144, 149, 151, 157, 199, 266, 267, 270, 272, 275, 280, 284, 286, 293, 302, 312, 329, 332, 360, 363, 364, 365, 367, 371, 372, 377, 389, 391, 394, 418, 419, 424, 427, 429, 430, 436, 440, 443, 447, 453, 455, 456, 457, 462, 463, 464, 465, 469, 473, 478, 487 und 490 als Modifikation eine Aminosäuresubstitution enthalten ist und dass die Aminosäuresubstituenten an den Positionen 10 und 456 gewählt aus Q, E, N, H, K und R sind, an den Positionen 33, 107, 293 und 332 gewählt aus E, Q, D, K, R und N sind, an den Positionen 66, 463 und 478 gewählt aus D, E, K, N, Q und R sind, an den Positionen 140 und 490 gewählt aus P, A, S und N sind, an den Positionen 144 und 367 gewählt aus I, L, M und V sind, an den Positionen 149, 270, 312, 329 und 372 gewählt aus F, Y, W und H sind, an den Positionen 151 und 453 gewählt aus D, E, K und R sind, an den Positionen 157 und 462 gewählt aus F, H, W und Y sind, an den Positionen 199, 302, 365 und 464 gewählt aus I, L, M und V sind, an den Positionen 266 und 455 und gewählt aus A, S und T sind, an den Positionen 267, 394 und 429 gewählt aus A, N, P und S sind, an der Position 272 gewählt aus H, N, Q und S sind, an der Position 275 gewählt aus A, D, E, G, K, N, Q, R und S sind, an der Position 280 gewählt aus A, D, E, K, N, P, Q, R und S, an der Position 284 gewählt aus A, N, P, S, T und V sind, an der Position 286 gewählt aus A, D, E, K, N, P, R und S sind, an den Positionen 360, 377, 391, 419 und 427 gewählt aus A, I, L S, T und V sind, an den Positionen 363, 443 und 457 gewählt aus A, S, T und V sind an der Position 364 gewählt aus H, I, L, M, N, Q, S und V sind, an der Position 371 gewählt aus A, I, L, M, S, T und V sind, an der Position 389 gewählt aus I, L, M und V sind, an den Positionen 418, 430, 447 und 473 gewählt aus A, G und S sind, an der Position 424 gewählt aus A, D, E, G, K, R und S sind, an der Position 436 gewählt aus A, G, S und T sind, an der Position 440 gewählt aus A, G, S und T sind, an der Position 465 gewählt aus A, G, H, N, Q, S und T sind, an der Position 469 gewählt aus D, E, K und R sind und/oder an der Position 487 gewählt aus N, D, Q, H und S sind.

2. Polypeptidvarianten nach Anspruch 1, **dadurch gekennzeichnet, dass** die Aminosäuresubstitution aus der Gruppe 10Q, 33E, 66D, 107E, 140P, 144M, 149F, 151R, 157Y, 199I, 266S, 267P, 270F, 272H, 275E, 275A, 280D, 280P, 284T, 284P, 286P, 286R, 293E, 302I, 312F, 329F, 332E, 360V, 363T, 364H, 364L, 365I, 367H, 371V, 371M, 372F, 377V, 389L, 391V, 394P, 418A, 419V, 424A, 424K, 427V, 429P, 430A, 436A, 436S, 440G, 440S, 443T, 447A, 453R, 455S, 456Q, 457T, 462Y, 463D, 464I, 465H, 465S, 465G, 469K, 473A, 478D, 487N und 490P gewählt ist.

3. Polypeptidvarianten nach einem der Ansprüche 1 oder 2, **dadurch gekennzeichnet, dass** sie an wenigstens einer Position ausgewählt aus der Gruppe bestehend aus 66, 199, 302, 377, 394, 424, 430 und 463 eine Aminosäuresubstitution aufweisen.

4. Polypeptidvarianten nach einem der Ansprüche 1, 2 oder 3 **dadurch gekennzeichnet, dass** die Aminosäuresubstitution gewählt aus der Gruppe 66D, 199I, 302I, 377V, 394P, 424A, 430A und 463D ist.

5. Polypeptidvarianten nach Anspruch 1, **dadurch gekennzeichnet, dass** die Polypeptidvariante wenigstens an zwei, insbesondere drei Positionen der Aminosäuresequenz ausgewählt aus der Gruppe bestehend aus 10, 33, 66, 107, 140, 144, 149, 151, 157, 199, 266, 267, 270, 272, 275, 280, 284, 286, 293, 302, 312, 329, 332, 360, 363, 364, 365, 367, 371, 372, 377, 389, 391, 394, 418, 419, 424, 427, 429, 430, 436, 440, 443, 447, 453, 455, 456, 457, 462, 463, 464, 465, 469, 473, 478, 487 und 490 eine Aminosäuresubstitutionen aufweisen, wobei die Aminosäuresubstitutionen gewählt aus der Gruppe 10Q, 66D, 144M, 151R, 199I, 266S, 267P, 272H, 275E, 275A, 280D, 284T, 286P, 286R, 293E, 302I, 360V, 363T, 364H, 364L, 365I, 367H, 371V, 371M, 372F, 377V, 389L, 391V, 394P, 418A, 419V, 424A, 424K, 427V, 429P, 430A, 436A, 436S, 440G, 440S, 443T, 447A, 453R, 455S, 456Q, 457T, 462Y, 463D, 464I, 465H, 465S, 465G, 469K, 473A, 478D, 487N und 490P sind.

6. Polypeptidvarianten nach Anspruch 1 oder 2, **dadurch gekennzeichnet, dass** die Aminosäuresequenz der Polypeptidvarianten Kombinationen von mehreren Aminosäuresubstitutionen enthält, wobei die Kombinationen der Positionen aus der Gruppe
66/199/302/394/424/430, 66/199/302/377/394/424/430, 66/199/302/377/394/424/430/463, 66/144/199/302/360/372/377/394/424/430/443/463, 199/302/377/394/424/430/463, 66/199/302/377/394, 66/199/302/364/377/394/424/430/463, 66/199/302/377/394/424/430/463/465, 66/199/302/377/394/424/430/440/463, 66/199/302/377/394/424/430/447/463, 66/199/302/377/394/418/424/430/463, 66/199/302/377/394/424/436/430/463, 66/199/302/364/377/394/424/430/463, 66/199/302/377/394/424/430/463/490, 66/199/302/377/394/424/430/463/469, 66/199/302/377/389/394/424/430/463, 66/199/302/377/394/424/430/463/465, 66/199/302/377/394/424/430/463/464, 66/199/302/377/394/424/430/463/465, 66/199/302/377/394/424/430/440/463, 66/199/302/377/394/424/430/457/463, 66/199/302/377/394/424/430/436/463, 66/199/302/363/371/377/394/424/430/463, 66/199/302/377/394/424/430/447/453/463, 66/199/302/377/394/424/430/456/462/463, 66/199/302/377/394/419/424/427/430/463, 66/199/302/365/377/394/424/430/463/487 und 66/199/302/371/377/394/424/430/463/487 gewählt sind.

7. Polypeptidvarianten Anspruch 6, **dadurch gekennzeichnet, dass** die Aminosäuresequenzen der Polypeptidvarianten gewählt sind aus der Gruppe der SEQ ID-Nrn. 2 bis 29.

8. Polypeptidvarianten nach Anspruch 1 oder 2, **dadurch gekennzeichnet, dass** die Aminosäuresequenz der Polypeptidvarianten Kombinationen von mehreren Aminosäuresubstitutionen enthält, wobei die Kombinationen der Positionen aus der Gruppe
66/99/302/364/377/389/394/419/424/427/430/44 7/463/465/469, 66/199/302/377/389/394/419/424/427/430/447/463/465/469, 66/199/302/363/364/371/377/389/394/419/424/427/430/447/463/464/465/469, 66/199/302/363/371/377/389/394/419/424/427/430/447/463/464/465/469, 66/199/302/364/367/371/377/389/394/418/419/424/427/430/436/440/447/463/464/ 465/
469/490, 66/199/302/367/371/377/389/394/418/419/424/427/430/436/440/447/463/464/465/ 469/
490,
66/199/302/363/367/371/377/394/424/430/463/490, 66/199/302/377/394/418/419/424/427/430/436/440/447/463, 66/199/302/377/389/394/424/430/457/463/464/465/469, 66/199/302/363/371/377/389/394/419/424/427/430/440/447/457/463/464/469/490, 66/199/302/377/394/424/430/463/447/490/469/465, 66/199/302/377/394/424/430/463/490/469/465/419/427/371/487, 66/199/302/371/377/394/419/424/427/430/447/453/463/465/469/487/490, 66/199/302/367/371/377/389/394/418/419/424/427/429/430/436/440/447/457/463/ 464/465/469/490, 66/199/302/371/377/389/394/419/424/427/430/436/447/453/456/462/463/465/469/ 490/487
und 66/199/302/367/371/377/389/394/418/419/424/427/429/430/436/440/447/453/456/ 457/462/463/464/465/469/487/490 gewählt sind.

9. Polypeptidvarianten nach Anspruch 8, **dadurch gekennzeichnet, dass** die Aminosäuresequenzen der Polypeptidvarianten aus der Gruppe der SEQ ID-Nrn. 30 bis 45 gewählt sind.

10. Isolierte Polynukleotide, **dadurch gekennzeichnet, dass** die Polynukleotide Nukleotidsequenzen besitzen, die für eine Polypeptidvariante nach einem der Ansprüche 1 bis 9 codiert.

11. Fusariumtoxin spaltender Zusatzstoff, **dadurch gekennzeichnet, dass** der Zusatzstoff wenigstens eine Polypeptidvariante nach einem der Ansprüche 1 bis 9 enthält und dass gegebenenfalls wenigstens ein Hilfsstoff enthalten ist.

12. Zusatzstoff nach Anspruch 11, **dadurch gekennzeichnet, dass** der Hilfsstoff gewählt ist aus Gruppe von inerten Trägern, Vitaminen, Mineralstoffen, phytogenen Substanzen, Enzymen und weiteren Komponenten zur Detoxifizierung von Mykotoxinen, wie Mykotoxin-abbauende Enzyme, insbesondere Aflatoxin Oxidasen, Ergotamin Hydrolasen, Ergotamin Amidasen, Zearalenon Esterasen, Zearalenon Lactonasen, Zearalenon Hydrolasen, Ochratoxin Amidasen, Fumonisin Aminotransferasen, Aminopolyol Aminoxidasen, Deoxynivalenol Epoxidhydrolasen, Deoxynivalenol Dehydrogenasen, Deoxynivalenol Oxidasen, Trichothecene Dehydrogenasen, Trichothecene Oxidasen; und Mykotoxinabbauenden Mikroorganismen; und Mykotoxin-bindende Stoffe, beispielsweise mikrobielle Zellwände oder anorganische Materialien, wie Bentonite.

13. Verwendung eines Zusatzstoffes nach einem der Ansprüche 11 oder 12 zur Spaltung von wenigstens einem Fusariumtoxin in, insbesondere pelletierten, Nahrungs- oder Futtermitteln, insbesondere für Schweine, Geflügel, Rinder, Pferde, Fische oder Aquakultur.

14. Verwendung eines Zusatzstoffes nach einem der Ansprüche 11 oder 12 zur Spaltung von wenigstens einem Fusariumtoxin in einem Verfahren, insbesondere bei Temperaturen von mindestens 50 °C, bevorzugt von mindestens 70 °C zur Herstellung oder Verarbeitung von Nahrungs- oder Futtermitteln.

15. Polypeptidvarianten nach einem der Ansprüche 1 bis 9 zur Verwendung in einem Präparat zur Prophylaxe und/oder Behandlung von Fusariumtoxin-Mykotoxikosen.

16. Verfahren zur hydrolytischen Spaltung von wenigstens einem Fusariumtoxin, **dadurch gekennzeichnet, dass** wenigstens ein Fusariumtoxin mit wenigstens einer Polypeptidvariante nach einem der Ansprüche 1 bis 9 oder wenigstens einem Zusatzstoff nach einem der Ansprüche 11 oder 12 in Kontakt gebracht wird, und dass die Mischung aus der Polypeptidvariante und dem Fusariumtoxin einer Temperaturbehandlung bei mindestens 50 °C, bevorzugt bei mindestens 70 °C unterworfen wird und dass die Mischung entweder während oder nach der Temperaturbehandlung mit einer für die hydrolytische Spaltung ausreichenden Menge Feuchtigkeit in Kontakt gebracht wird.

17. Verfahren nach Anspruch 16, **dadurch gekennzeichnet, dass** die Polypeptidvariante oder der Zusatzstoff mit einem mit wenigstens einem Fusariumtoxin kontaminierten Futter- oder Nahrungsmittel vermischt wird und gegebenenfalls die Temperaturbehandlung während eines Pelletierverfahrens erfolgt.

18. Verfahren nach einem der Ansprüche 16 oder 17, **dadurch gekennzeichnet, dass** die Polypeptidvariante pro Kilogramm Futter- oder Nahrungsmittel in einem Konzentrationsbereich von 5 U bis 500 U, bevorzugt von 10 U bis 300 U und bevorzugter von 15 U bis 100 zugesetzt wird.

## Claims

1. Fusarium toxin-cleaving polypeptide variants of a fusarium toxin carboxylesterase of SEQ ID No. 46, **characterized in that** the polypeptide variants each possess an amino acid sequence truncated by 47 amino acids at the N-terminus, the amino acid sequences sharing at least 70%, preferably 80%, particularly preferably 100%, sequence identity, namely SEQ ID No. 1, with the amino acid sequence section 48-540 of SEQ ID No. 46, that the temperature stability (T(50%) of the polypeptide of SEQ ID No. 46 is determined to be 42°C and that of the polypeptide variant of SEQ ID No. 1 is determined to be 45°, or modifications of SEQ ID No. 1 comprise relative increases of T(50%) as compared to the parental enzyme of SEQ ID No. 1, wherein on at least one position selected from the group consisting of 10, 33, 66, 107, 140, 144, 149, 151, 157, 199, 266, 267, 270, 272, 275, 280, 284, 286, 293, 302, 312, 329, 332, 360, 363, 364, 365, 367, 371, 372, 377, 389, 391, 394, 418, 419, 424, 427, 429, 430, 436, 440, 443, 447, 453, 455, 456, 457, 462, 463, 464, 465, 469, 473, 478, 487 and 490 an amino acid substitution is contained as modification, and that the amino acid substituents at positions 10 and 456 are selected from Q, E, N, H, K and R, at positions 33, 107, 293 and 332 from E, Q, D, K, R and N, at positions 66, 463 and 478 from D, E, K, N, Q and R, at positions 140 and 490 from P, A, S and N, at positions 144 and 367 from I, L, M and V, at positions 149, 270, 312, 329 and 372 from F, Y, W and H, at positions 151 and 453 from D, E, K and R, at positions 157 and 462 from F, H, W and Y, at positions 199, 302, 365 and 464 from I, L, M and V, at positions 266 and 455 and from A, S and T, at positions 267, 394 and 429 from A, N, P and S, at position 272 from H, N, Q and S, at position 275 from A, D, E, G, K, N, Q, R and S, at position 280 from A, D, E, K, N, P, Q, R and S, at position 284 from A, N, P, S, T and V, at position 286 from A, D, E, K, N, P, R and S, at positions 360, 377, 391, 419 and 427 from A, I, L S, T and V, at positions 363, 443 and 457 from A, S, T and V, at position 364 from H, I, L, M, N, Q, S and V, at position 371 from A, I, L, M, S, T and V, at position 389 from I, L, M and V, at positions 418, 430, 447 and 473 from A, G and S, at position 424 from A, D, E, G, K, R and S, at position 436 from A, G, S and T, at position 440 from A, G, S and T, at position 465 from A, G, H, N, Q, S and T, at position 469 from D, E, K and R and/or at position 487 from N, D, Q, H and S.

2. Polypeptide variants according to claim 1, **characterized in that** the amino acid substitutions are selected from the group consisting of 10Q, 33E, 66D, 107E, 140P, 144M, 149F, 151R, 157Y, 199I, 266S, 267P, 270F, 272H, 275E, 275A, 280D, 280P, 284T, 284P, 286P, 286R, 293E, 3021, 312F, 329F, 332E, 360V, 363T, 364H, 364L, 365I, 367H, 371V, 371M, 372F, 377V, 389L, 391V, 394P, 418A, 419V, 424A, 424K, 427V, 429P, 430A, 436A, 436S, 440G, 440S, 443T, 447A, 453R, 455S, 456Q, 457T, 462Y, 463D, 464I, 465H, 465S, 465G, 469K, 473A, 478D, 487N und 490P.

3. Polypeptide variants according to any one of claim 1 or 2, **characterized in that** they comprise an amino acid substitution on at least one position selected from the group consisting of 66, 199, 302, 377, 394, 424, 430 und 463.

4. Polypeptide variants according to any one of claim 1, 2 or 3, **characterized in that** the amino acid substitution is selected from the group consisting of 66D, 1991, 3021, 377V, 394P, 424A, 430A und 463D.

5. Polypeptide variants according to claim 1, **characterized in that** the polypeptide variants, on at least two, in particular three, positions of the amino acid sequence, selected from the group consisting of 10, 33, 66, 107, 140, 144, 149, 151, 157, 199, 266, 267, 270, 272, 275, 280, 284, 286, 293, 302, 312, 329, 332, 360, 363, 364, 365, 367, 371, 372, 377, 389, 391, 394, 418, 419, 424, 427, 429, 430, 436, 440, 443, 447, 453, 455, 456, 457, 462, 463, 464, 465, 469, 473, 478, 487 and 490, each comprise an amino acid substitution, the amino acid substitution being selected from the group consisting of 10Q, 66D, 144M, 151R, 199I, 266S, 267P, 272H, 275E, 275A, 280D, 284T, 286P, 286R, 293E, 3021, 360V, 363T, 364H, 364L, 3651, 367H, 371V, 371M, 372F, 377V, 389L, 391V, 394P, 418A, 419V, 424A, 424K, 427V, 429P, 430A, 436A, 436S, 440G, 440S, 443T, 447A, 453R, 455S, 456Q, 457T, 462Y, 463D, 464I, 465H, 465S, 465G, 469K, 473A, 478D, 487N and 490P.

6. Polypeptide variants according to claim 1 or 2, **characterized in that** the amino acid sequence of the polypeptide variants comprises combinations of several amino acid substitutions, the combinations of the positions being selected from the group consisting of
66/199/302/394/424/430, 66/199/302/377/394/424/430, 66/199/302/377/394/424/430/463, 66/144/199/302/360/372/377/394/424/430/443/463, 199/302/377/394/424/430/463, 66/199/302/377/394, 66/199/302/364/377/394/424/430/463, 66/199/302/377/394/424/430/463/465, 66/199/302/377/394/424/430/440/463, 66/199/302/377/394/424/430/447/463, 66/199/302/377/394/418/424/430/463, 66/199/302/377/394/424/436/430/463, 66/199/302/364/377/394/424/430/463, 66/199/302/377/394/424/430/463/490, 66/199/302/377/394/424/430/463/469, 66/199/302/377/389/394/424/430/463, 66/199/302/377/394/424/430/463/465, 66/199/302/377/394/424/430/463/464, 66/199/302/377/394/424/430/463/465, 66/199/302/377/394/424/430/440/463, 66/199/302/377/394/424/430/457/463, 66/199/302/377/394/424/430/436/463, 66/199/302/363/371/377/394/424/430/463, 66/199/302/377/394/424/430/447/453/463, 66/199/302/377/394/424/430/456/462/463, 66/199/302/377/394/419/424/427/430/463, 66/199/302/365/377/394/424/430/463/487 and 66/199/302/371/377/394/424/430/463/487.

7. Polypeptide variants according to claim 6, **characterized in that** the amino acid sequences of the polypeptide variants are selected from the group consisting of SEQ ID Nos. 2 to 29.

8. Polypeptide variants according to claim 1 or 2, **characterized in that** the amino acid sequence of the polypeptide variants comprises combinations of several amino acid substitutions, the combinations of the positions being selected from the group consisting of 66/99/302/364/377/389/394/419/424/427/430/447/463/465/469, 66/199/302/377/389/394/419/424/427/430/447/463/465/469, 66/199/302/363/364/371/377/389/394/419/424/427/430/447/463/464/465/469, 66/199/302/363/371/377/389/394/419/424/427/430/447/463/464/465/469, 66/199/302/364/367/371/377/389/394/418/419/424/427/430/436/440/447/463/464/465/ 469/490, 66/199/302/367/371/377/389/394/418/419/424/427/430/436/440/447/463/464/465/469/ 490, 66/199/302/363/367/371/377/394/424/430/463/490, 66/199/302/377/394/418/419/424/427/430/436/440/447/463, 66/199/302/377/389/394/424/430/457/463/464/465/469, 66/199/302/363/371/377/389/394/419/424/427/430/440/447/457/463/464/469/490, 66/199/302/377/394/424/430/463/447/490/469/465, 66/199/302/377/394/424/430/463/490/469/465/419/427/371/487, 66/199/302/371/377/394/419/424/427/430/447 /453/463/465/469/487/490, 66/199/302/367/371/377/389/394/418/419/424/427/429/430/436/440/447/457/463/464/46 5/469/490, 66/199/302/371/377/389/394/419/424/427/430/436/447/453/456/462/463/465/469/490/ 487
and 66/199/302/367/371/377/389/394/418/419/424/427/429/430/436/440/447/453/456/457/46 2/463/464/465/469/487/490.

9. Polypeptide variants according to claim 8, **characterized in that** the amino acid sequences of the polypeptide variants are selected from the group consisting of SEQ ID Nos. 30 to 45.

10. Isolated polynucleotides, **characterized in that** the polynucleotides comprises nucleotide sequences encoding a polypeptide variant according to any one of claims 1 to 9.

11. A fusarium toxin-cleaving additive, **characterized in that** said additive comprises at least one polypeptide variant according to any one of claims 1 to 9 and optionally at least one adjuvant.

12. An additive according to claim 11, **characterized in that** the adjuvant is selected from the group consisting of inert carriers, vitamins, minerals, phytogenic substances, enzymes and other components for detoxifying mycotoxins, such as mycotoxin-degrading enzymes, in particular aflatoxin oxidases, ergotamine hydrolases, ergotamine amidases, zearalenone esterases, zearalenone lactonases, zearalenone hydrolases, ochratoxin amidases, fumonisin aminotransferases, aminopolyol aminoxidases, deoxynivalenol epoxide hydrolases, deoxynivalenol dehydrogenases, deoxynivalenol oxidases, trichothecene dehydrogenases, trichothecene oxidases; and mycotoxin-degrading microorganisms; and mycotoxin-binding substances, for instance microbial cell walls or inorganic materials such as bentonite.

13. The use of an additive according to any one of claim 11 or 12 for cleaving at least one fusarium toxin in, in particular, pelletized food or feed products, in particular for pigs, poultry, cattle, horses, fishes or aquaculture.

14. The use of an additive according to any one of claim 11 or 12 for cleaving at least one fusarium toxin in a process, in particular at temperatures of at least 50°C, preferably at least 70°C, for the production or processing of food or feed products.

15. Polypeptide variants according to any one of claims 1 to 9 for use in a preparation for the prophylaxis and/or treatment of fusarium toxin mycotoxicoses.

16. A method for hydrolytically cleaving at least one fusarium toxin, **characterized in that** at least one fusarium toxin is contacted with at least one polypeptide variant according to any one of claims 1 to 9, or at least one additive according to any one of claim 11 or 12, and that the mixture of the polypeptide variant and the fusarium toxin is subjected to a temperature treatment at at least 50°C, preferably at least 70°C, and that the mixture is contacted with an amount of moisture sufficient for hydrolytic cleavage either during or after the temperature treatment.

17. A method according to claim 16, **characterized in that** the polypeptide variant, or the additive, is mixed with a feed or food product contaminated with at least one fusarium toxin, and the temperature treatment is optionally performed during a pelletizing process.

18. A method according to any one of claim 16 or 17, **characterized in that** the polypeptide variant is added at a concentration range of 5 U to 500 U, preferably 10 U to 300 U, and more preferably 15 U to 100 U, per kilogram of feed or food product.

## Revendications

1. Variants polypeptidiques dissociant des toxines de fusarium d'une toxine de fusarium carboxylestérase avec la SEQ ID NO 46, **caractérisés en ce que** les variants polypeptidiques possèdent respectivement une séquence d'acides aminés raccourcie de 47 acides aminés à l'extrémité N-terminale, dans lesquels les séquences d'acides aminés présentent une identité de séquence d'au moins 70 %, de préférence de 80 %, de manière particulièrement préférée de 100 %, par rapport au segment de séquence d'acides aminés 48 - 540 de la SEQ ID NO 46, que les stabilités de température (T(50 %)) du polypeptide de la SEQ ID NO 46 sont définies avec 42 °C et celles des variants polypeptidiques de la SEQ ID NO 1 avec 45 °C ou des modifications de la SEQ ID NO 1 présentent une augmentation relative de T (50 %) en comparaison avec l'enzyme parent de la SEQ ID NO 1, dans lesquels une substitution d'acides aminés est contenue en tant que modification sur au moins une position choisie parmi le groupe constitué de 10, 33, 66, 107, 140, 144, 149, 151, 157, 199, 266, 267, 270, 272, 275, 280, 284, 286, 293, 302, 312, 329, 332, 360, 363, 364, 365, 367, 371, 372, 377, 389, 391, 394, 418, 419, 424, 427, 429, 430, 436, 440, 443, 447, 453, 455, 456, 457, 462, 463, 464, 465, 469, 473, 478, 487 et 490, et que les substituts d'acides aminés sont choisis sur les positions 10 et 456 parmi Q, E, N, H, K et R, sont choisis sur les positions 33, 107, 293 et 332 parmi E, Q, D, K, R et N, sont choisis sur les positions 66, 463 et 478 parmi D, E, K, N, Q et R, sont choisis sur les positions 140 et 490 parmi P, A, S et N, sont choisis sur les positions 144 et 367 parmi I, L, M et V, sont choisis sur les positions 149, 270, 312, 329 et 372 parmi F, Y, W et H, sont choisis sur les positions 151 et 453 parmi D, E, K et R, sont choisis sur les positions 157 et 462 parmi F, H, W et Y, sont choisis sur les positions 199, 302, 365 et 464 parmi I, L, M et V, sont choisis sur les positions 266 et 455 parmi A, S et T, sont choisis sur les positions 267, 394 et 429 parmi A, N, P et S, sont choisis sur la position 272 parmi H, N, Q et S, sont choisis sur la position 275 parmi A, D, E, G, K, N, Q, R et S, sont choisis sur la position 280 parmi A, D, E, K, N, P, Q, R et S, sont choisis sur la position 284 parmi A, N, P, S, T et V, sont choisis sur la position 286 parmi A, D, E, K, N, P, R et S, sont choisis sur les positions 360, 377, 391, 419 et 427 parmi A, I, L, S, T et V, sont choisis sur les positions 363, 443 et 457 parmi A, S, T et V, sont choisis sur la position 364 parmi H, I, L, M, N, Q, S et V, sont choisis sur la position 371 parmi A, I, L, M, S, T et V, sont choisis sur la position 389 parmi I, L, M et V, sont choisis sur les positions 418, 430, 447 et 473 parmi A, G, et S, sont choisis sur la position 424 parmi A, D, E, G, K, R et S, sont choisis sur la position 436 parmi A, G, S, et T, sont choisis sur la position 440 parmi A, G, S et T, sont choisis sur la position 465 parmi A, G, H, N, Q, S et T, sont choisis sur la position 469 parmi D, E, K et R et/ou sont choisis sur la position 487 parmi N, D, Q, H et S.

2. Variants polypeptidiques selon la revendication 1, **caractérisés en ce que** la substitution d'acides aminés est choisie parmi le groupe 10Q, 33E, 66D, 107E, 140P, 144M, 149F, 151R, 157Y, 199I, 266S, 267P, 270F, 272H, 275E, 275A, 280D, 280P, 284T, 284P, 286P, 286R, 293E, 302I, 312F, 329F, 332E, 360V, 363T, 364H, 364L, 365I, 367H, 371V, 371M, 372F, 377V, 389L, 391V, 394P, 418A, 419V, 424A, 424K, 427V, 429P, 430A, 436A, 436S, 440G, 440S, 443T, 447A, 453R, 455S, 456Q, 457T, 462Y, 463D, 464I, 465H, 465S, 465G, 469K, 473A, 478D, 487N et 490P.

3. Variants polypeptidiques selon l'une des revendications 1 ou 2, **caractérisés en ce qu'**ils présentent sur au moins une position choisie parmi le groupe constitué de 66, 199, 302, 377, 394, 424, 430 et 463 une substitution d'acides aminés.

4. Variants polypeptidiques selon l'une des revendications 1, 2 ou 3, **caractérisés en ce que** la substitution d'acides aminés est choisie parmi le groupe 66D, 199I, 302I, 377V, 394P, 424A, 430A et 463D.

5. Variants polypeptidiques selon la revendication 1, **caractérisés en ce que** les variants polypeptidiques présentent au moins sur deux, en particulier trois, positions de la séquence d'acides aminés choisies parmi le groupe constitué de 10, 33, 66, 107, 140, 144, 149, 151, 157, 199, 266, 267, 270, 272, 275, 280, 284, 286, 293, 302, 312, 329, 332, 360, 363, 364, 365, 367, 371, 372, 377, 389, 391, 394, 418, 419, 424, 427, 429, 430, 436, 440, 443, 447, 453, 455, 456, 457, 462, 463, 464, 465, 469, 473, 478, 487 et 490 des substitutions d'acides aminés, dans lesquels les substitutions d'acides aminés sont choisies parmi le groupe 10Q, 66D, 144M, 151R, 199I, 266S, 267P, 272H, 275E, 275A, 280D, 284T, 286P, 286R, 293E, 302I, 360V, 363T, 364H, 364L, 365I, 367H, 371V, 371M, 372F, 377V, 389L, 391V, 394P, 418A, 419V, 424A, 424K, 427V, 429P, 430A, 436A, 436S, 440G, 440S, 443T, 447A, 453R, 455S, 456Q, 457T, 462Y, 463D, 464I, 465H, 465S, 465G, 469K, 473A, 478D, 487N et 490P.

6. Variants polypeptidiques selon la revendication 1 ou 2, **caractérisés en ce que** la séquence d'acides aminés des variants polypeptidiques contient des combinaisons de plusieurs substitutions d'acides aminés, dans lesquels les combinaisons des positions sont choisies parmi le groupe
66/199/302/394/424/430, 66/199/302/377/394/424/430, 66/199/302/377/394/424/430/463, 66/144/199/302/360/372/377/394/424/430/443/463, 199/302/377/394/424/430/463, 66/199/302/377/394. 66/199/302/364/377/394/424/430/463, 66/199/302/377/394/424/430/463/465, 66/199/302/377/394/424/430/440/463, 66/199/302/377/394/424/430/447/463, 66/199/302/377/394/418/424/430/463, 66/199/302/377/394/424/436/430/463, 66/199/302/364/377/394/424/430/463, 66/199/302/377/394/424/430/463/490, 66/199/302/377/394/424/430/463/469, 66/199/302/377/389/394/424/430/463, 66/199/302/377/394/424/430/463/465, 66/199/302/377/394/424/430/463/464, 66/199/302/377/394/424/430/463/465, 66/199/302/377/394/424/430/440/463. 66/199/302137713941424/4301457/463, 66/199/302/377/394/424/430/436/463, 66/199/302/363/371/377/394/424/430/463, 66/199/302/377/394/424/430/44714531463, 66/199/302/377/394/424/430/456/462/463, 66/199/302/377/394/419/424/427/430/463, 66/199/302/365/377/394/424/430/463/487 et 66/199/302/371/377/394/424/430/463/487.

7. Variants polypeptidiques selon la revendication 6, **caractérisés en ce que** les séquences d'acides aminés des variants polypeptidiques sont choisies parmi le groupe des SEQ ID NO 2 à 29.

8. Variants polypeptidiques selon la revendication 1 ou 2, **caractérisés en ce que** la séquence d'acides aminés des variants polypeptidiques contient des combinaisons de plusieurs substitutions d'acides aminés, dans lesquels les combinaisons des positions sont choisies parmi le groupe
66/99/302/364/377/389/394/419/424/427/430/447/463/465/469, 66/199/302/377/389/394/419/424/427/430/447/463/465/469, 66/199/302/363/364/371/377/389/394/419/424/427/430/447/463/464/465/469, 66/199/302/36313711377/389139414191424/42714301447/463/464/465/469, 66/199/302/364/367/371/377/389/394/418/419/424/427/430/436/440/447/463/464/ 465/
469/490, 66/199/302/367/371/377/389/394/418/419/424/427/430/436/440/447/463/464/465/ 469/
490,
66/199/302/363/367/371/377/394/424/430/463/490, 66/199/302/377/394/418/419/424/427/430/436/440/447/463, 66/199/302/377/389/394/424/430/457/463/464/465/469, 66/199/302/363/371/377/389/394/419/424/427/430/440/447/457/463/464/469/490, 66/199/302/377/394/424/430/463/447/490/469/465, 66/199/302/3771394/424/430/4631490/469/465/4191427/3711487, 66/199/302/371/377/394/419/424/427/430/447/453/463/465/469/487/490, 66/199/302/367/371/377/389/394/418/419/424/427/429/430/436/440/447/457/463/ 464/465/469/490, 66/199/302/371/377/389/394/419/424/427/430/436/447/453/456/462/463/465/469/ 490/487
et 66/199/302/367/371/377/389/394/418/419/424/427/429/430/436/440/447/453/456/ 457/462/463/464/465/469/487/490.

9. Variants polypeptidiques selon la revendication 8, **caractérisés en ce que** les séquences d'acides aminés des variants polypeptidiques sont choisies parmi le groupe des SEQ ID NO 30 à 45.

10. Polynucléotides isolés, **caractérisés en ce que** les polynucléotides possèdent des séquences de nucléotides, qui codent pour un variant polypeptidique selon l'une des revendications 1 à 9.

11. Additif à dissociation de toxine de fusarium, **caractérisé en ce que** l'additif contient au moins un variant polypeptidique selon l'une des revendications 1 à 9, et qu'éventuellement au moins un adjuvant est contenu.

12. Additif selon la revendication 11, **caractérisé en ce que** l'adjuvant est choisi parmi le groupe d'excipients inertes, de vitamines, de substances minérales, de substances phytogènes, d'enzymes et d'autres composants pour détoxifier des mycotoxines, tels que des enzymes de dégradation de mycotoxine, en particulier des aflatoxine oxydases, des ergotamine hydrolases, des ergotamine amidases, des zéaralénone estérases, des zéaralénone lactonases, des zéaralénone hydrolases, des ochratoxine amidases, des fumonisine aminotransférases, des aminopolyol aminoxydases, des déoxynivalénol époxydes hydrolases, des déoxynivalénol déshydrogénases, des déoxynivalénol oxydases, des trichothécène déshydrogénases, des trichothécène oxydases ; et des micro-organismes de dégradation de mycotoxine ; et des substances de liaison de mycotoxine, par exemple des parois de cellules microbiennes ou des matériaux inorganiques tels que la bentonite.

13. Utilisation d'un additif selon l'une des revendications 11 ou 12 pour dissocier au moins une toxine de fusarium dans des produits alimentaires ou aliments pour animaux, en particulier pastillés, en particulier pour les porcs, volailles, bovins, chevaux, poissons ou l'aquaculture.

14. Utilisation d'un additif selon l'une des revendications 11 ou 12 pour dissocier au moins une toxine de fusarium lors d'un procédé, en particulier à des températures d'au moins 50 °C, de manière préférée d'au moins 70 °C, pour fabriquer ou transformer des produits alimentaires ou des aliments pour animaux.

15. Variants polypeptidiques selon l'une des revendications 1 à 9 destinés à être utilisés dans une préparation de prophylaxie et/ou de traitement de mycotoxicoses à la toxine de fusarium.

16. Procédé pour dissocier par hydrolyse au moins une toxine de fusarium, **caractérisé en ce qu'**au moins une toxine de fusarium est amenée en contact avec au moins un variant polypeptidique selon l'une des revendications 1 à 9 ou au moins un additif selon l'une des revendications 11 ou 12, et que le mélange composé du variant polypeptidique et de la toxine de fusarium est soumis à un traitement thermique à au moins 50 °C, de manière préférée à au moins 70 °C, et que le mélange est amené en contact avec une quantité d'humidité suffisante pour la dissociation par hydrolyse soit pendant soit après le traitement thermique.

17. Procédé selon la revendication 16, **caractérisé en ce que** le variant polypeptidique ou l'additif est mélangé à un aliment pour animaux ou un produit alimentaire contaminé par au moins une toxine de fusarium, et éventuellement le traitement thermique est effectué pendant un procédé de pelletisation.

18. Procédé selon l'une des revendications 16 ou 17, **caractérisé en ce que** le variant polypeptidique est ajouté, par kilogramme d'aliment pour animaux ou de produit alimentaire, dans une plage de concentration de 5 U à 500 U, de manière préférée de 10 U à 300 U et de manière davantage préférée de 15 U à 100.


### SEQUENCE LISTING

<110> Erber Aktiengesellschaft
<120> FUSARIUMTOXIN SPALTENDE POLYPEPTIDVARIANTEN, ZUATZSTOFF ENTHALTEND DIESELBEN UND VERWENDUNG DERSELBEN SOWIE VERFAHREN ZUR SPALTUNG VON FUSARIUMTOXIN
<130> P05368PCT
<160> 46
<170> PatentIn version 3.5
<210> 1
   <211> 493
   <212> PRT
   <213> Artificial sequence
<220>
   <223> Modified from SEQ ID-Nr. 46
<400> 1
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asn Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Leu Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Leu Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn Asn Gly
           355                 360                 365
   Ile Glu Leu Leu Ser Ala Ala Phe Ala Lys Trp Arg Thr Pro Leu Trp
       370                 375                 380
   Arg Tyr Arg Phe Thr Gly Ile Pro Gly Ala Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ser Met Phe Gly Ser Leu Glu Gly Gly Ala Gly Ala Ser Asp Ile
               420                 425                 430
   Lys Leu Ala Thr Glu Met Ser Ala Ala Trp Val Ser Phe Ala Val His
           435                 440                 445
   Gly Val Pro Asp Gln Gly Thr Lys Ser His Trp Pro Arg Phe Glu Arg
   450 455 460
   Arg Gly Glu Ile Met Thr Phe Gly Ser Gln Val Gly Ser Gly Glu Gly
   465 470 475 480
   Leu Gly Val Ser Pro Ser Lys Ala Cys Gln Pro Ser Lys
   485 490
<210> 2
   <211> 493
   <212> PRT
   <213> Artificial sequence
<220>
   <223> Polypeptidvariante der SEQ ID-Nr. 1
<400> 2
   Gln Thr Asp Asp Pro Lys Leu Val Arg His Thr Gln Ser Gly Ala Val
   1 5 10 15
   Glu Gly Val Glu Gly Asp Val Glu Thr Phe Leu Gly Ile Pro Phe Ala
   20 25 30
   Ala Pro Pro Val Gly Asp Leu Arg Trp Arg Pro Pro Ala Pro Pro Arg
   35 40 45
   Ala Trp Ala Gly Thr Arg Asp Gly Arg Arg Phe Ala Pro Asp Cys Ile
   50 55 60
   Gly Asp Glu Arg Leu Arg Glu Gly Ser Arg Ala Ala Gly Thr Ser Glu
   65 70 75 80
   Asp Cys Leu Tyr Leu Asn Ile Trp Ser Pro Lys Gln Val Gly Lys Gly
   85 90 95
   Gly Leu Pro Val Met Ile Trp Val Tyr Gly Gly Gly Phe Ser Gly Gly
   100 105 110
   Ser Gly Ala Val Pro Tyr Tyr Asp Gly Ser Ala Leu Ala Gln Lys Gly
   115 120 125
   Val Val Val Val Thr Phe Asn Tyr Arg Ala Gly Ile Leu Gly Phe Leu
   130 135 140
   Ala His Pro Ala Leu Ser Lys Glu Ser Pro Asn Gly Val Ser Gly Asn
   145 150 155 160
   Tyr Gly Leu Leu Asp Met Leu Ala Ala Phe Lys Trp Val Gln Asn Asn
   165 170 175
   Ile Arg Glu Phe Gly Gly Asp Pro Asn Arg Val Thr Val Phe Gly Glu
   180 185 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn Asn Gly
           355                 360                 365
   Ile Glu Leu Leu Ser Ala Ala Phe Ala Lys Trp Arg Thr Pro Leu Trp
       370                 375                 380
   Arg Tyr Arg Phe Thr Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ser Met Phe Gly Ser Leu Ala Gly Gly Ala Gly Ala Ala Asp Ile
               420                 425                 430
   Lys Leu Ala Thr Glu Met Ser Ala Ala Trp Val Ser Phe Ala Val His
           435                 440                 445
   Gly Val Pro Asp Gln Gly Thr Lys Ser His Trp Pro Arg Phe Glu Arg
       450                 455                 460
   Arg Gly Glu Ile Met Thr Phe Gly Ser Gln Val Gly Ser Gly Glu Gly
   465                 470                 475                 480
   Leu Gly Val Ser Pro Ser Lys Ala Cys Gln Pro Ser Lys
                   485                 490
<210> 3
   <211> 493
   <212> PRT
   <213> Artificial sequence
<220>
   <223> Polypeptidvariante der SEQ ID-Nr. 1
<400> 3
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn Asn Gly
           355                 360                 365
   Ile Glu Leu Leu Ser Ala Ala Phe Val Lys Trp Arg Thr Pro Leu Trp
       370                 375                 380
   Arg Tyr Arg Phe Thr Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ser Met Phe Gly Ser Leu Ala Gly Gly Ala Gly Ala Ala Asp Ile
               420                 425                 430
   Lys Leu Ala Thr Glu Met Ser Ala Ala Trp Val Ser Phe Ala Val His
           435                 440                 445
   Gly Val Pro Asp Gln Gly Thr Lys Ser His Trp Pro Arg Phe Glu Arg
       450                 455                 460
   Arg Gly Glu Ile Met Thr Phe Gly Ser Gln Val Gly Ser Gly Glu Gly
   465                 470                 475                 480
   Leu Gly Val Ser Pro Ser Lys Ala Cys Gln Pro Ser Lys
                   485                 490
<210> 4
   <211> 493
   <212> PRT
   <213> Artificial sequence
<220>
   <223> Polypeptidvariante der SEQ ID-Nr. 1
<400> 4
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
   210 215 220
   Pro Leu Ala Thr Leu Ser Glu Ser Glu Ala Asn Gly Leu Glu Leu Gly
   225 230 235 240
   Ala Asp Ile Ser Ala Leu Arg Arg Ala Asp Ala Gly Glu Leu Thr Lys
   245 250 255
   Ile Ala Gln Ser Arg Ile Pro Met Ser Arg Gln Phe Thr Lys Pro Arg
   260 265 270
   Pro Met Gly Pro Ile Leu Asp Gly Tyr Val Leu Arg Thr Leu Asp Val
   275 280 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Ser Gln Phe Asn Asn Gly
   355 360 365
   Ile Glu Leu Leu Ser Ala Ala Phe Val Lys Trp Arg Thr Pro Leu Trp
   370 375 380
   Arg Tyr Arg Phe Thr Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Met Phe Gly Ser Leu Ala Gly Gly Ala Gly Ala Ala Asp Ile
   420 425 430
   Lys Leu Ala Thr Glu Met Ser Ala Ala Trp Val Ser Phe Ala Val His
   435 440 445
   Gly Val Pro Asp Gln Gly Thr Lys Ser His Trp Pro Arg Phe Asp Arg
   450 455 460
   Arg Gly Glu Ile Met Thr Phe Gly Ser Gln Val Gly Ser Gly Glu Gly
   465 470 475 480
   Leu Gly Val Ser Pro Ser Lys Ala Cys Gln Pro Ser Lys
   485 490
<210> 5
   <211> 493
   <212> PRT
   <213> Artificial sequence
<220>
   <223> Polypeptidvariante der SEQ ID-Nr. 1
<400> 5
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Met
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
   210 215 220
   Pro Leu Ala Thr Leu Ser Glu Ser Glu Ala Asn Gly Leu Glu Leu Gly
   225 230 235 240
   Ala Asp Ile Ser Ala Leu Arg Arg Ala Asp Ala Gly Glu Leu Thr Lys
   245 250 255
   Ile Ala Gln Ser Arg Ile Pro Met Ser Arg Gln Phe Thr Lys Pro Arg
   260 265 270
   Pro Met Gly Pro Ile Leu Asp Gly Tyr Val Leu Arg Thr Leu Asp Val
   275 280 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Val Gly Asp Ser Gln Phe Asn Asn Gly
   355 360 365
   Ile Glu Leu Phe Ser Ala Ala Phe Val Lys Trp Arg Thr Pro Leu Trp
   370 375 380
   Arg Tyr Arg Phe Thr Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Met Phe Gly Ser Leu Ala Gly Gly Ala Gly Ala Ala Asp Ile
   420 425 430
   Lys Leu Ala Thr Glu Met Ser Ala Ala Trp Thr Ser Phe Ala Val His
   435 440 445
   Gly Val Pro Asp Gln Gly Thr Lys Ser His Trp Pro Arg Phe Asp Arg
   450 455 460
   Arg Gly Glu Ile Met Thr Phe Gly Ser Gln Val Gly Ser Gly Glu Gly
   465 470 475 480
   Leu Gly Val Ser Pro Ser Lys Ala Cys Gln Pro Ser Lys
   485 490
<210> 6
   <211> 493
   <212> PRT
   <213> Artificial sequence
<220>
   <223> Polypeptidvariante der SEQ ID-Nr. 1
<400> 6
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asn Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225 230 235 240
   Ala Asp Ile Ser Ala Leu Arg Arg Ala Asp Ala Gly Glu Leu Thr Lys
   245 250 255
   Ile Ala Gln Ser Arg Ile Pro Met Ser Arg Gln Phe Thr Lys Pro Arg
   260 265 270
   Pro Met Gly Pro Ile Leu Asp Gly Tyr Val Leu Arg Thr Leu Asp Val
   275 280 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Ser Gln Phe Asn Asn Gly
   355 360 365
   Ile Glu Leu Leu Ser Ala Ala Phe Val Lys Trp Arg Thr Pro Leu Trp
   370 375 380
   Arg Tyr Arg Phe Thr Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Met Phe Gly Ser Leu Ala Gly Gly Ala Gly Ala Ala Asp Ile
   420 425 430
   Lys Leu Ala Thr Glu Met Ser Ala Ala Trp Val Ser Phe Ala Val His
   435 440 445
   Gly Val Pro Asp Gln Gly Thr Lys Ser His Trp Pro Arg Phe Asp Arg
   450 455 460
   Arg Gly Glu Ile Met Thr Phe Gly Ser Gln Val Gly Ser Gly Glu Gly
   465 470 475 480
   Leu Gly Val Ser Pro Ser Lys Ala Cys Gln Pro Ser Lys
   485 490
<210> 7
   <211> 493
   <212> PRT
   <213> Artificial sequence
<220>
   <223> Polypeptidvariante der SEQ ID-Nr. 1
<400> 7
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225 230 235 240
   Ala Asp Ile Ser Ala Leu Arg Arg Ala Asp Ala Gly Glu Leu Thr Lys
   245 250 255
   Ile Ala Gln Ser Arg Ile Pro Met Ser Arg Gln Phe Thr Lys Pro Arg
   260 265 270
   Pro Met Gly Pro Ile Leu Asp Gly Tyr Val Leu Arg Thr Leu Asp Val
   275 280 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Ser Gln Phe Asn Asn Gly
   355 360 365
   Ile Glu Leu Leu Ser Ala Ala Phe Val Lys Trp Arg Thr Pro Leu Trp
   370 375 380
   Arg Tyr Arg Phe Thr Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Met Phe Gly Ser Leu Glu Gly Gly Ala Gly Ala Ser Asp Ile
   420 425 430
   Lys Leu Ala Thr Glu Met Ser Ala Ala Trp Val Ser Phe Ala Val His
   435 440 445
   Gly Val Pro Asp Gln Gly Thr Lys Ser His Trp Pro Arg Phe Glu Arg
   450 455 460
   Arg Gly Glu Ile Met Thr Phe Gly Ser Gln Val Gly Ser Gly Glu Gly
   465 470 475 480
   Leu Gly Val Ser Pro Ser Lys Ala Cys Gln Pro Ser Lys
   485 490
<210> 8
   <211> 493
   <212> PRT
   <213> Artificial sequence
<220>
   <223> Polypeptidvariante der SEQ ID-Nr. 1
<400> 8
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser His Phe Asn Asn Gly
           355                 360                 365
   Ile Glu Leu Leu Ser Ala Ala Phe Val Lys Trp Arg Thr Pro Leu Trp
       370                 375                 380
   Arg Tyr Arg Phe Thr Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ser Met Phe Gly Ser Leu Ala Gly Gly Ala Gly Ala Ala Asp Ile
               420                 425                 430
   Lys Leu Ala Thr Glu Met Ser Ala Ala Trp Val Ser Phe Ala Val His
           435                 440                 445
   Gly Val Pro Asp Gln Gly Thr Lys Ser His Trp Pro Arg Phe Asp Arg
       450                 455                 460
   Arg Gly Glu Ile Met Thr Phe Gly Ser Gln Val Gly Ser Gly Glu Gly
   465                 470                 475                 480
   Leu Gly Val Ser Pro Ser Lys Ala Cys Gln Pro Ser Lys
                   485                 490
<210> 9
   <211> 493
   <212> PRT
   <213> Artificial sequence
<220>
   <223> Polypeptidvariante der SEQ ID-Nr. 1
<400> 9
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn Asn Gly
           355                 360                 365
   Ile Glu Leu Leu Ser Ala Ala Phe Val Lys Trp Arg Thr Pro Leu Trp
       370                 375                 380
   Arg Tyr Arg Phe Thr Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ser Met Phe Gly Ser Leu Ala Gly Gly Ala Gly Ala Ala Asp Ile
               420                 425                 430
   Lys Leu Ala Thr Glu Met Ser Ala Ala Trp Val Ser Phe Ala Val His
           435                 440                 445
   Gly Val Pro Asp Gln Gly Thr Lys Ser His Trp Pro Arg Phe Asp Arg
       450                 455                 460
   His Gly Glu Ile Met Thr Phe Gly Ser Gln Val Gly Ser Gly Glu Gly
   465                 470                 475                 480
   Leu Gly Val Ser Pro Ser Lys Ala Cys Gln Pro Ser Lys
                   485                 490
<210> 10
   <211> 493
   <212> PRT
   <213> Artificial sequence
<220>
   <223> Polypeptidvariante der SEQ ID-Nr. 1
<400> 10
   Gln Thr Asp Asp Pro Lys Leu Val Arg His Thr Gln Ser Gly Ala Val
   1 5 10 15
   Glu Gly Val Glu Gly Asp Val Glu Thr Phe Leu Gly Ile Pro Phe Ala
   20 25 30
   Ala Pro Pro Val Gly Asp Leu Arg Trp Arg Pro Pro Ala Pro Pro Arg
   35 40 45
   Ala Trp Ala Gly Thr Arg Asp Gly Arg Arg Phe Ala Pro Asp Cys Ile
   50 55 60
   Gly Asp Glu Arg Leu Arg Glu Gly Ser Arg Ala Ala Gly Thr Ser Glu
   65 70 75 80
   Asp Cys Leu Tyr Leu Asn Ile Trp Ser Pro Lys Gln Val Gly Lys Gly
   85 90 95
   Gly Leu Pro Val Met Ile Trp Val Tyr Gly Gly Gly Phe Ser Gly Gly
   100 105 110
   Ser Gly Ala Val Pro Tyr Tyr Asp Gly Ser Ala Leu Ala Gln Lys Gly
   115 120 125
   Val Val Val Val Thr Phe Asn Tyr Arg Ala Gly Ile Leu Gly Phe Leu
   130 135 140
   Ala His Pro Ala Leu Ser Lys Glu Ser Pro Asn Gly Val Ser Gly Asn
   145 150 155 160
   Tyr Gly Leu Leu Asp Met Leu Ala Ala Phe Lys Trp Val Gln Asn Asn
   165 170 175
   Ile Arg Glu Phe Gly Gly Asp Pro Asn Arg Val Thr Val Phe Gly Glu
   180 185 190
   Ser Ala Gly Ala Ser Ala Ile Gly Leu Leu Leu Thr Ser Pro Leu Ser
   195 200 205
   Glu Ser Ala Phe Asn Gln Ala Ile Leu Gln Ser Pro Gly Leu Ala Arg
   210 215 220
   Pro Leu Ala Thr Leu Ser Glu Ser Glu Ala Asn Gly Leu Glu Leu Gly
   225 230 235 240
   Ala Asp Ile Ser Ala Leu Arg Arg Ala Asp Ala Gly Glu Leu Thr Lys
   245 250 255
   Ile Ala Gln Ser Arg Ile Pro Met Ser Arg Gln Phe Thr Lys Pro Arg
   260 265 270
   Pro Met Gly Pro Ile Leu Asp Gly Tyr Val Leu Arg Thr Leu Asp Val
   275 280 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Ser Gln Phe Asn Asn Gly
   355 360 365
   Ile Glu Leu Leu Ser Ala Ala Phe Val Lys Trp Arg Thr Pro Leu Trp
   370 375 380
   Arg Tyr Arg Phe Thr Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Met Phe Gly Ser Leu Ala Gly Gly Ala Gly Ala Ala Asp Ile
   420 425 430
   Lys Leu Ala Thr Glu Met Ser Gly Ala Trp Val Ser Phe Ala Val His
   435 440 445
   Gly Val Pro Asp Gln Gly Thr Lys Ser His Trp Pro Arg Phe Asp Arg
   450 455 460
   Arg Gly Glu Ile Met Thr Phe Gly Ser Gln Val Gly Ser Gly Glu Gly
   465 470 475 480
   Leu Gly Val Ser Pro Ser Lys Ala Cys Gln Pro Ser Lys
   485 490
<210> 11
   <211> 493
   <212> PRT
   <213> Artificial sequence
<220>
   <223> Polypeptidvariante der SEQ ID-Nr. 1
<400> 11
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
   260 265 270
   Pro Met Gly Pro Ile Leu Asp Gly Tyr Val Leu Arg Thr Leu Asp Val
   275 280 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Ser Gln Phe Asn Asn Gly
   355 360 365
   Ile Glu Leu Leu Ser Ala Ala Phe Val Lys Trp Arg Thr Pro Leu Trp
   370 375 380
   Arg Tyr Arg Phe Thr Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Met Phe Gly Ser Leu Ala Gly Gly Ala Gly Ala Ala Asp Ile
   420 425 430
   Lys Leu Ala Thr Glu Met Ser Ala Ala Trp Val Ser Phe Ala Ala His
   435 440 445
   Gly Val Pro Asp Gln Gly Thr Lys Ser His Trp Pro Arg Phe Asp Arg
   450 455 460
   Arg Gly Glu Ile Met Thr Phe Gly Ser Gln Val Gly Ser Gly Glu Gly
   465 470 475 480
   Leu Gly Val Ser Pro Ser Lys Ala Cys Gln Pro Ser Lys
   485 490
<210> 12
   <211> 493
   <212> PRT
   <213> Artificial sequence
<220>
   <223> Polypeptidvariante der SEQ ID-Nr. 1
<400> 12
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn Asn Gly
           355                 360                 365
   Ile Glu Leu Leu Ser Ala Ala Phe Val Lys Trp Arg Thr Pro Leu Trp
       370                 375                 380
   Arg Tyr Arg Phe Thr Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ala Met Phe Gly Ser Leu Ala Gly Gly Ala Gly Ala Ala Asp Ile
               420                 425                 430
   Lys Leu Ala Thr Glu Met Ser Ala Ala Trp Val Ser Phe Ala Val His
           435                 440                 445
   Gly Val Pro Asp Gln Gly Thr Lys Ser His Trp Pro Arg Phe Asp Arg
       450                 455                 460
   Arg Gly Glu Ile Met Thr Phe Gly Ser Gln Val Gly Ser Gly Glu Gly
   465                 470                 475                 480
   Leu Gly Val Ser Pro Ser Lys Ala Cys Gln Pro Ser Lys
                   485                 490
<210> 13
   <211> 493
   <212> PRT
   <213> Artificial sequence
<220>
   <223> Polypeptidvariante der SEQ ID-Nr. 1
<400> 13
   Gln Thr Asp Asp Pro Lys Leu Val Arg His Thr Gln Ser Gly Ala Val
   1 5 10 15
   Glu Gly Val Glu Gly Asp Val Glu Thr Phe Leu Gly Ile Pro Phe Ala
   20 25 30
   Ala Pro Pro Val Gly Asp Leu Arg Trp Arg Pro Pro Ala Pro Pro Arg
   35 40 45
   Ala Trp Ala Gly Thr Arg Asp Gly Arg Arg Phe Ala Pro Asp Cys Ile
   50 55 60
   Gly Asp Glu Arg Leu Arg Glu Gly Ser Arg Ala Ala Gly Thr Ser Glu
   65 70 75 80
   Asp Cys Leu Tyr Leu Asn Ile Trp Ser Pro Lys Gln Val Gly Lys Gly
   85 90 95
   Gly Leu Pro Val Met Ile Trp Val Tyr Gly Gly Gly Phe Ser Gly Gly
   100 105 110
   Ser Gly Ala Val Pro Tyr Tyr Asp Gly Ser Ala Leu Ala Gln Lys Gly
   115 120 125
   Val Val Val Val Thr Phe Asn Tyr Arg Ala Gly Ile Leu Gly Phe Leu
   130 135 140
   Ala His Pro Ala Leu Ser Lys Glu Ser Pro Asn Gly Val Ser Gly Asn
   145 150 155 160
   Tyr Gly Leu Leu Asp Met Leu Ala Ala Phe Lys Trp Val Gln Asn Asn
   165 170 175
   Ile Arg Glu Phe Gly Gly Asp Pro Asn Arg Val Thr Val Phe Gly Glu
   180 185 190
   Ser Ala Gly Ala Ser Ala Ile Gly Leu Leu Leu Thr Ser Pro Leu Ser
   195 200 205
   Glu Ser Ala Phe Asn Gln Ala Ile Leu Gln Ser Pro Gly Leu Ala Arg
   210 215 220
   Pro Leu Ala Thr Leu Ser Glu Ser Glu Ala Asn Gly Leu Glu Leu Gly
   225 230 235 240
   Ala Asp Ile Ser Ala Leu Arg Arg Ala Asp Ala Gly Glu Leu Thr Lys
   245 250 255
   Ile Ala Gln Ser Arg Ile Pro Met Ser Arg Gln Phe Thr Lys Pro Arg
   260 265 270
   Pro Met Gly Pro Ile Leu Asp Gly Tyr Val Leu Arg Thr Leu Asp Val
   275 280 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Ser Gln Phe Asn Asn Gly
   355 360 365
   Ile Glu Leu Leu Ser Ala Ala Phe Val Lys Trp Arg Thr Pro Leu Trp
   370 375 380
   Arg Tyr Arg Phe Thr Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Met Phe Gly Ser Leu Ala Gly Gly Ala Gly Ala Ala Asp Ile
   420 425 430
   Lys Leu Ala Ala Glu Met Ser Ala Ala Trp Val Ser Phe Ala Val His
   435 440 445
   Gly Val Pro Asp Gln Gly Thr Lys Ser His Trp Pro Arg Phe Asp Arg
   450 455 460
   Arg Gly Glu Ile Met Thr Phe Gly Ser Gln Val Gly Ser Gly Glu Gly
   465 470 475 480
   Leu Gly Val Ser Pro Ser Lys Ala Cys Gln Pro Ser Lys
   485 490
<210> 14
   <211> 493
   <212> PRT
   <213> Artificial sequence
<220>
   <223> Polypeptidvariante der SEQ ID-Nr. 1
<400> 14
   Gln Thr Asp Asp Pro Lys Leu Val Arg His Thr Gln Ser Gly Ala Val
   1 5 10 15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Ser Leu Phe Asn Asn Gly
   355 360 365
   Ile Glu Leu Leu Ser Ala Ala Phe Val Lys Trp Arg Thr Pro Leu Trp
   370 375 380
   Arg Tyr Arg Phe Thr Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Met Phe Gly Ser Leu Ala Gly Gly Ala Gly Ala Ala Asp Ile
   420 425 430
   Lys Leu Ala Thr Glu Met Ser Ala Ala Trp Val Ser Phe Ala Val His
   435 440 445
   Gly Val Pro Asp Gln Gly Thr Lys Ser His Trp Pro Arg Phe Asp Arg
   450 455 460
   Arg Gly Glu Ile Met Thr Phe Gly Ser Gln Val Gly Ser Gly Glu Gly
   465 470 475 480
   Leu Gly Val Ser Pro Ser Lys Ala Cys Gln Pro Ser Lys
   485 490
<210> 15
   <211> 493
   <212> PRT
   <213> Artificial sequence
<220>
   <223> Polypeptidvariante der SEQ ID-Nr. 1
<400> 15
   Gln Thr Asp Asp Pro Lys Leu Val Arg His Thr Gln Ser Gly Ala Val
   1 5 10 15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn Asn Gly
           355                 360                 365
   Ile Glu Leu Leu Ser Ala Ala Phe Val Lys Trp Arg Thr Pro Leu Trp
       370                 375                 380
   Arg Tyr Arg Phe Thr Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ser Met Phe Gly Ser Leu Ala Gly Gly Ala Gly Ala Ala Asp Ile
               420                 425                 430
   Lys Leu Ala Thr Glu Met Ser Ala Ala Trp Val Ser Phe Ala Val His
           435                 440                 445
   Gly Val Pro Asp Gln Gly Thr Lys Ser His Trp Pro Arg Phe Asp Arg
       450                 455                 460
   Arg Gly Glu Ile Met Thr Phe Gly Ser Gln Val Gly Ser Gly Glu Gly
   465                 470                 475                 480
   Leu Gly Val Ser Pro Ser Lys Ala Cys Pro Pro Ser Lys
                   485                 490
<210> 16
   <211> 493
   <212> PRT
   <213> Artificial sequence
<220>
   <223> Polypeptidvariante der SEQ ID-Nr. 1
<400> 16
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn Asn Gly
           355                 360                 365
   Ile Glu Leu Leu Ser Ala Ala Phe Val Lys Trp Arg Thr Pro Leu Trp
       370                 375                 380
   Arg Tyr Arg Phe Thr Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ser Met Phe Gly Ser Leu Ala Gly Gly Ala Gly Ala Ala Asp Ile
               420                 425                 430
   Lys Leu Ala Thr Glu Met Ser Ala Ala Trp Val Ser Phe Ala Val His
           435                 440                 445
   Gly Val Pro Asp Gln Gly Thr Lys Ser His Trp Pro Arg Phe Asp Arg
       450                 455                 460
   Arg Gly Glu Ile Lys Thr Phe Gly Ser Gln Val Gly Ser Gly Glu Gly
   465                 470                 475                 480
   Leu Gly Val Ser Pro Ser Lys Ala Cys Gln Pro Ser Lys
                   485                 490
<210> 17
   <211> 493
   <212> PRT
   <213> Artificial sequence
<220>
   <223> Polypeptidvariante der SEQ ID-Nr. 1
<400> 17
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
   35 40 45
   Ala Trp Ala Gly Thr Arg Asp Gly Arg Arg Phe Ala Pro Asp Cys Ile
   50 55 60
   Gly Asp Glu Arg Leu Arg Glu Gly Ser Arg Ala Ala Gly Thr Ser Glu
   65 70 75 80
   Asp Cys Leu Tyr Leu Asn Ile Trp Ser Pro Lys Gln Val Gly Lys Gly
   85 90 95
   Gly Leu Pro Val Met Ile Trp Val Tyr Gly Gly Gly Phe Ser Gly Gly
   100 105 110
   Ser Gly Ala Val Pro Tyr Tyr Asp Gly Ser Ala Leu Ala Gln Lys Gly
   115 120 125
   Val Val Val Val Thr Phe Asn Tyr Arg Ala Gly Ile Leu Gly Phe Leu
   130 135 140
   Ala His Pro Ala Leu Ser Lys Glu Ser Pro Asn Gly Val Ser Gly Asn
   145 150 155 160
   Tyr Gly Leu Leu Asp Met Leu Ala Ala Phe Lys Trp Val Gln Asn Asn
   165 170 175
   Ile Arg Glu Phe Gly Gly Asp Pro Asn Arg Val Thr Val Phe Gly Glu
   180 185 190
   Ser Ala Gly Ala Ser Ala Ile Gly Leu Leu Leu Thr Ser Pro Leu Ser
   195 200 205
   Glu Ser Ala Phe Asn Gln Ala Ile Leu Gln Ser Pro Gly Leu Ala Arg
   210 215 220
   Pro Leu Ala Thr Leu Ser Glu Ser Glu Ala Asn Gly Leu Glu Leu Gly
   225 230 235 240
   Ala Asp Ile Ser Ala Leu Arg Arg Ala Asp Ala Gly Glu Leu Thr Lys
   245 250 255
   Ile Ala Gln Ser Arg Ile Pro Met Ser Arg Gln Phe Thr Lys Pro Arg
   260 265 270
   Pro Met Gly Pro Ile Leu Asp Gly Tyr Val Leu Arg Thr Leu Asp Val
   275 280 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Ser Gln Phe Asn Asn Gly
   355 360 365
   Ile Glu Leu Leu Ser Ala Ala Phe Val Lys Trp Arg Thr Pro Leu Trp
   370 375 380
   Arg Tyr Arg Phe Leu Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Met Phe Gly Ser Leu Ala Gly Gly Ala Gly Ala Ala Asp Ile
   420 425 430
   Lys Leu Ala Thr Glu Met Ser Ala Ala Trp Val Ser Phe Ala Val His
   435 440 445
   Gly Val Pro Asp Gln Gly Thr Lys Ser His Trp Pro Arg Phe Asp Arg
   450 455 460
   Arg Gly Glu Ile Met Thr Phe Gly Ser Gln Val Gly Ser Gly Glu Gly
   465 470 475 480
   Leu Gly Val Ser Pro Ser Lys Ala Cys Gln Pro Ser Lys
   485 490
<210> 18
   <211> 493
   <212> PRT
   <213> Artificial sequence
<220>
   <223> Polypeptidvariante der SEQ ID-Nr. 1
<400> 18
   Gln Thr Asp Asp Pro Lys Leu Val Arg His Thr Gln Ser Gly Ala Val
   1 5 10 15
   Glu Gly Val Glu Gly Asp Val Glu Thr Phe Leu Gly Ile Pro Phe Ala
   20 25 30
   Ala Pro Pro Val Gly Asp Leu Arg Trp Arg Pro Pro Ala Pro Pro Arg
   35 40 45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn Asn Gly
           355                 360                 365
   Ile Glu Leu Leu Ser Ala Ala Phe Val Lys Trp Arg Thr Pro Leu Trp
       370                 375                 380
   Arg Tyr Arg Phe Thr Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ser Met Phe Gly Ser Leu Ala Gly Gly Ala Gly Ala Ala Asp Ile
               420                 425                 430
   Lys Leu Ala Thr Glu Met Ser Ala Ala Trp Val Ser Phe Ala Val His
           435                 440                 445
   Gly Val Pro Asp Gln Gly Thr Lys Ser His Trp Pro Arg Phe Asp Arg
       450                 455                 460
   Ser Gly Glu Ile Met Thr Phe Gly Ser Gln Val Gly Ser Gly Glu Gly
   465                 470                 475                 480
   Leu Gly Val Ser Pro Ser Lys Ala Cys Gln Pro Ser Lys
                   485                 490
<210> 19
   <211> 493
   <212> PRT
   <213> Artificial sequence
<220>
   <223> Polypeptidvariante der SEQ ID-Nr. 1
<400> 19
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Ser Gln Phe Asn Asn Gly
   355 360 365
   Ile Glu Leu Leu Ser Ala Ala Phe Val Lys Trp Arg Thr Pro Leu Trp
   370 375 380
   Arg Tyr Arg Phe Thr Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Met Phe Gly Ser Leu Ala Gly Gly Ala Gly Ala Ala Asp Ile
   420 425 430
   Lys Leu Ala Thr Glu Met Ser Ala Ala Trp Val Ser Phe Ala Val His
   435 440 445
   Gly Val Pro Asp Gln Gly Thr Lys Ser His Trp Pro Arg Phe Asp Ile
   450 455 460
   Arg Gly Glu Ile Met Thr Phe Gly Ser Gln Val Gly Ser Gly Glu Gly
   465 470 475 480
   Leu Gly Val Ser Pro Ser Lys Ala Cys Gln Pro Ser Lys
   485 490
<210> 20
   <211> 493
   <212> PRT
   <213> Artificial sequence
<220>
   <223> Polypeptidvariante der SEQ ID-Nr. 1
<400> 20
   Gln Thr Asp Asp Pro Lys Leu Val Arg His Thr Gln Ser Gly Ala Val
   1 5 10 15
   Glu Gly Val Glu Gly Asp Val Glu Thr Phe Leu Gly Ile Pro Phe Ala
   20 25 30
   Ala Pro Pro Val Gly Asp Leu Arg Trp Arg Pro Pro Ala Pro Pro Arg
   35 40 45
   Ala Trp Ala Gly Thr Arg Asp Gly Arg Arg Phe Ala Pro Asp Cys Ile
   50 55 60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn Asn Gly
           355                 360                 365
   Ile Glu Leu Leu Ser Ala Ala Phe Val Lys Trp Arg Thr Pro Leu Trp
       370                 375                 380
   Arg Tyr Arg Phe Thr Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ser Met Phe Gly Ser Leu Ala Gly Gly Ala Gly Ala Ala Asp Ile
               420                 425                 430
   Lys Leu Ala Thr Glu Met Ser Ala Ala Trp Val Ser Phe Ala Val His
           435                 440                 445
   Gly Val Pro Asp Gln Gly Thr Lys Ser His Trp Pro Arg Phe Asp Arg
       450                 455                 460
   Gly Gly Glu Ile Met Thr Phe Gly Ser Gln Val Gly Ser Gly Glu Gly
   465                 470                 475                 480
   Leu Gly Val Ser Pro Ser Lys Ala Cys Gln Pro Ser Lys
                   485                 490
<210> 21
   <211> 493
   <212> PRT
   <213> Artificial sequence
<220>
   <223> Polypeptidvariante der SEQ ID-Nr. 1
<400> 21
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn Asn Gly
           355                 360                 365
   Ile Glu Leu Leu Ser Ala Ala Phe Val Lys Trp Arg Thr Pro Leu Trp
       370                 375                 380
   Arg Tyr Arg Phe Thr Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ser Met Phe Gly Ser Leu Ala Gly Gly Ala Gly Ala Ala Asp Ile
               420                 425                 430
   Lys Leu Ala Thr Glu Met Ser Ser Ala Trp Val Ser Phe Ala Val His
           435                 440                 445
   Gly Val Pro Asp Gln Gly Thr Lys Ser His Trp Pro Arg Phe Asp Arg
       450                 455                 460
   Arg Gly Glu Ile Met Thr Phe Gly Ser Gln Val Gly Ser Gly Glu Gly
   465                 470                 475                 480
   Leu Gly Val Ser Pro Ser Lys Ala Cys Gln Pro Ser Lys
                   485                 490
<210> 22
   <211> 493
   <212> PRT
   <213> Artificial sequence
<220>
   <223> Polypeptidvariante der SEQ ID-Nr. 1
<400> 22
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn Asn Gly
   355 360 365
   Ile Glu Leu Leu Ser Ala Ala Phe Val Lys Trp Arg Thr Pro Leu Trp
   370 375 380
   Arg Tyr Arg Phe Thr Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Met Phe Gly Ser Leu Ala Gly Gly Ala Gly Ala Ala Asp Ile
   420 425 430
   Lys Leu Ala Thr Glu Met Ser Ala Ala Trp Val Ser Phe Ala Val His
   435 440 445
   Gly Val Pro Asp Gln Gly Thr Lys Thr His Trp Pro Arg Phe Asp Arg
   450 455 460
   Arg Gly Glu Ile Met Thr Phe Gly Ser Gln Val Gly Ser Gly Glu Gly
   465 470 475 480
   Leu Gly Val Ser Pro Ser Lys Ala Cys Gln Pro Ser Lys
   485 490
<210> 23
   <211> 493
   <212> PRT
   <213> Artificial sequence
<220>
   <223> Polypeptidvariante der SEQ ID-Nr. 1
<400> 23
   Gln Thr Asp Asp Pro Lys Leu Val Arg His Thr Gln Ser Gly Ala Val
   1 5 10 15
   Glu Gly Val Glu Gly Asp Val Glu Thr Phe Leu Gly Ile Pro Phe Ala
   20 25 30
   Ala Pro Pro Val Gly Asp Leu Arg Trp Arg Pro Pro Ala Pro Pro Arg
   35 40 45
   Ala Trp Ala Gly Thr Arg Asp Gly Arg Arg Phe Ala Pro Asp Cys Ile
   50 55 60
   Gly Asp Glu Arg Leu Arg Glu Gly Ser Arg Ala Ala Gly Thr Ser Glu
   65 70 75 80
   Asp Cys Leu Tyr Leu Asn Ile Trp Ser Pro Lys Gln Val Gly Lys Gly
   85 90 95
   Gly Leu Pro Val Met Ile Trp Val Tyr Gly Gly Gly Phe Ser Gly Gly
   100 105 110
   Ser Gly Ala Val Pro Tyr Tyr Asp Gly Ser Ala Leu Ala Gln Lys Gly
   115 120 125
   Val Val Val Val Thr Phe Asn Tyr Arg Ala Gly Ile Leu Gly Phe Leu
   130 135 140
   Ala His Pro Ala Leu Ser Lys Glu Ser Pro Asn Gly Val Ser Gly Asn
   145 150 155 160
   Tyr Gly Leu Leu Asp Met Leu Ala Ala Phe Lys Trp Val Gln Asn Asn
   165 170 175
   Ile Arg Glu Phe Gly Gly Asp Pro Asn Arg Val Thr Val Phe Gly Glu
   180 185 190
   Ser Ala Gly Ala Ser Ala Ile Gly Leu Leu Leu Thr Ser Pro Leu Ser
   195 200 205
   Glu Ser Ala Phe Asn Gln Ala Ile Leu Gln Ser Pro Gly Leu Ala Arg
   210 215 220
   Pro Leu Ala Thr Leu Ser Glu Ser Glu Ala Asn Gly Leu Glu Leu Gly
   225 230 235 240
   Ala Asp Ile Ser Ala Leu Arg Arg Ala Asp Ala Gly Glu Leu Thr Lys
   245 250 255
   Ile Ala Gln Ser Arg Ile Pro Met Ser Arg Gln Phe Thr Lys Pro Arg
   260 265 270
   Pro Met Gly Pro Ile Leu Asp Gly Tyr Val Leu Arg Thr Leu Asp Val
   275 280 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Ser Gln Phe Asn Asn Gly
           355                 360                 365
   Ile Glu Leu Leu Ser Ala Ala Phe Val Lys Trp Arg Thr Pro Leu Trp
       370                 375                 380
   Arg Tyr Arg Phe Thr Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ser Met Phe Gly Ser Leu Ala Gly Gly Ala Gly Ala Ala Asp Ile
               420                 425                 430
   Lys Leu Ala Ser Glu Met Ser Ala Ala Trp Val Ser Phe Ala Val His
           435                 440                 445
   Gly Val Pro Asp Gln Gly Thr Lys Ser His Trp Pro Arg Phe Asp Arg
       450                 455                 460
   Arg Gly Glu Ile Met Thr Phe Gly Ser Gln Val Gly Ser Gly Glu Gly
   465                 470                 475                 480
   Leu Gly Val Ser Pro Ser Lys Ala Cys Gln Pro Ser Lys
                   485                 490
<210> 24
   <211> 493
   <212> PRT
   <213> Artificial sequence
<220>
   <223> Polypeptidvariante der SEQ ID-Nr. 1
<400> 24
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
   100 105 110
   Ser Gly Ala Val Pro Tyr Tyr Asp Gly Ser Ala Leu Ala Gln Lys Gly
   115 120 125
   Val Val Val Val Thr Phe Asn Tyr Arg Ala Gly Ile Leu Gly Phe Leu
   130 135 140
   Ala His Pro Ala Leu Ser Lys Glu Ser Pro Asn Gly Val Ser Gly Asn
   145 150 155 160
   Tyr Gly Leu Leu Asp Met Leu Ala Ala Phe Lys Trp Val Gln Asn Asn
   165 170 175
   Ile Arg Glu Phe Gly Gly Asp Pro Asn Arg Val Thr Val Phe Gly Glu
   180 185 190
   Ser Ala Gly Ala Ser Ala Ile Gly Leu Leu Leu Thr Ser Pro Leu Ser
   195 200 205
   Glu Ser Ala Phe Asn Gln Ala Ile Leu Gln Ser Pro Gly Leu Ala Arg
   210 215 220
   Pro Leu Ala Thr Leu Ser Glu Ser Glu Ala Asn Gly Leu Glu Leu Gly
   225 230 235 240
   Ala Asp Ile Ser Ala Leu Arg Arg Ala Asp Ala Gly Glu Leu Thr Lys
   245 250 255
   Ile Ala Gln Ser Arg Ile Pro Met Ser Arg Gln Phe Thr Lys Pro Arg
   260 265 270
   Pro Met Gly Pro Ile Leu Asp Gly Tyr Val Leu Arg Thr Leu Asp Val
   275 280 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Thr Gln Phe Asn Asn Gly
   355 360 365
   Ile Glu Val Leu Ser Ala Ala Phe Val Lys Trp Arg Thr Pro Leu Trp
   370 375 380
   Arg Tyr Arg Phe Thr Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Met Phe Gly Ser Leu Ala Gly Gly Ala Gly Ala Ala Asp Ile
   420 425 430
   Lys Leu Ala Thr Glu Met Ser Ala Ala Trp Val Ser Phe Ala Val His
   435 440 445
   Gly Val Pro Asp Gln Gly Thr Lys Ser His Trp Pro Arg Phe Asp Arg
   450 455 460
   Arg Gly Glu Ile Met Thr Phe Gly Ser Gln Val Gly Ser Gly Glu Gly
   465 470 475 480
   Leu Gly Val Ser Pro Ser Lys Ala Cys Gln Pro Ser Lys
   485 490
<210> 25
   <211> 493
   <212> PRT
   <213> Artificial sequence
<220>
   <223> Polypeptidvariante der SEQ ID-Nr. 1
<400> 25
   Gln Thr Asp Asp Pro Lys Leu Val Arg His Thr Gln Ser Gly Ala Val
   1 5 10 15
   Glu Gly Val Glu Gly Asp Val Glu Thr Phe Leu Gly Ile Pro Phe Ala
   20 25 30
   Ala Pro Pro Val Gly Asp Leu Arg Trp Arg Pro Pro Ala Pro Pro Arg
   35 40 45
   Ala Trp Ala Gly Thr Arg Asp Gly Arg Arg Phe Ala Pro Asp Cys Ile
   50 55 60
   Gly Asp Glu Arg Leu Arg Glu Gly Ser Arg Ala Ala Gly Thr Ser Glu
   65 70 75 80
   Asp Cys Leu Tyr Leu Asn Ile Trp Ser Pro Lys Gln Val Gly Lys Gly
   85 90 95
   Gly Leu Pro Val Met Ile Trp Val Tyr Gly Gly Gly Phe Ser Gly Gly
   100 105 110
   Ser Gly Ala Val Pro Tyr Tyr Asp Gly Ser Ala Leu Ala Gln Lys Gly
   115 120 125
   Val Val Val Val Thr Phe Asn Tyr Arg Ala Gly Ile Leu Gly Phe Leu
   130 135 140
   Ala His Pro Ala Leu Ser Lys Glu Ser Pro Asn Gly Val Ser Gly Asn
   145 150 155 160
   Tyr Gly Leu Leu Asp Met Leu Ala Ala Phe Lys Trp Val Gln Asn Asn
   165 170 175
   Ile Arg Glu Phe Gly Gly Asp Pro Asn Arg Val Thr Val Phe Gly Glu
   180 185 190
   Ser Ala Gly Ala Ser Ala Ile Gly Leu Leu Leu Thr Ser Pro Leu Ser
   195 200 205
   Glu Ser Ala Phe Asn Gln Ala Ile Leu Gln Ser Pro Gly Leu Ala Arg
   210 215 220
   Pro Leu Ala Thr Leu Ser Glu Ser Glu Ala Asn Gly Leu Glu Leu Gly
   225 230 235 240
   Ala Asp Ile Ser Ala Leu Arg Arg Ala Asp Ala Gly Glu Leu Thr Lys
   245 250 255
   Ile Ala Gln Ser Arg Ile Pro Met Ser Arg Gln Phe Thr Lys Pro Arg
   260 265 270
   Pro Met Gly Pro Ile Leu Asp Gly Tyr Val Leu Arg Thr Leu Asp Val
   275 280 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Ser Gln Phe Asn Asn Gly
   355 360 365
   Ile Glu Leu Leu Ser Ala Ala Phe Val Lys Trp Arg Thr Pro Leu Trp
   370 375 380
   Arg Tyr Arg Phe Thr Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Met Phe Gly Ser Leu Ala Gly Gly Ala Gly Ala Ala Asp Ile
   420 425 430
   Lys Leu Ala Thr Glu Met Ser Ala Ala Trp Val Ser Phe Ala Ala His
   435 440 445
   Gly Val Pro Asp Arg Gly Thr Lys Ser His Trp Pro Arg Phe Asp Arg
   450 455 460
   Arg Gly Glu Ile Met Thr Phe Gly Ser Gln Val Gly Ser Gly Glu Gly
   465 470 475 480
   Leu Gly Val Ser Pro Ser Lys Ala Cys Gln Pro Ser Lys
   485 490
<210> 26
   <211> 493
   <212> PRT
   <213> Artificial sequence
<220>
   <223> Polypeptidvariante der SEQ ID-Nr. 1
<400> 26
   Gln Thr Asp Asp Pro Lys Leu Val Arg His Thr Gln Ser Gly Ala Val
   1 5 10 15
   Glu Gly Val Glu Gly Asp Val Glu Thr Phe Leu Gly Ile Pro Phe Ala
   20 25 30
   Ala Pro Pro Val Gly Asp Leu Arg Trp Arg Pro Pro Ala Pro Pro Arg
   35 40 45
   Ala Trp Ala Gly Thr Arg Asp Gly Arg Arg Phe Ala Pro Asp Cys Ile
   50 55 60
   Gly Asp Glu Arg Leu Arg Glu Gly Ser Arg Ala Ala Gly Thr Ser Glu
   65 70 75 80
   Asp Cys Leu Tyr Leu Asn Ile Trp Ser Pro Lys Gln Val Gly Lys Gly
   85 90 95
   Gly Leu Pro Val Met Ile Trp Val Tyr Gly Gly Gly Phe Ser Gly Gly
   100 105 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn Asn Gly
           355                 360                 365
   Ile Glu Leu Leu Ser Ala Ala Phe Val Lys Trp Arg Thr Pro Leu Trp
       370                 375                 380
   Arg Tyr Arg Phe Thr Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ser Met Phe Gly Ser Leu Ala Gly Gly Ala Gly Ala Ala Asp Ile
               420                 425                 430
   Lys Leu Ala Thr Glu Met Ser Ala Ala Trp Val Ser Phe Ala Val His
           435                 440                 445
   Gly Val Pro Asp Gln Gly Thr Gln Ser His Trp Pro Arg Tyr Asp Arg
       450                 455                 460
   Arg Gly Glu Ile Met Thr Phe Gly Ser Gln Val Gly Ser Gly Glu Gly
   465                 470                 475                 480
   Leu Gly Val Ser Pro Ser Lys Ala Cys Gln Pro Ser Lys
                   485                 490
<210> 27
   <211> 493
   <212> PRT
   <213> Artificial sequence
<220>
   <223> Polypeptidvariante der SEQ ID-Nr. 1
<400> 27
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn Asn Gly
           355                 360                 365
   Ile Glu Leu Leu Ser Ala Ala Phe Val Lys Trp Arg Thr Pro Leu Trp
       370                 375                 380
   Arg Tyr Arg Phe Thr Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Val Phe Gly Ser Leu Ala Gly Gly Val Gly Ala Ala Asp Ile
   420 425 430
   Lys Leu Ala Thr Glu Met Ser Ala Ala Trp Val Ser Phe Ala Val His
   435 440 445
   Gly Val Pro Asp Gln Gly Thr Lys Ser His Trp Pro Arg Phe Asp Arg
   450 455 460
   Arg Gly Glu Ile Met Thr Phe Gly Ser Gln Val Gly Ser Gly Glu Gly
   465 470 475 480
   Leu Gly Val Ser Pro Ser Lys Ala Cys Gln Pro Ser Lys
   485 490
<210> 28
   <211> 493
   <212> PRT
   <213> Artificial sequence
<220>
   <223> Polypeptidvariante der SEQ ID-Nr. 1
<400> 28
   Gln Thr Asp Asp Pro Lys Leu Val Arg His Thr Gln Ser Gly Ala Val
   1 5 10 15
   Glu Gly Val Glu Gly Asp Val Glu Thr Phe Leu Gly Ile Pro Phe Ala
   20 25 30
   Ala Pro Pro Val Gly Asp Leu Arg Trp Arg Pro Pro Ala Pro Pro Arg
   35 40 45
   Ala Trp Ala Gly Thr Arg Asp Gly Arg Arg Phe Ala Pro Asp Cys Ile
   50 55 60
   Gly Asp Glu Arg Leu Arg Glu Gly Ser Arg Ala Ala Gly Thr Ser Glu
   65 70 75 80
   Asp Cys Leu Tyr Leu Asn Ile Trp Ser Pro Lys Gln Val Gly Lys Gly
   85 90 95
   Gly Leu Pro Val Met Ile Trp Val Tyr Gly Gly Gly Phe Ser Gly Gly
   100 105 110
   Ser Gly Ala Val Pro Tyr Tyr Asp Gly Ser Ala Leu Ala Gln Lys Gly
   115 120 125
   Val Val Val Val Thr Phe Asn Tyr Arg Ala Gly Ile Leu Gly Phe Leu
   130 135 140
   Ala His Pro Ala Leu Ser Lys Glu Ser Pro Asn Gly Val Ser Gly Asn
   145 150 155 160
   Tyr Gly Leu Leu Asp Met Leu Ala Ala Phe Lys Trp Val Gln Asn Asn
   165 170 175
   Ile Arg Glu Phe Gly Gly Asp Pro Asn Arg Val Thr Val Phe Gly Glu
   180 185 190
   Ser Ala Gly Ala Ser Ala Ile Gly Leu Leu Leu Thr Ser Pro Leu Ser
   195 200 205
   Glu Ser Ala Phe Asn Gln Ala Ile Leu Gln Ser Pro Gly Leu Ala Arg
   210 215 220
   Pro Leu Ala Thr Leu Ser Glu Ser Glu Ala Asn Gly Leu Glu Leu Gly
   225 230 235 240
   Ala Asp Ile Ser Ala Leu Arg Arg Ala Asp Ala Gly Glu Leu Thr Lys
   245 250 255
   Ile Ala Gln Ser Arg Ile Pro Met Ser Arg Gln Phe Thr Lys Pro Arg
   260 265 270
   Pro Met Gly Pro Ile Leu Asp Gly Tyr Val Leu Arg Thr Leu Asp Val
   275 280 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Ser Gln Ile Asn Asn Gly
   355 360 365
   Ile Glu Leu Leu Ser Ala Ala Phe Val Lys Trp Arg Thr Pro Leu Trp
   370 375 380
   Arg Tyr Arg Phe Thr Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Met Phe Gly Ser Leu Ala Gly Gly Ala Gly Ala Ala Asp Ile
   420 425 430
   Lys Leu Ala Thr Glu Met Ser Ala Ala Trp Val Ser Phe Ala Val His
   435 440 445
   Gly Val Pro Asp Gln Gly Thr Lys Ser His Trp Pro Arg Phe Asp Arg
   450 455 460
   Arg Gly Glu Ile Met Thr Phe Gly Ser Gln Val Gly Ser Gly Glu Gly
   465 470 475 480
   Leu Gly Val Ser Pro Ser Asn Ala Cys Gln Pro Ser Lys
   485 490
<210> 29
   <211> 493
   <212> PRT
   <213> Artificial sequence
<220>
   <223> Polypeptidvariante der SEQ ID-Nr. 1
<400> 29
   Gln Thr Asp Asp Pro Lys Leu Val Arg His Thr Gln Ser Gly Ala Val
   1 5 10 15
   Glu Gly Val Glu Gly Asp Val Glu Thr Phe Leu Gly Ile Pro Phe Ala
   20 25 30
   Ala Pro Pro Val Gly Asp Leu Arg Trp Arg Pro Pro Ala Pro Pro Arg
   35 40 45
   Ala Trp Ala Gly Thr Arg Asp Gly Arg Arg Phe Ala Pro Asp Cys Ile
   50 55 60
   Gly Asp Glu Arg Leu Arg Glu Gly Ser Arg Ala Ala Gly Thr Ser Glu
   65 70 75 80
   Asp Cys Leu Tyr Leu Asn Ile Trp Ser Pro Lys Gln Val Gly Lys Gly
   85 90 95
   Gly Leu Pro Val Met Ile Trp Val Tyr Gly Gly Gly Phe Ser Gly Gly
   100 105 110
   Ser Gly Ala Val Pro Tyr Tyr Asp Gly Ser Ala Leu Ala Gln Lys Gly
   115 120 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn Asn Gly
           355                 360                 365
   Ile Glu Met Leu Ser Ala Ala Phe Val Lys Trp Arg Thr Pro Leu Trp
       370                 375                 380
   Arg Tyr Arg Phe Thr Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
   405 410 415
   Val Ser Met Phe Gly Ser Leu Ala Gly Gly Ala Gly Ala Ala Asp Ile
   420 425 430
   Lys Leu Ala Thr Glu Met Ser Ala Ala Trp Val Ser Phe Ala Val His
   435 440 445
   Gly Val Pro Asp Gln Gly Thr Lys Ser His Trp Pro Arg Phe Asp Arg
   450 455 460
   Arg Gly Glu Ile Met Thr Phe Gly Ser Gln Val Gly Ser Gly Glu Gly
   465 470 475 480
   Leu Gly Val Ser Pro Ser Asn Ala Cys Gln Pro Ser Lys
   485 490
<210> 30
   <211> 493
   <212> PRT
   <213> Artificial sequence
<220>
   <223> Polypeptidvariante der SEQ ID-Nr. 1
<400> 30
   Gln Thr Asp Asp Pro Lys Leu Val Arg His Thr Gln Ser Gly Ala Val
   1 5 10 15
   Glu Gly Val Glu Gly Asp Val Glu Thr Phe Leu Gly Ile Pro Phe Ala
   20 25 30
   Ala Pro Pro Val Gly Asp Leu Arg Trp Arg Pro Pro Ala Pro Pro Arg
   35 40 45
   Ala Trp Ala Gly Thr Arg Asp Gly Arg Arg Phe Ala Pro Asp Cys Ile
   50 55 60
   Gly Asp Glu Arg Leu Arg Glu Gly Ser Arg Ala Ala Gly Thr Ser Glu
   65 70 75 80
   Asp Cys Leu Tyr Leu Asn Ile Trp Ser Pro Lys Gln Val Gly Lys Gly
   85 90 95
   Gly Leu Pro Val Met Ile Trp Val Tyr Gly Gly Gly Phe Ser Gly Gly
   100 105 110
   Ser Gly Ala Val Pro Tyr Tyr Asp Gly Ser Ala Leu Ala Gln Lys Gly
   115 120 125
   Val Val Val Val Thr Phe Asn Tyr Arg Ala Gly Ile Leu Gly Phe Leu
   130 135 140
   Ala His Pro Ala Leu Ser Lys Glu Ser Pro Asn Gly Val Ser Gly Asn
   145 150 155 160
   Tyr Gly Leu Leu Asp Met Leu Ala Ala Phe Lys Trp Val Gln Asn Asn
   165 170 175
   Ile Arg Glu Phe Gly Gly Asp Pro Asn Arg Val Thr Val Phe Gly Glu
   180 185 190
   Ser Ala Gly Ala Ser Ala Ile Gly Leu Leu Leu Thr Ser Pro Leu Ser
   195 200 205
   Glu Ser Ala Phe Asn Gln Ala Ile Leu Gln Ser Pro Gly Leu Ala Arg
   210 215 220
   Pro Leu Ala Thr Leu Ser Glu Ser Glu Ala Asn Gly Leu Glu Leu Gly
   225 230 235 240
   Ala Asp Ile Ser Ala Leu Arg Arg Ala Asp Ala Gly Glu Leu Thr Lys
   245 250 255
   Ile Ala Gln Ser Arg Ile Pro Met Ser Arg Gln Phe Thr Lys Pro Arg
   260 265 270
   Pro Met Gly Pro Ile Leu Asp Gly Tyr Val Leu Arg Thr Leu Asp Val
   275 280 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Ser Leu Phe Asn Asn Gly
   355 360 365
   Ile Glu Leu Leu Ser Ala Ala Phe Val Lys Trp Arg Thr Pro Leu Trp
   370 375 380
   Arg Tyr Arg Phe Leu Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Val Phe Gly Ser Leu Ala Gly Gly Val Gly Ala Ala Asp Ile
   420 425 430
   Lys Leu Ala Thr Glu Met Ser Ala Ala Trp Val Ser Phe Ala Ala His
   435 440 445
   Gly Val Pro Asp Gln Gly Thr Lys Ser His Trp Pro Arg Phe Asp Arg
   450 455 460
   Ser Gly Glu Ile Lys Thr Phe Gly Ser Gln Val Gly Ser Gly Glu Gly
   465 470 475 480
   Leu Gly Val Ser Pro Ser Lys Ala Cys Gln Pro Ser Lys
   485 490
<210> 31
   <211> 493
   <212> PRT
   <213> Artificial sequence
<220>
   <223> Polypeptidvariante der SEQ ID-Nr. 1
<400> 31
   Gln Thr Asp Asp Pro Lys Leu Val Arg His Thr Gln Ser Gly Ala Val
   1 5 10 15
   Glu Gly Val Glu Gly Asp Val Glu Thr Phe Leu Gly Ile Pro Phe Ala
   20 25 30
   Ala Pro Pro Val Gly Asp Leu Arg Trp Arg Pro Pro Ala Pro Pro Arg
   35 40 45
   Ala Trp Ala Gly Thr Arg Asp Gly Arg Arg Phe Ala Pro Asp Cys Ile
   50 55 60
   Gly Asp Glu Arg Leu Arg Glu Gly Ser Arg Ala Ala Gly Thr Ser Glu
   65 70 75 80
   Asp Cys Leu Tyr Leu Asn Ile Trp Ser Pro Lys Gln Val Gly Lys Gly
   85 90 95
   Gly Leu Pro Val Met Ile Trp Val Tyr Gly Gly Gly Phe Ser Gly Gly
   100 105 110
   Ser Gly Ala Val Pro Tyr Tyr Asp Gly Ser Ala Leu Ala Gln Lys Gly
   115 120 125
   Val Val Val Val Thr Phe Asn Tyr Arg Ala Gly Ile Leu Gly Phe Leu
   130 135 140
   Ala His Pro Ala Leu Ser Lys Glu Ser Pro Asn Gly Val Ser Gly Asn
   145 150 155 160
   Tyr Gly Leu Leu Asp Met Leu Ala Ala Phe Lys Trp Val Gln Asn Asn
   165 170 175
   Ile Arg Glu Phe Gly Gly Asp Pro Asn Arg Val Thr Val Phe Gly Glu
   180 185 190
   Ser Ala Gly Ala Ser Ala Ile Gly Leu Leu Leu Thr Ser Pro Leu Ser
   195 200 205
   Glu Ser Ala Phe Asn Gln Ala Ile Leu Gln Ser Pro Gly Leu Ala Arg
   210 215 220
   Pro Leu Ala Thr Leu Ser Glu Ser Glu Ala Asn Gly Leu Glu Leu Gly
   225 230 235 240
   Ala Asp Ile Ser Ala Leu Arg Arg Ala Asp Ala Gly Glu Leu Thr Lys
   245 250 255
   Ile Ala Gln Ser Arg Ile Pro Met Ser Arg Gln Phe Thr Lys Pro Arg
   260 265 270
   Pro Met Gly Pro Ile Leu Asp Gly Tyr Val Leu Arg Thr Leu Asp Val
   275 280 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Ser Gln Phe Asn Asn Gly
   355 360 365
   Ile Glu Leu Leu Ser Ala Ala Phe Val Lys Trp Arg Thr Pro Leu Trp
   370 375 380
   Arg Tyr Arg Phe Leu Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Val Phe Gly Ser Leu Ala Gly Gly Val Gly Ala Ala Asp Ile
   420 425 430
   Lys Leu Ala Thr Glu Met Ser Ala Ala Trp Val Ser Phe Ala Ala His
   435 440 445
   Gly Val Pro Asp Gln Gly Thr Lys Ser His Trp Pro Arg Phe Asp Arg
   450 455 460
   Ser Gly Glu Ile Lys Thr Phe Gly Ser Gln Val Gly Ser Gly Glu Gly
   465 470 475 480
   Leu Gly Val Ser Pro Ser Lys Ala Cys Gln Pro Ser Lys
   485 490
<210> 32
   <211> 493
   <212> PRT
   <213> Artificial sequence
<220>
   <223> Polypeptidvariante der SEQ ID-Nr. 1
<400> 32
   Gln Thr Asp Asp Pro Lys Leu Val Arg His Thr Gln Ser Gly Ala Val
   1 5 10 15
   Glu Gly Val Glu Gly Asp Val Glu Thr Phe Leu Gly Ile Pro Phe Ala
   20 25 30
   Ala Pro Pro Val Gly Asp Leu Arg Trp Arg Pro Pro Ala Pro Pro Arg
   35 40 45
   Ala Trp Ala Gly Thr Arg Asp Gly Arg Arg Phe Ala Pro Asp Cys Ile
   50 55 60
   Gly Asp Glu Arg Leu Arg Glu Gly Ser Arg Ala Ala Gly Thr Ser Glu
   65 70 75 80
   Asp Cys Leu Tyr Leu Asn Ile Trp Ser Pro Lys Gln Val Gly Lys Gly
   85 90 95
   Gly Leu Pro Val Met Ile Trp Val Tyr Gly Gly Gly Phe Ser Gly Gly
   100 105 110
   Ser Gly Ala Val Pro Tyr Tyr Asp Gly Ser Ala Leu Ala Gln Lys Gly
   115 120 125
   Val Val Val Val Thr Phe Asn Tyr Arg Ala Gly Ile Leu Gly Phe Leu
   130 135 140
   Ala His Pro Ala Leu Ser Lys Glu Ser Pro Asn Gly Val Ser Gly Asn
   145 150 155 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Thr Leu Phe Asn Asn Gly
           355                 360                 365
   Ile Glu Val Leu Ser Ala Ala Phe Val Lys Trp Arg Thr Pro Leu Trp
       370                 375                 380
   Arg Tyr Arg Phe Leu Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ser Val Phe Gly Ser Leu Ala Gly Gly Val Gly Ala Ala Asp Ile
               420                 425                 430
   Lys Leu Ala Thr Glu Met Ser Ala Ala Trp Val Ser Phe Ala Ala His
           435                 440                 445
   Gly Val Pro Asp Gln Gly Thr Lys Ser His Trp Pro Arg Phe Asp Ile
       450                 455                 460
   Ser Gly Glu Ile Lys Thr Phe Gly Ser Gln Val Gly Ser Gly Glu Gly
   465                 470                 475                 480
   Leu Gly Val Ser Pro Ser Lys Ala Cys Gln Pro Ser Lys
                   485                 490
<210> 33
   <211> 493
   <212> PRT
   <213> Artificial sequence
<220>
   <223> Polypeptidvariante der SEQ ID-Nr. 1
<400> 33
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Thr Gln Phe Asn Asn Gly
           355                 360                 365
   Ile Glu Val Leu Ser Ala Ala Phe Val Lys Trp Arg Thr Pro Leu Trp
       370                 375                 380
   Arg Tyr Arg Phe Leu Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ser Val Phe Gly Ser Leu Ala Gly Gly Val Gly Ala Ala Asp Ile
               420                 425                 430
   Lys Leu Ala Thr Glu Met Ser Ala Ala Trp Val Ser Phe Ala Ala His
           435                 440                 445
   Gly Val Pro Asp Gln Gly Thr Lys Ser His Trp Pro Arg Phe Asp Ile
       450                 455                 460
   Ser Gly Glu Ile Lys Thr Phe Gly Ser Gln Val Gly Ser Gly Glu Gly
   465                 470                 475                 480
   Leu Gly Val Ser Pro Ser Lys Ala Cys Gln Pro Ser Lys
                   485                 490
<210> 34
   <211> 493
   <212> PRT
   <213> Artificial sequence
<220>
   <223> Polypeptidvariante der SEQ ID-Nr. 1
<400> 34
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Leu Phe Asn His Gly
           355                 360                 365
   Ile Glu Val Leu Ser Ala Ala Phe Val Lys Trp Arg Thr Pro Leu Trp
       370                 375                 380
   Arg Tyr Arg Phe Leu Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ala Val Phe Gly Ser Leu Ala Gly Gly Val Gly Ala Ala Asp Ile
               420                 425                 430
   Lys Leu Ala Ala Glu Met Ser Ser Ala Trp Val Ser Phe Ala Ala His
           435                 440                 445
   Gly Val Pro Asp Gln Gly Thr Lys Ser His Trp Pro Arg Phe Asp Ile
       450                 455                 460
   Ser Gly Glu Ile Lys Thr Phe Gly Ser Gln Val Gly Ser Gly Glu Gly
   465                 470                 475                 480
   Leu Gly Val Ser Pro Ser Lys Ala Cys Pro Pro Ser Lys
                   485                 490
<210> 35
   <211> 493
   <212> PRT
   <213> Artificial sequence
<220>
   <223> Polypeptidvariante der SEQ ID-Nr. 1
<400> 35
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
   180 185 190
   Ser Ala Gly Ala Ser Ala Ile Gly Leu Leu Leu Thr Ser Pro Leu Ser
   195 200 205
   Glu Ser Ala Phe Asn Gln Ala Ile Leu Gln Ser Pro Gly Leu Ala Arg
   210 215 220
   Pro Leu Ala Thr Leu Ser Glu Ser Glu Ala Asn Gly Leu Glu Leu Gly
   225 230 235 240
   Ala Asp Ile Ser Ala Leu Arg Arg Ala Asp Ala Gly Glu Leu Thr Lys
   245 250 255
   Ile Ala Gln Ser Arg Ile Pro Met Ser Arg Gln Phe Thr Lys Pro Arg
   260 265 270
   Pro Met Gly Pro Ile Leu Asp Gly Tyr Val Leu Arg Thr Leu Asp Val
   275 280 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Ser Gln Phe Asn His Gly
   355 360 365
   Ile Glu Val Leu Ser Ala Ala Phe Val Lys Trp Arg Thr Pro Leu Trp
   370 375 380
   Arg Tyr Arg Phe Leu Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ala Val Phe Gly Ser Leu Ala Gly Gly Val Gly Ala Ala Asp Ile
   420 425 430
   Lys Leu Ala Ala Glu Met Ser Ser Ala Trp Val Ser Phe Ala Ala His
   435 440 445
   Gly Val Pro Asp Gln Gly Thr Lys Ser His Trp Pro Arg Phe Asp Ile
   450 455 460
   Ser Gly Glu Ile Lys Thr Phe Gly Ser Gln Val Gly Ser Gly Glu Gly
   465 470 475 480
   Leu Gly Val Ser Pro Ser Lys Ala Cys Pro Pro Ser Lys
   485 490
<210> 36
   <211> 493
   <212> PRT
   <213> Artificial sequence
<220>
   <223> Polypeptidvariante der SEQ ID-Nr. 1
<400> 36
   Gln Thr Asp Asp Pro Lys Leu Val Arg His Thr Gln Ser Gly Ala Val
   1 5 10 15
   Glu Gly Val Glu Gly Asp Val Glu Thr Phe Leu Gly Ile Pro Phe Ala
   20 25 30
   Ala Pro Pro Val Gly Asp Leu Arg Trp Arg Pro Pro Ala Pro Pro Arg
   35 40 45
   Ala Trp Ala Gly Thr Arg Asp Gly Arg Arg Phe Ala Pro Asp Cys Ile
   50 55 60
   Gly Asp Glu Arg Leu Arg Glu Gly Ser Arg Ala Ala Gly Thr Ser Glu
   65 70 75 80
   Asp Cys Leu Tyr Leu Asn Ile Trp Ser Pro Lys Gln Val Gly Lys Gly
   85 90 95
   Gly Leu Pro Val Met Ile Trp Val Tyr Gly Gly Gly Phe Ser Gly Gly
   100 105 110
   Ser Gly Ala Val Pro Tyr Tyr Asp Gly Ser Ala Leu Ala Gln Lys Gly
   115 120 125
   Val Val Val Val Thr Phe Asn Tyr Arg Ala Gly Ile Leu Gly Phe Leu
   130 135 140
   Ala His Pro Ala Leu Ser Lys Glu Ser Pro Asn Gly Val Ser Gly Asn
   145 150 155 160
   Tyr Gly Leu Leu Asp Met Leu Ala Ala Phe Lys Trp Val Gln Asn Asn
   165 170 175
   Ile Arg Glu Phe Gly Gly Asp Pro Asn Arg Val Thr Val Phe Gly Glu
   180 185 190
   Ser Ala Gly Ala Ser Ala Ile Gly Leu Leu Leu Thr Ser Pro Leu Ser
   195 200 205
   Glu Ser Ala Phe Asn Gln Ala Ile Leu Gln Ser Pro Gly Leu Ala Arg
   210 215 220
   Pro Leu Ala Thr Leu Ser Glu Ser Glu Ala Asn Gly Leu Glu Leu Gly
   225 230 235 240
   Ala Asp Ile Ser Ala Leu Arg Arg Ala Asp Ala Gly Glu Leu Thr Lys
   245 250 255
   Ile Ala Gln Ser Arg Ile Pro Met Ser Arg Gln Phe Thr Lys Pro Arg
   260 265 270
   Pro Met Gly Pro Ile Leu Asp Gly Tyr Val Leu Arg Thr Leu Asp Val
   275 280 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Thr Gln Phe Asn His Gly
   355 360 365
   Ile Glu Val Leu Ser Ala Ala Phe Val Lys Trp Arg Thr Pro Leu Trp
   370 375 380
   Arg Tyr Arg Phe Thr Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Met Phe Gly Ser Leu Ala Gly Gly Ala Gly Ala Ala Asp Ile
   420 425 430
   Lys Leu Ala Thr Glu Met Ser Ala Ala Trp Val Ser Phe Ala Val His
   435 440 445
   Gly Val Pro Asp Gln Gly Thr Lys Ser His Trp Pro Arg Phe Asp Arg
   450 455 460
   Arg Gly Glu Ile Met Thr Phe Gly Ser Gln Val Gly Ser Gly Glu Gly
   465 470 475 480
   Leu Gly Val Ser Pro Ser Lys Ala Cys Pro Pro Ser Lys
   485 490
<210> 37
   <211> 493
   <212> PRT
   <213> Artificial sequence
<220>
   <223> Polypeptidvariante der SEQ ID-Nr. 1
<400> 37
   Gln Thr Asp Asp Pro Lys Leu Val Arg His Thr Gln Ser Gly Ala Val
   1 5 10 15
   Glu Gly Val Glu Gly Asp Val Glu Thr Phe Leu Gly Ile Pro Phe Ala
   20 25 30
   Ala Pro Pro Val Gly Asp Leu Arg Trp Arg Pro Pro Ala Pro Pro Arg
   35 40 45
   Ala Trp Ala Gly Thr Arg Asp Gly Arg Arg Phe Ala Pro Asp Cys Ile
   50 55 60
   Gly Asp Glu Arg Leu Arg Glu Gly Ser Arg Ala Ala Gly Thr Ser Glu
   65 70 75 80
   Asp Cys Leu Tyr Leu Asn Ile Trp Ser Pro Lys Gln Val Gly Lys Gly
   85 90 95
   Gly Leu Pro Val Met Ile Trp Val Tyr Gly Gly Gly Phe Ser Gly Gly
   100 105 110
   Ser Gly Ala Val Pro Tyr Tyr Asp Gly Ser Ala Leu Ala Gln Lys Gly
   115 120 125
   Val Val Val Val Thr Phe Asn Tyr Arg Ala Gly Ile Leu Gly Phe Leu
   130 135 140
   Ala His Pro Ala Leu Ser Lys Glu Ser Pro Asn Gly Val Ser Gly Asn
   145 150 155 160
   Tyr Gly Leu Leu Asp Met Leu Ala Ala Phe Lys Trp Val Gln Asn Asn
   165 170 175
   Ile Arg Glu Phe Gly Gly Asp Pro Asn Arg Val Thr Val Phe Gly Glu
   180 185 190
   Ser Ala Gly Ala Ser Ala Ile Gly Leu Leu Leu Thr Ser Pro Leu Ser
   195 200 205
   Glu Ser Ala Phe Asn Gln Ala Ile Leu Gln Ser Pro Gly Leu Ala Arg
   210 215 220
   Pro Leu Ala Thr Leu Ser Glu Ser Glu Ala Asn Gly Leu Glu Leu Gly
   225 230 235 240
   Ala Asp Ile Ser Ala Leu Arg Arg Ala Asp Ala Gly Glu Leu Thr Lys
   245 250 255
   Ile Ala Gln Ser Arg Ile Pro Met Ser Arg Gln Phe Thr Lys Pro Arg
   260 265 270
   Pro Met Gly Pro Ile Leu Asp Gly Tyr Val Leu Arg Thr Leu Asp Val
   275 280 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Ser Gln Phe Asn Asn Gly
   355 360 365
   Ile Glu Leu Leu Ser Ala Ala Phe Val Lys Trp Arg Thr Pro Leu Trp
   370 375 380
   Arg Tyr Arg Phe Thr Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ala Val Phe Gly Ser Leu Ala Gly Gly Val Gly Ala Ala Asp Ile
   420 425 430
   Lys Leu Ala Ala Glu Met Ser Ser Ala Trp Val Ser Phe Ala Ala His
   435 440 445
   Gly Val Pro Asp Gln Gly Thr Lys Ser His Trp Pro Arg Phe Asp Arg
   450 455 460
   Arg Gly Glu Ile Met Thr Phe Gly Ser Gln Val Gly Ser Gly Glu Gly
   465                 470                 475                 480
   Leu Gly Val Ser Pro Ser Lys Ala Cys Gln Pro Ser Lys
                   485                 490
<210> 38
   <211> 493
   <212> PRT
   <213> Artificial sequence
<220>
   <223> Polypeptidvariante der SEQ ID-Nr. 1
<400> 38
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                     240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                     320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn Asn Gly
           355                 360                 365
   Ile Glu Leu Leu Ser Ala Ala Phe Val Lys Trp Arg Thr Pro Leu Trp
       370                 375                 380
   Arg Tyr Arg Phe Leu Gly Ile Pro Gly Pro Gly Arg Arg Pro Ala Thr
   385                 390                 395                     400
   His Gly Asp Glu Ile Pro Tyr Val Phe Ala Asn Leu Gly Pro Ser Ser
                   405                 410                 415
   Val Ser Met Phe Gly Ser Leu Ala Gly Gly Ala Gly Ala Ala Asp Ile
               420                 425                 430
   Lys Leu Ala Thr Glu Met Ser Ala Ala Trp Val Ser Phe Ala Val His
           435                 440                 445
   Gly Val Pro Asp Gln Gly Thr Lys Thr His Trp Pro Arg Phe Asp Ile
       450                 455                 460
   Ser Gly Glu Ile Lys Thr Phe Gly Ser Gln Val Gly Ser Gly Glu Gly
   465                 470                 475                     480
   Leu Gly Val Ser Pro Ser Lys Ala Cys Gln Pro Ser Lys
                   485                 490
<210> 39
   <211> 493
   <212> PRT
   <213> Artificial sequence
<220>
   <223> Polypeptidvariante der SEQ ID-Nr. 1
<400> 39
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
   210 215 220
   Pro Leu Ala Thr Leu Ser Glu Ser Glu Ala Asn Gly Leu Glu Leu Gly
   225 230 235 240
   Ala Asp Ile Ser Ala Leu Arg Arg Ala Asp Ala Gly Glu Leu Thr Lys
   245 250 255
   Ile Ala Gln Ser Arg Ile Pro Met Ser Arg Gln Phe Thr Lys Pro Arg
   260 265 270
   Pro Met Gly Pro Ile Leu Asp Gly Tyr Val Leu Arg Thr Leu Asp Val
   275 280 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Thr Gln Phe Asn Asn Gly
   355 360 365
   Ile Glu Val Leu Ser Ala Ala Phe Val Lys Trp Arg Thr Pro Leu Trp
   370 375 380
   Arg Tyr Arg Phe Leu Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Val Phe Gly Ser Leu Ala Gly Gly Val Gly Ala Ala Asp Ile
   420 425 430
   Lys Leu Ala Thr Glu Met Ser Ser Ala Trp Val Ser Phe Ala Ala His
   435 440 445
   Gly Val Pro Asp Gln Gly Thr Lys Thr His Trp Pro Arg Phe Asp Ile
   450 455 460
   Arg Gly Glu Ile Lys Thr Phe Gly Ser Gln Val Gly Ser Gly Glu Gly
   465 470 475 480
   Leu Gly Val Ser Pro Ser Lys Ala Cys Pro Pro Ser Lys
   485 490
<210> 40
   <211> 493
   <212> PRT
   <213> Artificial sequence
<220>
   <223> Polypeptidvariante der SEQ ID-Nr. 1
<400> 40
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn Asn Gly
           355                 360                 365
   Ile Glu Leu Leu Ser Ala Ala Phe Val Lys Trp Arg Thr Pro Leu Trp
       370                 375                 380
   Arg Tyr Arg Phe Thr Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ser Met Phe Gly Ser Leu Ala Gly Gly Ala Gly Ala Ala Asp Ile
               420                 425                 430
   Lys Leu Ala Thr Glu Met Ser Ala Ala Trp Val Ser Phe Ala Ala His
           435                 440                 445
   Gly Val Pro Asp Gln Gly Thr Lys Ser His Trp Pro Arg Phe Asp Arg
       450                 455                 460
   Ser Gly Glu Ile Lys Thr Phe Gly Ser Gln Val Gly Ser Gly Glu Gly
   465                 470                 475                 480
   Leu Gly Val Ser Pro Ser Lys Ala Cys Pro Pro Ser Lys
                   485                 490
<210> 41
   <211> 493
   <212> PRT
   <213> Artificial sequence
<220>
   <223> Polypeptidvariante der SEQ ID-Nr. 1
<400> 41
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn Asn Gly
           355                 360                 365
   Ile Glu Met Leu Ser Ala Ala Phe Val Lys Trp Arg Thr Pro Leu Trp
       370                 375                 380
   Arg Tyr Arg Phe Thr Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ser Val Phe Gly Ser Leu Ala Gly Gly Val Gly Ala Ala Asp Ile
               420                 425                 430
   Lys Leu Ala Thr Glu Met Ser Ala Ala Trp Val Ser Phe Ala Val His
           435                 440                 445
   Gly Val Pro Asp Gln Gly Thr Lys Ser His Trp Pro Arg Phe Asp Arg
       450                 455                 460
   Ser Gly Glu Ile Lys Thr Phe Gly Ser Gln Val Gly Ser Gly Glu Gly
   465                 470                 475                 480
   Leu Gly Val Ser Pro Ser Asn Ala Cys Pro Pro Ser Lys
                   485                 490
<210> 42
   <211> 493
   <212> PRT
   <213> Artificial sequence
<220>
   <223> Polypeptidvariante der SEQ ID-Nr. 1
<400> 42
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn Asn Gly
           355                 360                 365
   Ile Glu Met Leu Ser Ala Ala Phe Val Lys Trp Arg Thr Pro Leu Trp
       370                 375                 380
   Arg Tyr Arg Phe Thr Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ser Val Phe Gly Ser Leu Ala Gly Gly Val Gly Ala Ala Asp Ile
               420                 425                 430
   Lys Leu Ala Thr Glu Met Ser Ala Ala Trp Val Ser Phe Ala Ala His
           435                 440                 445
   Gly Val Pro Asp Arg Gly Thr Lys Ser His Trp Pro Arg Phe Asp Arg
       450                 455                 460
   Ser Gly Glu Ile Lys Thr Phe Gly Ser Gln Val Gly Ser Gly Glu Gly
   465                 470                 475                 480
   Leu Gly Val Ser Pro Ser Asn Ala Cys Pro Pro Ser Lys
                   485                 490
<210> 43
   <211> 493
   <212> PRT
   <213> Artificial sequence
<220>
   <223> Polypeptidvariante der SEQ ID-Nr. 1
<400> 43
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn His Gly
           355                 360                 365
   Ile Glu Val Leu Ser Ala Ala Phe Val Lys Trp Arg Thr Pro Leu Trp
       370                 375                 380
   Arg Tyr Arg Phe Leu Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ala Val Phe Gly Ser Leu Ala Gly Gly Val Gly Pro Ala Asp Ile
               420                 425                 430
   Lys Leu Ala Ala Glu Met Ser Ser Ala Trp Val Ser Phe Ala Ala His
           435                 440                 445
   Gly Val Pro Asp Gln Gly Thr Lys Thr His Trp Pro Arg Phe Asp Ile
       450                 455                 460
   Ser Gly Glu Ile Lys Thr Phe Gly Ser Gln Val Gly Ser Gly Glu Gly
   465                 470                 475                 480
   Leu Gly Val Ser Pro Ser Lys Ala Cys Pro Pro Ser Lys
                   485                 490
<210> 44
   <211> 493
   <212> PRT
   <213> Artificial sequence
<220>
   <223> Polypeptidvariante der SEQ ID-Nr. 1
<400> 44
   Gln Thr Asp Asp Pro Lys Leu Val Arg His Thr Gln Ser Gly Ala Val
   1               5                   10                  15
   Glu Gly Val Glu Gly Asp Val Glu Thr Phe Leu Gly Ile Pro Phe Ala
               20                  25                  30
   Ala Pro Pro Val Gly Asp Leu Arg Trp Arg Pro Pro Ala Pro Pro Arg
           35                  40                  45
   Ala Trp Ala Gly Thr Arg Asp Gly Arg Arg Phe Ala Pro Asp Cys Ile
       50                  55                  60
   Gly Asp Glu Arg Leu Arg Glu Gly Ser Arg Ala Ala Gly Thr Ser Glu
   65                  70                  75                  80
   Asp Cys Leu Tyr Leu Asn Ile Trp Ser Pro Lys Gln Val Gly Lys Gly
                   85                  90                  95
   Gly Leu Pro Val Met Ile Trp Val Tyr Gly Gly Gly Phe Ser Gly Gly
               100                 105                 110
   Ser Gly Ala Val Pro Tyr Tyr Asp Gly Ser Ala Leu Ala Gln Lys Gly
           115                 120                 125
   Val Val Val Val Thr Phe Asn Tyr Arg Ala Gly Ile Leu Gly Phe Leu
       130                 135                 140
   Ala His Pro Ala Leu Ser Lys Glu Ser Pro Asn Gly Val Ser Gly Asn
   145                 150                 155                 160
   Tyr Gly Leu Leu Asp Met Leu Ala Ala Phe Lys Trp Val Gln Asn Asn
                   165                 170                 175
   Ile Arg Glu Phe Gly Gly Asp Pro Asn Arg Val Thr Val Phe Gly Glu
               180                 185                 190
   Ser Ala Gly Ala Ser Ala Ile Gly Leu Leu Leu Thr Ser Pro Leu Ser
           195                 200                 205
   Glu Ser Ala Phe Asn Gln Ala Ile Leu Gln Ser Pro Gly Leu Ala Arg
       210                 215                 220
   Pro Leu Ala Thr Leu Ser Glu Ser Glu Ala Asn Gly Leu Glu Leu Gly
   225                 230                 235                 240
   Ala Asp Ile Ser Ala Leu Arg Arg Ala Asp Ala Gly Glu Leu Thr Lys
                   245                 250                 255
   Ile Ala Gln Ser Arg Ile Pro Met Ser Arg Gln Phe Thr Lys Pro Arg
   260 265 270
   Pro Met Gly Pro Ile Leu Asp Gly Tyr Val Leu Arg Thr Leu Asp Val
   275 280 285
   Asp Ala Phe Ala Lys Gly Ala Phe Arg Lys Ile Pro Val Ile Val Gly
   290 295 300
   Gly Asn Ala Asp Glu Gly Arg Ala Phe Thr Asp Arg Leu Pro Val Lys
   305 310 315 320
   Thr Val Leu Glu Tyr Arg Ala Tyr Leu Thr Glu Gln Phe Gly Asp Glu
   325 330 335
   Ala Asp Ala Trp Glu Arg Cys Tyr Pro Ala Asn Ser Asp Ala Asp Val
   340 345 350
   Pro Ala Ala Val Ala Arg Leu Phe Gly Asp Ser Gln Phe Asn Asn Gly
   355 360 365
   Ile Glu Met Leu Ser Ala Ala Phe Val Lys Trp Arg Thr Pro Leu Trp
   370 375 380
   Arg Tyr Arg Phe Leu Gly Ile Pro Gly Pro Gly Arg Arg Pro Ala Thr
   385 390 395 400
   His Gly Asp Glu Ile Pro Tyr Val Phe Ala Asn Leu Gly Pro Ser Ser
   405 410 415
   Val Ser Val Phe Gly Ser Leu Ala Gly Gly Val Gly Ala Ala Asp Ile
   420 425 430
   Lys Leu Ala Ala Glu Met Ser Ala Ala Trp Val Ser Phe Ala Ala His
   435 440 445
   Gly Val Pro Asp Arg Gly Thr Gln Ser His Trp Pro Arg Tyr Asp Arg
   450 455 460
   Ser Gly Glu Ile Lys Thr Phe Gly Ser Gln Val Gly Ser Gly Glu Gly
   465 470 475 480
   Leu Gly Val Ser Pro Ser Asn Ala Cys Pro Pro Ser Lys
   485 490
<210> 45
   <211> 493
   <212> PRT
   <213> Artificial sequence
<220>
   <223> Polypeptidvariante der SEQ ID-Nr. 1
<400> 45
   Gln Thr Asp Asp Pro Lys Leu Val Arg His Thr Gln Ser Gly Ala Val
   1 5 10 15
   Glu Gly Val Glu Gly Asp Val Glu Thr Phe Leu Gly Ile Pro Phe Ala
   20 25 30
   Ala Pro Pro Val Gly Asp Leu Arg Trp Arg Pro Pro Ala Pro Pro Arg
   35 40 45
   Ala Trp Ala Gly Thr Arg Asp Gly Arg Arg Phe Ala Pro Asp Cys Ile
   50 55 60
   Gly Asp Glu Arg Leu Arg Glu Gly Ser Arg Ala Ala Gly Thr Ser Glu
   65 70 75 80
   Asp Cys Leu Tyr Leu Asn Ile Trp Ser Pro Lys Gln Val Gly Lys Gly
   85 90 95
   Gly Leu Pro Val Met Ile Trp Val Tyr Gly Gly Gly Phe Ser Gly Gly
   100 105 110
   Ser Gly Ala Val Pro Tyr Tyr Asp Gly Ser Ala Leu Ala Gln Lys Gly
   115 120 125
   Val Val Val Val Thr Phe Asn Tyr Arg Ala Gly Ile Leu Gly Phe Leu
   130 135 140
   Ala His Pro Ala Leu Ser Lys Glu Ser Pro Asn Gly Val Ser Gly Asn
   145 150 155 160
   Tyr Gly Leu Leu Asp Met Leu Ala Ala Phe Lys Trp Val Gln Asn Asn
   165 170 175
   Ile Arg Glu Phe Gly Gly Asp Pro Asn Arg Val Thr Val Phe Gly Glu
   180 185 190
   Ser Ala Gly Ala Ser Ala Ile Gly Leu Leu Leu Thr Ser Pro Leu Ser
   195 200 205
   Glu Ser Ala Phe Asn Gln Ala Ile Leu Gln Ser Pro Gly Leu Ala Arg
   210 215 220
   Pro Leu Ala Thr Leu Ser Glu Ser Glu Ala Asn Gly Leu Glu Leu Gly
   225 230 235 240
   Ala Asp Ile Ser Ala Leu Arg Arg Ala Asp Ala Gly Glu Leu Thr Lys
   245 250 255
   Ile Ala Gln Ser Arg Ile Pro Met Ser Arg Gln Phe Thr Lys Pro Arg
               260                 265                 270
   Pro Met Gly Pro Ile Leu Asp Gly Tyr Val Leu Arg Thr Leu Asp Val
           275                 280                 285
   Asp Ala Phe Ala Lys Gly Ala Phe Arg Lys Ile Pro Val Ile Val Gly
       290                 295                 300
   Gly Asn Ala Asp Glu Gly Arg Ala Phe Thr Asp Arg Leu Pro Val Lys
   305                 310                 315                 320
   Thr Val Leu Glu Tyr Arg Ala Tyr Leu Thr Glu Gln Phe Gly Asp Glu
                   325                 330                 335
   Ala Asp Ala Trp Glu Arg Cys Tyr Pro Ala Asn Ser Asp Ala Asp Val
               340                 345                 350
   Pro Ala Ala Val Ala Arg Leu Phe Gly Asp Ser Gln Phe Asn His Gly
           355                 360                 365
   Ile Glu Val Leu Ser Ala Ala Phe Val Lys Trp Arg Thr Pro Leu Trp
       370                 375                 380
   Arg Tyr Arg Phe Leu Gly Ile Pro Gly Pro Gly Arg Arg Pro Ala Thr
   385                 390                 395                 400
   His Gly Asp Glu Ile Pro Tyr Val Phe Ala Asn Leu Gly Pro Ser Ser
                   405                 410                 415
   Val Ala Val Phe Gly Ser Leu Ala Gly Gly Val Gly Pro Ala Asp Ile
               420                 425                 430
   Lys Leu Ala Ala Glu Met Ser Ser Ala Trp Val Ser Phe Ala Ala His
           435                 440                 445
   Gly Val Pro Asp Arg Gly Thr Gln Thr His Trp Pro Arg Tyr Asp Ile
       450                 455                 460
   Ser Gly Glu Ile Lys Thr Phe Gly Ser Gln Val Gly Ser Gly Glu Gly
   465                 470                 475                 480
   Leu Gly Val Ser Pro Ser Asn Ala Cys Pro Pro Ser Lys
                   485                 490
<210> 46
   <211> 540
   <212> PRT
   <213> Sphingopyxis sp.
<400> 46
   Val Lys Glu His Gln Cys Arg Gly Gly Arg Ala Ser Pro Ala Ala Pro
   1 5 10 15
   Ala Thr Trp Leu Ala Arg Ile Ser Val Ser Arg Gly Ala Ser Ala Ile
   20 25 30
   Ala Trp Thr Phe Met Leu Gly Ala Thr Ala Ile Pro Val Ala Ala Gln
   35 40 45
   Thr Asp Asp Pro Lys Leu Val Arg His Thr Gln Ser Gly Ala Val Glu
   50 55 60
   Gly Val Glu Gly Asp Val Glu Thr Phe Leu Gly Ile Pro Phe Ala Ala
   65 70 75 80
   Pro Pro Val Gly Asp Leu Arg Trp Arg Pro Pro Ala Pro Pro Arg Ala
   85 90 95
   Trp Ala Gly Thr Arg Asp Gly Arg Arg Phe Ala Pro Asp Cys Ile Gly
   100 105 110
   Asn Glu Arg Leu Arg Glu Gly Ser Arg Ala Ala Gly Thr Ser Glu Asp
   115 120 125
   Cys Leu Tyr Leu Asn Ile Trp Ser Pro Lys Gln Val Gly Lys Gly Gly
   130 135 140
   Leu Pro Val Met Ile Trp Val Tyr Gly Gly Gly Phe Ser Gly Gly Ser
   145 150 155 160
   Gly Ala Val Pro Tyr Tyr Asp Gly Ser Ala Leu Ala Gln Lys Gly Val
   165 170 175
   Val Val Val Thr Phe Asn Tyr Arg Ala Gly Ile Leu Gly Phe Leu Ala
   180 185 190
   His Pro Ala Leu Ser Lys Glu Ser Pro Asn Gly Val Ser Gly Asn Tyr
   195 200 205
   Gly Leu Leu Asp Met Leu Ala Ala Phe Lys Trp Val Gln Asn Asn Ile
   210 215 220
   Arg Glu Phe Gly Gly Asp Pro Asn Arg Val Thr Val Phe Gly Glu Ser
   225 230 235 240
   Ala Gly Ala Ser Ala Leu Gly Leu Leu Leu Thr Ser Pro Leu Ser Glu
   245 250 255
   Ser Ala Phe Asn Gln Ala Ile Leu Gln Ser Pro Gly Leu Ala Arg Pro
   260 265 270
   Leu Ala Thr Leu Ser Glu Ser Glu Ala Asn Gly Leu Glu Leu Gly Ala
                   275                 280                 285
   Asp Ile Ser Ala Leu Arg Arg Ala Asp Ala Gly Glu Leu Thr Lys Ile
       290                 295                 300
   Ala Gln Ser Arg Ile Pro Met Ser Arg Gln Phe Thr Lys Pro Arg Pro
   305                 310                 315                 320
   Met Gly Pro Ile Leu Asp Gly Tyr Val Leu Arg Thr Leu Asp Val Asp
                   325                 330                 335
   Ala Phe Ala Lys Gly Ala Phe Arg Lys Ile Pro Val Leu Val Gly Gly
               340                 345                 350
   Asn Ala Asp Glu Gly Arg Ala Phe Thr Asp Arg Leu Pro Val Lys Thr
           355                 360                 365
   Val Leu Glu Tyr Arg Ala Tyr Leu Thr Glu Gln Phe Gly Asp Glu Ala
       370                 375                 380
   Asp Ala Trp Glu Arg Cys Tyr Pro Ala Asn Ser Asp Ala Asp Val Pro
   385                 390                 395                 400
   Ala Ala Val Ala Arg Leu Phe Gly Asp Ser Gln Phe Asn Asn Gly Ile
                   405                 410                 415
   Glu Leu Leu Ser Ala Ala Phe Ala Lys Trp Arg Thr Pro Leu Trp Arg
               420                 425                 430
   Tyr Arg Phe Thr Gly Ile Pro Gly Ala Gly Arg Arg Pro Ala Thr His
           435                 440                 445
   Gly Asp Glu Ile Pro Tyr Val Phe Ala Asn Leu Gly Pro Ser Ser Val
       450                 455                 460
   Ser Met Phe Gly Ser Leu Glu Gly Gly Ala Gly Ala Ser Asp Ile Lys
   465                 470                 475                 480
   Leu Ala Thr Glu Met Ser Ala Ala Trp Val Ser Phe Ala Val His Gly
                   485                 490                 495
   Val Pro Asp Gln Gly Thr Lys Ser His Trp Pro Arg Phe Glu Arg Arg
               500                 505                 510
   Gly Glu Ile Met Thr Phe Gly Ser Gln Val Gly Ser Gly Glu Gly Leu
           515                 520                 525
   Gly Val Ser Pro Ser Lys Ala Cys Gln Pro Ser Lys
       530                 535                 540